# EUROPEAN PATENT SPECIFICATION

(11) **EP 2 396 329 B1**
(45) Date of publication and mention of the grant of the patent: **29.10.2014**
(21) Application number: 09789461.2
(22) Date of filing: 11.02.2009
(51) Int. Cl.: C07D 487/04, A61K 31/55, A61P 31/14

(54) **COMPOUNDS FOR THE TREATMENT OF HEPATITIS C**
VERBINDUNG ZUR BEHANDLUNG VON HEPATITIS C
COMPOSÉS DESTINÉS AU TRAITEMENT DE L'HÉPATITE C

(43) Date of publication of application: 21.12.2011
(73) Proprietor: Bristol-Myers Squibb Company, Princeton, NJ 05843-4000 (US)
(72) Inventor: MARTIN, Scott, W., Wallingford, Connecticut 06492 (US); BERGSTROM, Carl P., Wallingford, Connecticut 06492 (US); GENTLES, Robert G., Wallingford, Connecticut 06492 (US); YEUNG, Kap-Sun, Wallingford, Connecticut 06492 (US)
(74) Representative: Reitstötter Kinzebach
(86) International application number: PCT/US2009/033801
(87) International publication number: WO 2010/093359

(56) References cited:
- WO-A-2007/033175
- WO-A-2007/092888
- WO-A-2009/029384

## Description

### BACKGROUND OF THE INVENTION

Hepatitis C virus (HCV) is a major human pathogen, infecting an estimated 170 million persons worldwide - roughly five times the number infected by human immunodeficiency virus type 1. A substantial fraction of these HCV infected individuals develop serious progressive liver disease, including cirrhosis and hepatocellular carcinoma (Lauer, G. M.; Walker, B. D. N. Engl. J. Med. 2001, 345, 41-52).

HCV is a positive-stranded RNA virus. Based on a comparison of the deduced amino acid sequence and the extensive similarity in the 5'-untranslated region, HCV has been classified as a separate genus in the Flaviviridae family. All members of the Flaviviridae family have enveloped virions that contain a positive stranded RNA genome encoding all known virus-specific proteins via translation of a single, uninterrupted, open reading frame.

Considerable heterogeneity is found within the nucleotide and encoded amino acid sequence throughout the HCV genome. At least six major genotypes have been characterized, and more than 50 subtypes have been described. The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature non-structural proteins (NS2, NS3, NS4A, NS4B, NS5A, and NS5B) is effected by two viral proteases. The first one is believed to be a metalloprotease and cleaves at the NS2-NS3 junction; the second one is a serine protease contained within the N-terminal region of NS3 (also referred to as NS3 protease) and mediates all the subsequent cleavages downstream of NS3, both in cis, at the NS3-NS4A cleavage site, and in trans, for the remaining NS4A-NS4B, NS4B-NSSA, NS5A-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B (also referred to as HCV polymerase) is a RNA-dependent RNA polymerase that is involved in the replication of HCV. The HCV NS5B protein is described in "Structural Analysis of the Hepatitis C Virus RNA Polymerase in Complex with Ribonucleotides (Bressanelli; S. et al., Journal of Virology 2002, 3482-3492; and Defrancesco and Rice, Clinics in Liver Disease 2003, 7, 211-242.

Currently, the most effective HCV therapy employs a combination of alpha-interferon and ribavirin, leading to sustained efficacy in 40% of patients (Poynard, T. et al. Lancet 1998, 352, 1426-1432). Recent clinical results demonstrate that pegylated alpha-interferon is superior to unmodified alpha-interferon as monotherapy (Zeuzem, S. et al. N. Engl. J. Med. 2000, 343, 1666-1672). However, even with experimental therapeutic regimens involving combinations of pegylated alpha-interferon and ribavirin, a substantial fraction of patients do not have a sustained reduction in viral load. Thus, there is a clear and important need to develop effective therapeutics for treatment of HCV infection.

### DESCRIPTION OF THE INVENTION

The compounds of the invention are selected from the group consisting of
7H-indolo[2,1-a][2]benzazepine-10-carboxamide, 13-cyclohexyl-6-[1-ethyl-3-methyl-4-[(3-methyl-3,8-diazabicyclo[3.2.1]oct-8-yl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-4-[[(3R,5S)-3,4,5-trimethyl-1-piperazinyl]carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-4-[(1S,4S)-2-oxa-5-azabicyclo[2.2.1]hept-5-ylcarbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1,3-dimethyl-4-(8-oxa-3-azabicyclo[3.2.1]oct-3-ylcarbonyl)-1H-pyrazol-5-yl]-3-methoxy-N-[(1-methylethyl)sulfonyl]-;
7H-indolo[2,1-a][2]benzazepine-10-carboxamide, 13-cyclohexyl-3-methoxy-6-[4-[[(2S)-2-(methoxymethyl)-4-morpholinyl]carbonyl]-1,3-dimethyl-1H-pyrazol-5-yl]-N-[(1-methylethyl)sulfonyl]-;
7H-indolo[2,1-a][2]benzazepine-10-carboxamide, 13-cyclohexyl-6-[1,3-dimethyl-4-[(4-methyl-1-piperazinyl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1,3-dimethyl-4-[[(3R,5S)-3,4,5-trimethyl-1-piperazinyl]carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[4-[[(2R,6S)-2,6-dimethyl-4-morpholinyl]carbonyl]-1-(1-methylethyl)-1H-1,2,3-triazol-5-yl]-3-methoxy-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[1-(1-methylethyl)-4-[[(3R,SS)-3,4,5-trimethyl-1-piperazinyl]carbonyl]-1H-1,2,3-triazol-5-yl]-;
7H-indolo[2,1-a][2]benzazepine-10-carboxylic acid, 13-cyclohexyl-6-[4-(ethoxycarbonyl)-1-ethyl-3-methyl-1H-pyrazol-5-yl]-3-methoxy-;
1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-, ethyl ester;
7H-indolo[2,1-a][2]benzazepine-10-carboxamide, 13-cyclohexyl-6-[4-[[(2R,6S)-2,6-dimethyl-4-morpholinyl]carbonyl]-5-methyl-1-(1-methylethyl)-1H-pyrazol-3-yl]-3-methoxy-N-[(1-methylethyl)sulfonyl]-;
1H-pyrazole-4-carboxylic acid, 5-[10-(aminocarbonyl)-13-cyclohexyl-3-methoxy-7H-indolo[2,1-a][2]benzazepin-6-yl]-1-ethyl-3-methyl-, ethyl ester;
7H-indolo[2,1-a][2]benzazepine-10-carboxylic acid, 13-cyclohexyl-6-[4-(ethoxycarbonyl)-1-ethyl-1H-pyrazol-5-yl]-3-methoxy-,1,1-dimethylethyl ester;
7H-indolo[2,1-a][2]benzazepine-10-carboxylic acid, 13-cyclohexyl-6-[4-(ethoxycarbonyl)-1-ethyl-1H-pyrazol-3-yl]-3-methoxy-, 1,1-dimethylethyl ester;
1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-;
7H-indolo[2,1-a][2]benzazepine-10-carboxamide, 13-cyclohexyl-6-[4-[[(2R,6S)-2,6-dimethyl-4-morpholinyl]carbonyl]-1-(1-methylethyl)-1H-imidazol-5-yl]-3-methoxy-N-[(1-methylethyl)sulfonyl]-;
7H-indolo[2,1-a][2]benzazepine-10-carboxamide, 13-cyclohexyl-3-methoxy-6-[1-(1-methylethyl)-4-(4-morpholinylcarbonyl)-1H-imidazol-5-yl]-N-[(1-methylethyl)sulfonyl]-;
7H-indolo[2,1-a][2]benzazepine-10-carboxamide, 13-cyclohexyl-3-methoxy-6-[4-[(3-methyl-3,8-diazabicyclo[3.2.1]oct-8-yl)carbonyl]-1-(1-methylethyl)-1H-imidazol-5-yl]-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[1-(1-methylethyl)-4-[[(3R,5S)-3,4,5-trimethyl-1-piperazinyl]carbonyl]-1H-imidazol-5-yl]-;
7H-indolo[2,1-a][2]benzazepine-10-carboxamide, 13-cyclohexyl-3-methoxy-N-[(1-methylethyl)sulfonyl]-6-[5-methyl-4-[(3-methyl-3,8-diazabicyclo[3.2.1]oct-8-yl)carbonyl]-1-(1-methylethyl)-1H-pyrazol-3-yl]-;
7H-indolo[2,1-a][2]benzazepine-10-carboxylic acid, 13-cyclohexyl-6-[4-(ethoxycarbonyl)-1-ethyl-1H-pyrazol-5-yl]-3-methoxy-;
1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-, ethyl ester;
7H-indolo[2,1-a][2]benzazepine-10-carboxamide, 13-cyclohexyl-N-[(dimethylamino)sulfonyl]-6-[5-[[(2R,6S)-2,6-dimethyl-4-morpholinyl]carbonyl]-1-(1-methylethyl)-1H-imidazol-4-yl]-3-methoxy-;
7H-indolo[2,1-a][2]benzazepine-10-carboxamide, 13-cyclohexyl-N-[(dimethylamino)sulfonyl]-3-methoxy-6-[1-(1-methylethyl)-5-(4-morpholinylcarbonyl)-1H-imidazol-4-yl]-;
1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-;
7H-indolo[2,1-a][2]benzazepine-10-carboxamide, 13-cyclohexyl-N-[(dimethylamino)sulfonyl]-3-methoxy-6-[1-(1-methylethyl)-4-[[(3R,5S)-3,4,5-trimethyl-1-piperazinyl]carbonyl]-1H-1,2,3-triazol-5-yl]-;
7H-indolo[2,1-a][2]benzazepine-10-carboxamide, 13-cyclohexyl-N-[(dimethylamino)sulfonyl]-6-[4-[[(2R,6S)-2,6-dimethyl-4-morpholinyl]carbonyl]-1-(1-methylethyl)-1H-1,2,3-triazol-5-yl)-3-methoxy-;
7H-indolo[2,1-a][2]benzazepine-10-carboxamide, 13-cyclohexyl-N-[(dimethylamino)sulfonyl]-3-methoxy-6-[4-[(3-methyl-3,8-diazabicyclo[3.2.1]oct-8-yl)carbonyl)-1-(1-methylethyl)-1H-1,2,3-triazol-5-yl]-;
7H-indolo[2,1-a][2]benzazepine-10-carboxamide, 13-cyclohexyl-6-[4-[[(2R,6S)-2,6-dimethyl-4-morpholinyl]carbonyl]-1-ethyl-1H-pyrazol-5-yl]-3-methoxy-N-[(1-methylethyl)sulfonyl]-;
7H-indolo[2,1-a][2]benzazepine-10-carboxamide, 13-cyclohexyl-N-[(dimethylamino)sulfonyl]-6-[4-[[(2R,6S)-2,6-dimethyl-4-morpholinyl]carbonyl]-1-ethyl-3-methyl-1 H-pyrazol-5-yl]-3-methoxy-;
7H-indolo[2,1-a][2]benzazepine-10-carboxamide, 13-cyclohexyl-N-[(dimethylamino)sulfonyl]-3-methoxy-6-[1-(1-methylethyl)-4-(4-morpholinylcarbonyl)-1H-1,2,3-triazol-5-yl]-;
7H-indolo[2,1-a][2]benzazepine-10-carboxamide, 13-cyclohexyl-6-[4-[(6,6-difluorohexahydro-4-methyl-1H-1,4-diazepin-1-yl)carbonyl]-1-(1-methylethyl)-1H-1,2,3-triazol-5-yl]-N-[(dimethylamino)sulfonyl]-3-methoxy-;
7H-indolo[2,1-a][2]benzazepine-10-carboxamide, 13-cyclohexyl-6-[1-ethyl-4-[[(2R)-2-(methoxymethyl)-4-morpholinyl]carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-(3-oxa-9-azabicyclo[3.3.1]non-9-ylcarbonyl)-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-(2-oxa-5-azabicyclo[2.2.1]hept-5-ylcarbonyl)-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-[[(3R,5S)-3,4,5-trimethyl-1-piperazinyl]carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-[(3-methyl-3,8-diazabicyclo[3.2.1]oct-8-yl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-[(5-ethyl-2,5-diazabicyclo[2.2.1]hept-2-yl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[1-methyl-3-(1-methylethyl)-4-(4-morpholinylcarbonyl)-1H-pyrazol-5-yl]-;
7H-indolo[2,1-a][2]benzazepine-10-carboxamide, 13-cyclohexyl-6-[4-[[(2R,6S)-2,6-dimethyl-4-morpholinyl]carbonyl]-1-methyl-3-(1-methylethyl)-1H-pyrazol-5-yl]-3-methoxy-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[1-methyl-3-(1-methylethyl)-4-[(4-methyl-1-piperazinyl)carbonyl]-1H-pyrazol-5-yl]-;
7H-indolo[2,1-a][2]benzazepine-10-carboxamide, 13-cyclohexyl-3-methoxy-N-[(1-methylethyl)sulfonyl]-6-[1-methyl-3-(1-methylethyl)-4-[[(3R,5S)-3,4,5-trimethyl-1-piperazinyl]carbonyl]- I H-pyrazol-5-yl]-;
7H-indolo[2,1-a][2]benzazepine-10-carboxamide, 13-cyclohexyl-3-methoxy-N-[(1-methylethyl)sulfonyl]-6-[4-(8-oxa-3-azabicyclo[3.2-1]oct-3-ylcarbonyl)-1-(2,2,2-trifluoroethyl)-1H-pyrazol-5-yl]-;
7H-indolo[2,1-a][2]benzazepine-10-carboxamide, 13-cyclohexyl-6-[4-(3,7-dioxa-9-azabicyclo[3.3.1]non-9-ylcarbonyl)-1-(2,2,2-trifluoroethyl)-1H-pyrazol-5-yl]-3-methoxy-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[4-(2-oxa-5-azabicyclo[2.2.1]hept-5-ylcarbonyl)-1-(2,2,2-trifluoroethyl)-1H-pyrazol-5-yl]-;
7H-indolo[2,1-a][2]benzazepine-10-carboxamide, 13-cyclohexyl-3-methoxy-N-[(1-methylethyl)sulfonyl]-6-[4-[(4-methyl-1-piperazinyl)carbonyl]-1-(2,2,2-trifluoroethyl)-1H-pyrazol-5-yl]-;
7H-indolo[2,1-a][2]benzazepine-10-carboxamide, 13-cyclohexyl-3-methoxy-6-[1-(1-methylethyl)-4-(8-oxa-3-azabicyclo[3.2.1]oct-3-ylcarbonyl)-1H-pyrazol-5-yl]-N-[(1-methylethyl)sulfonyl]-;
7H-indolo[2,1-a][2]benzazepine-10-carboxamide, 13-cyclohexyl-3-methoxy-6-[1-(1-methylethyl)-4-(3-oxa-9-azabicyclo[3.3.1]non-9-ylcarbonyl)-1H-pyrazol-5-yl]-N-[(1-methylethyl)sulfonyl]-;
7H-indolo[2,1-a][2]benzazepine-10-carboxamide, 13-cyclohexyl-3-methoxy-6-[1-(1-methylethyl)-4-(2-oxa-5-azabicyclo[2.2.1]hept-5-ylcarbonyl)-1H-pyrazol-5-yl]-N-[(1-methylethyl)sulfonyl]-;
7H-indolo[2,1-a][2]benzazepine-10-carboxamide, 13-cyclohexyl-3-methoxy-6-[1-(1-methylethyl)-4-[(4-methyl-1-piperazinyl)carbonyl]-1H-pyrazol-5-yl]-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[3-methyl-1-(1-methylethyl)-4-(8-oxa-3-azabicyclo[3.2.1]oct-3-ylcarbonyl)-1H-pyrazol-5-yl]-;
7H-indolo[2,1-a][2]benzazepine-10-carboxamide, 13-cyclohexyl-3-methoxy-N-[(1-methylethyl)sulfonyl]-6-[3-methyl-1-(1-methylethyl)-4-(2-oxa-5-azabicyclo[2.2.1]hept-5-ylcarbonyl)-1H-pyrazol-5-yl]-;
7H-indolo[2,1-a][2]benzazepine-10-carboxamide, 13-cyclohexyl-3-methoxy-N-[(1-methylethyl)sulfonyl]-6-[3-methyl-4-[(3-methyl-3,8-diazabicyclo[3.2.1]oct-8-yl)carbbnyl]-1-(1-methylethyl)-1H-pyrazol-5-yl]-;
7H-indolo[2,1-a][2]benzazepine-10-carboxamide, 13-cyclohexyl-6-[4-[(5-ethyl-2,5-diazabicyclo[2.2.1]hept-2-yl)carbonyl]-3-methyl-1-(1-methylethyl)-1H-pyrazol-5-yl]-3-methoxy-N-[(1-methylethyl)sulfonyl]-;
7H-indolo[2,1-a][2]benzazepine-10-carboxamide, 13-cyclohexyl-N-(cyclopropylsulfonyl)-6-[4-[[(2R,6S)-2,6-dimethyl-4-morpholinyl]carbonyl]-1-ethyl-3-methyl-1H-pyrazol-5-yl]-3-methoxy-;
7H-indolo[2,1-a][2]benzazepine-10-carboxamide, 13-cyclohexyl-3-methoxy-N-[(1-methylethyl)sulfonyl]-6-[5-methyl-1-(1-methylethyl)-4-[[(3R,5S)-3,4,5-trimethyl-1-piperazinyl]carbonyl]-1H-pyrazol-3-yl]-;
7H-indolo[2,1-a][2]benzazepine-10-carboxamide, 13-cyclohexyl-6-[1-ethyl-4-(8-oxa-3-azabicyclo[3.2.1]oct-3-ylcarbonyl)-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-[(8-methyl-3,8-diazabicyclo[3.2.1]oct-3-yl)carbonyl]-1H-pyrazol-5-yl]-3-methoxy-N-[(1-methylethyl)sulfonyl]-1H-pyrazole-4-carboxylic acid;
5-[13-cyclohexyl-10-[[[(dimethylamino)sulfonyl]amino]carbonyl]-3-methoxy-7H-indolo[2,1-a][2]benzazepin-6-yl]-1-ethyl-3-methyl-, ethyl ester;
7H-indolo[2,1-a][2]benzazepine-10-carboxamide, 13-cyclohexyl-6-[1-ethyl-3-methyl-5-(4-morpholinylcarbonyl)-1H-pyrazol-4-yl]-3-methoxy-N-[(1-methylethyl)sulfonyl]-;
7H-indolo[2,1-a][2]benzazepine-10-carboxamide, 13-cyclohexyl-6-[5-[[(2R,6S)-2,6-dimethyl-4-morpholinyl]carbonyl]-1-ethyl-3-methyl-1H-pyrazol-4-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-5-[(4-methyl-1-piperazinyl)carbonyl]-1H-pyrazol-4-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-5-[[(3R,5S)-3,4,5-trimethyl-1-piperazinyl]carbonyl]-1H-pyrazol-4-yl]-3-methoxy-N-[(1-methylethyl)sulfonyl]-1H-pyrazole-4-carboxylic acid;
5-[13-cyclohexyl-10-[[[(dimethylamino)sulfonyl]amino]carbonyl]-3-methoxy-7H-indolo[2,1-a][2]benzazepin-6-yl]-1-ethyl-3-methyl-;
7H-indolo[2,1-a][2]benzazepine-10-carboxamide, 13-cyclohexyl-6-[1-ethyl-4-[(5-ethyl-2,5-diazabicyclo[2.2.1]hept-2-yl)carbonyl]-3-methyl-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-4-[(8-methyl-3,8-diazabicyclo[3.2.1]oct-3-yl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-4-(4-morpholinylcarbonyl)-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-4-(3-oxa-9-azabicyclo[3.3.1]non-9-ylcarbonyl)-1H-pyrazol-5-yl]-3-methoxy-N-[(1-methylethyl)sulfonyl]-; and
7H-indolo[2,1-a][2]benzazepine-10-carboxamide, 13-cyclohexyl-6-[1-ethyl-4-[[(2S)-2-(methoxymethyl)-4-morpholinyl]carbonyl]-3-methyl-1H-pyrazol-5-yl]-3-methoxy-N-[(1-methylethyl)sulfonyl]-;
or a pharmaceutically acceptable salt thereof.

Unless specified otherwise, these terms have the following meanings. "Alkyl" means a straight or branched alkyl group composed of 1 to 6 carbons. "Alkenyl" means a straight or branched alkyl group composed of 2 to 6 carbons with at least one double bond. "Cycloalkyl" means a monocyclic ring system composed of 3 to 7 carbons. "Hydroxyalkyl," "alkoxy" and other terms with a substituted alkyl moiety include straight and branched isomers composed of 1 to 6 carbon atoms for the alkyl moiety. "Haloalkyl" and "haloalkoxy" include all halogenated isomers from monohalo substituted alkyl to perhalo substituted alkyl. "Aryl" includes carbocyclic and heterocyclic aromatic substituents. Parenthetic and multiparenthetic terms are intended to clarify bonding relationships to those skilled in the art. For example, a term such as ((R)alkyl) means an alkyl substituent further substituted with the substituent R.

The invention includes all pharmaceutically acceptable salt forms of the compounds. Pharmaceutically acceptable salts are those in which the counter ions do not contribute significantly to the physiological activity or toxicity of the compounds and as such function as pharmacological equivalents. These salts can be made according to common organic techniques employing commercially available reagents. Some anionic salt forms include acetate, acistrate, besylate, bromide, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc.

Some of the compounds of the invention possess asymmetric carbon atoms. The invention includes all stereoisomeric forms, including enantiomers and diastereomers as well as mixtures of stereoisomers such as racemates. Some stereoisomers can be made using methods known in the art. Stereoisomeric mixtures of the compounds and related intermediates can be separated into individual isomers according to methods commonly known in the art.

### Synthetic Methods

The compounds may be made by methods known in the art including those described below. Some reagents and intermediates are known in the art. Other reagents and intermediates can be made by methods known in the art using available materials. The variables (e.g. numbered "R" substituents) used to describe the synthesis of the compounds are intended only to illustrate how to make the compounds and are not to be confused with variables used in the claims or in other sections of the specification. Abbreviations used within the schemes generally follow conventions used in the art.

The scheme shown below illustrates methods that can be used for making intermediates and compounds.

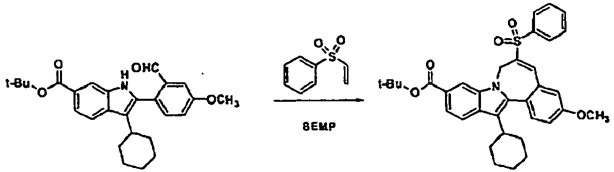

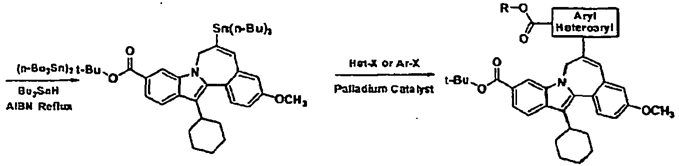

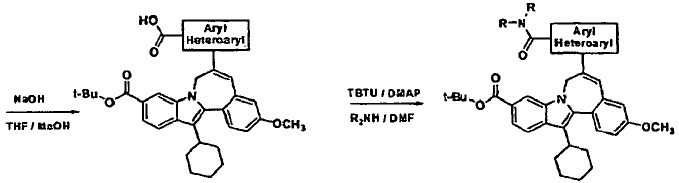

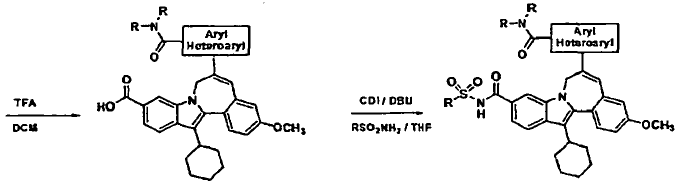

Alternatively, the reaction sequence can be modified as shown below.

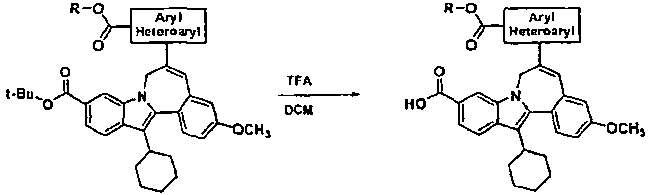

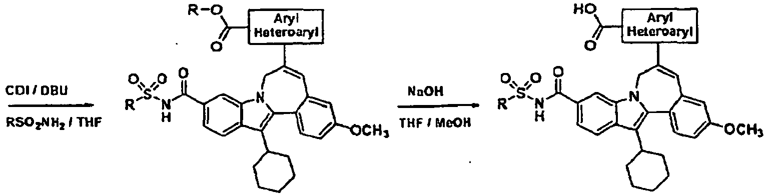

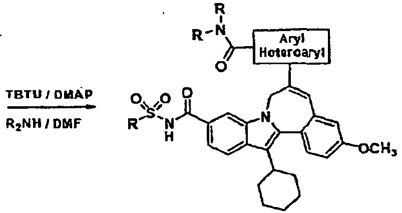

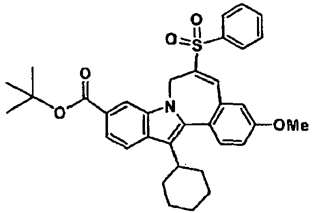

### 7H-Indolo[2,1-a][2]benzazepine-10-carboxylic acid, 13-cyclohexyl-3-methoxy-6-(phenyllsulfonyl)-. tert-butyl ester.

To a solution of 3-cyclohexyl-2-(2-formyl-4-methoxyphenyl)-1H-indole-6-carboxylate (6.00 g, 13.8 mmol) in dioxane (28.0 mL) and BEMP (7.97 mL, 27.6 mmol) was added phenyl vinyl sulfone (27.6 g, 2.21 mmol). The resulting mixture was stirred in a sealed tube in a microwave at 120 °C for 15 min. The resulting solution was concentrated under reduced pressure. Silica gel chromatography (CH₂Cl₂) of the concentrate afforded the title compound 6.36g (79%) as a yellow oil. MS m/z 584 (MH⁺). 1H NMR (500 MHz, *CHLOROFORM-d)* δ ppm 1.18-1.33 (1 H, m), 1.34-1.45 (2 H, m), 1.49-57 (1 H, m), 1.64 (9 H, s.), 1.74-1.82 (2 H, m), 1.90-2.09 (4 H, m), 2.73 (1 H, m,), 3.93 (3 H, s), 4.38 (1 H, broad d), 5.08 (1 H, br, d), 7.09 (1 H, d, *J*=2.75 Hz), 7.12-7.18 (3 H, m), 7.22 (1 H, d, *J*=7.45 Hz), 7.30 (1H, s), 7.48 (1 H, d, *J*=8.85 Hz), 7.54 (1 H, dd, *J*=8.55, 1.22 Hz), 7.61 (2 H, m), 7.67 (1 H, d, *J*=8.55), 8.01 (1 H, s).

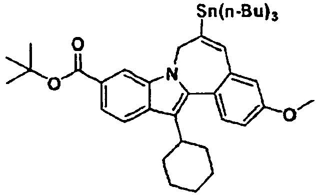

*7H-indolo[2,1-a][2]benzazepine-10-carboxylic acid, 13-cyclohexyl-3-methoxy-6-(tributylstannyl)-, 1,1-dimethylethyl ester, 1,1-dimethylethyl 13-cyclohexyl-3-(methyloxy)-6-(tributylstannanyl)-7H-indolo[2,1-a][2]benzazepine-10-carboxylate, 7H*-indolo[2,1-a][2]benzazepine-10-carboxylic acid, 13-cyclohexyl-3-methoxy-6-(phenylsulfonyl)-, 1,1-dimethylethyl ester (1.00g, 1.71 mMol) was dissolved in 26mL of benzene along with bis(tributyltin) (2.8mL), 5.54 mMol), tributyltin hydride (136uL, 0.513 mMol) and triethylamine (1.05mL, 7.5 mMol). The solution was sparged for approximately for 10 minutes with nitrogen then 2,2'-bisazoisobutyronitrile (AIBN) (96mg, 0.58mMol) added to the reaction. The reaction was heated to reflux under nitrogen for 2hr. The reaction was followed by LC-MS using the following HPLC conditions: Shimadzu Analytical HPLC using Discovery VP software: %A= 5% acetonitrile, 95% water, 10mmol Ammonium Acetate %B= 95% acetonitrile, 5% water, 10mmol Ammonium Acetate; Initial %B= 0; Final % B=100; Gradient= 3 min; Runtime= 10 min; Flow rate= 5 ml/min; Wavelength= 220nm; Column= Waters Xterra, 3mm x 50mm, S7. To the reaction was added tributyltin hydride (0.45mL, 1.7mMol) and AIBN(95mg, 0.58mMol), the reaction heated to reflux for 2hrs, and analyzed for progress. AIBN (99mg, 0.60mMol) added to the reaction and the reaction heated to rcflux under for an additional 6hrs using a timer. The reaction was analyzed by LC-MS for progress then tributyltin hydride(1.0ml, 3.8mMol) and AIBN (97mg, 0.59mMol) was added and the reaction heated to reflux for 2hrs 20min. The reaction was analyzed by LC-MS and AIBN (97mg, 0.59mMol) added to the reaction. The reaction was heated for 1hr under nitrogen at reflux and the cooled and analyzed by LC-MS. Volatiles were removed *in vacuuo* from the reaction and the reaction was purified by column chromatography using a C₁₈ packing of 190g of YMC GEL ODS-A, 120A spherical 75 uM. The reaction residue (6.67g of yellow oil) was dissolved in a minimum of dichloromethane and the solution applied onto the reverse phase column packed in 10% dichloromethane in acetonitrile. Initial elution was done using 10% dichloromethane in acetonitrile followed by elution with 15% dichloromethane in acetonitrile. The chromatography was monitored by TLC using Whatman MKC18F reverse phase 1"x3" 200uM thickness TLC plates eluting using 15% dichloromethane in acetonitrile. Compound observation was accomplished by UV lamp at 254nm and iodine staining of TLC plates. Product fractions were collected and volatiles removed *in vacuuo* to yield 647mg (52%) as a pale yellow foam. 1H NMR (500 MHz, CHLOROFORM-D) δ ppm 0.71 - 0.83 (m, 9 H) 0.85 - 0.96 (m, 3 H) 0.95 - 1.08 (m, 6 H) 1.15 - 1.27 (m, 7 H) 1.27 - 1.49 (m, 11 H) 1.53 (s, 5 H) 1.60 - 1.67 (m, 9 H) 1.68 - 1.82 (m, 2 H) 1.84 - 1.96 (m, 1 H) 1.96 - 2.16 (m, 3 H) 2.74 - 2.91 (m, 1 H) 3.90 (s, 3 H) 4.16 - 4.40 (m, 1 H) 4.82 - 5.03 (m, 1 H) 6.72 - 6.90 (m, 2 H) 6.96 (dd, *J*=8*.*55, 2.44 Hz, 1H) 7.43 (d, *J*=8.55 Hz, 1 H) 7.66 (dd, *J*=8.39, 1.37 Hz, 1 H) 7.81 (d, *J*=8.55 Hz, 1 H) 8.04 (s, 1 H) LC-MS: Shimadzu Analytical HPLC using Discovery VP software: %A= 5% acetonitrile, 95% water, 10mmol Ammonium Acetate %B= 95% acetonitrile, 5% water, 10mmol Ammonium Acetate; Initial %B= 0; Final % B=100; Gradient= 3 min; Runtime= 10 min; Flow rate= 5 ml/min; Wavelength= 220nm; Column= Waters Xterra, 3mm x 50mm, S7. Retention Time= 4.2min, MS m/z 734(MH⁺).

### 7H-indolo[2,1-a][2]benzazepine-10-carboxylic acid, 13-cyclohexyl-3-methoxy-6-(tributylstannyl)-, 1,1-dimethylethyl ester, 1,1-dimethylethyl 13-cyclihexyl-3-(methyloxy)-6-(tributylstannanyl)-7H-indolo[2,1-a][2]benzazepine-10-carboxylate.

An assembly of a three necked flask fitted with an argon bubbler, reflux condenser and dropping funnel was flame dried and then cooled under a stream of argon. The flask was then charged with benzene (5 mL) and 7H-indolo[2,1-a][2]benzazepine-10-carboxylic acid, 13-cyclohexyl-3-methoxy-6-(phenylsulfonyl)-, 1,1-dimethylethyl ester 1 (500 mg, 0.857 mmol). The resultant mixture was sonnicated under argon for 5 min (to remove oxygen) and then heated under reflux. A solution of tri-N-butyltin hydride (0.459 ml, 1.713 mmol) and 2,2'-azobis(2-methylpropionitrile (52.0 mg, 0.317 mmol) in degassed benzene (5 mL) was then added to the dropping funnel. Approximately 2.5 mL of this solution was added dropwise over a period of approximately 30 min, and the resultant solution was left to stir under reflux for 1.5 h. The remaining solution was added dropwise, slowly over a period of approximately 30 min, and heating was continued for a further 1.5 h. The mixture was then evaporated under reduced pressure to remove volatiles. The residue was slurried in hexane and applied to a silica gel biotage cartridge and then loaded onto a silica gel column equilibrated in 100% hexanes. The product was then eluted using a step gradient of ethyl acetate-hexane: 0 -100%, then 2-98%, then 5-95%. Homogeneous fractions were combined and evaporated to give yellow oil. This was placed under high vacuum overnight to give the title compound as a viscous yellow colored oil. [373 mg, 57%]. The product was stored under nitrogen in a refrigerator.

The scheme depiced below is illustrative of methods that can be used to make intermediates and compounds.

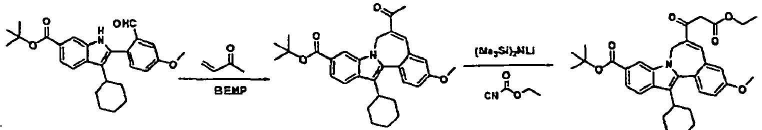

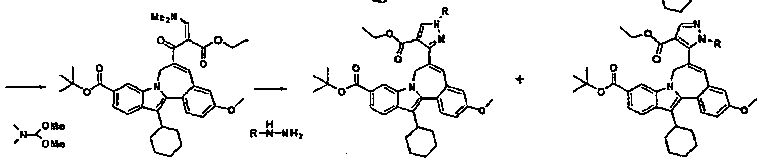

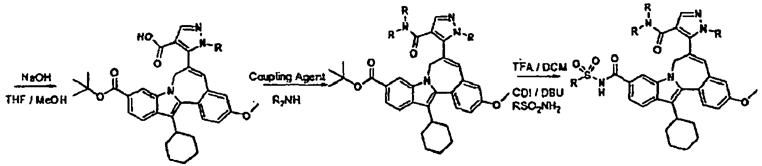

An additional variation in the methodology that can be employed for the synthesis of intermediates and compounds is shown in the following scheme.

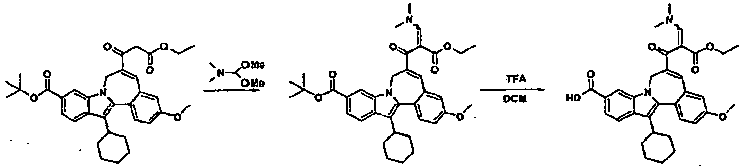

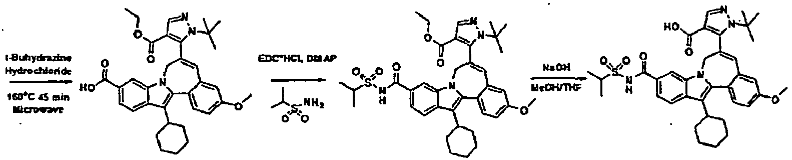

A further variation of the methodology that can be used for the synthesis of further intermediates and compouns is depicted the the scheme shown below.

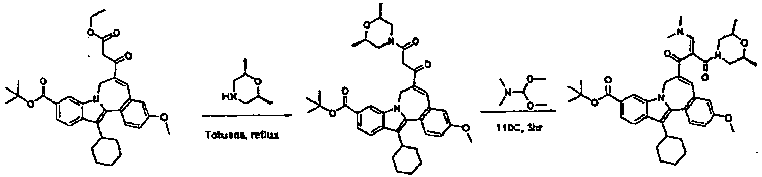

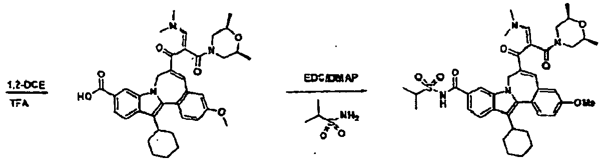

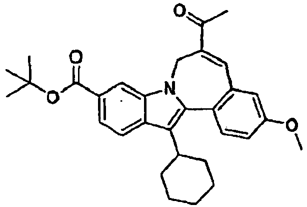

### tert-Butyl 6-acetyl-13-cyclohexyl-3-methoxy-7H-indolo[2,1-a][2]benzazepine-10-carboxylate.

To a suspension of tert-butyl 3-cyclohexyl-2-(2-formyl-4-methoxyphenyl)-IH-indole-6-carboxylate (2 g, 4.61 mmol) in dioxane (9.2 mL) add 2-tert-butylimino-2-diethylamino-1,3-di- methylperhydrodiazaphosphorine (2.00 mL, 6.92 mmol) then but-3-en-2-one (0.756 mL, 9.23 mmol) in a 20ml microwave vessel Cap the heterogeneous reaction under nitrogen and heat in the microwave at 120C for 40 minutes. This reaction was repeated two more times on the above scale and one last time with tert-butyl 3-cyclohexyl-2-(2-formyl-4-methoxyphenyl)-1H-indole-6-carboxylate (2.51g, 5.79mmol) dissolved in dioxane (11.5ml), add BEMP (2.5ml, 8.64 mmol) followed by but-3-en-2-one (0.95ml, 11.46 mmol). Each reaction segment was worked up while the next sequence was running in the microwave. Work up consisted of partitioning the reaction between ethyl acetate and 1N aqueous hydrochloric acid in a 250ml separatory funnel. The aqueous phase was back extracted once with ethyl acetate and the organic phases combined and washed sequentially with 1N aqueous hydrochloric acid, 0.1 M NaH2PO4, and brine. The solution was then combined with a previous workup in a 1L erlenmeyer flask containing magnesium sulfate. Filter and remove volatiles, dry *in vacuuo* at room temperature overnight to obtain 10.67g of a yellow-orange foam. Absorb the crude product onto 25g of silica gel using dichloromethane, removing volatiles *in vacuuo.* Chromatograph on 299g of silica gel slurry packed in 20% ethyl acetate in hexanes and eluting with 20% ethyl acetate in hexanes. Pure product fractions from the chromatography were combined and solvents removed *in vacuuo.* The residue was dissolved in benzene and removed *in vacuuo* to aid in the removal of trace ethyl acetate. The title compound as a yellow foam/amorphous solid was dried *in vacuuo* overnight at room temperature to yield 6.83g (71.7%). 1H NMR (500 MHz, CHLOROFORM-D) δ ppm 1.12 - 1.29 (m, 1 H) 1.30 - 1.61 (m, 4 H) 1.64 (s, 10 H) 1.67 - 2.00 (m, 4 H) 1.98 - 2.19 (m, 3 H) 2.43 (s, 3 H) 2.75 - 2.87 (m, 1 H) 3.85 - 4.01 (m, 4 H) 5.86 (s, 1 H) 7.00 (d, *J*=2.75 Hz, 1 H) 7.11 (dd, *J*=8.55, 2.75 Hz, 1 H) 7.35 (s, 1.8 H, benzene) 7.69 (dd, *J*=8.55, 1.53 Hz, 1 H) 7.82 (d, *J*=8.24 Hz, 1 H) 8.24 (s, 1 H). LC-MS retention time 3.28 min; m/z (MH+). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 30% solvent A / 70% solvent B to 0% solvent A / 100% solvent B, a gradient time of 5min, a hold time of 1 min, and an analysis time of 6 min where solvent A was 10% MeOH / 90% H2O / 0.1% trifluoroacetic acid and solvent B was 10% H2O / 90% MeOH / 0.1% trifluoroacetic acid. MS data was determined using a Micromass Platform for LC in electrospray mode. 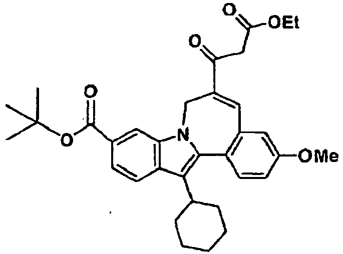 *1H-Indolo[2,1-a][2]benzazepine-6-propanoic acid, 13-cyclohexyl-10-[(1,1-dimethylethoxy)carbonyl]-3-methoxy-beta-oxo-, ethyl ester.* To a solution of 7H-indolo[2,1-a][2]benzazepine-10-carboxylic acid, 6-acetyl-13-cyclohexyl-3-methoxy-, 1,1-dimethylethyl ester (2.50 g, 5.15 mmol) in THF (15.0 mL) was added a 1M solution of LHMDS (5.41 mL, 5.41 mmol) in THF at -78 °C and stirred for 15 min. Ethyl cyanoformate (0.510 mL, 5.15 mmol) was added at -78 °C to the resulting solution and stirring continued for 30 min. Saturated aqueous NH₄Cl (50 mL) was added and the aqueous layer was extracted with CHCl₃ (2 x 50 mL). The organic phase was dried over Na₂SO₄, filtered, and concentrated under reduced pressure. Silica gel chromatography (2:1 methylene chloride:hexanes) of the concentrate afforded the title compound (1.75 g, 61%) as a yellow oil. MS m/z 558 (MH⁺). A previous reaction which was run in an analogous fashion to the above reaction and in which starting material 7H-indolo[2,1-a][2]benzazepine-10-carboxylic acid, 6-acetyl-13-cyclohexyl-3-methoxy-, 1,1-dimethylethyl ester was still present was separated under the following conditions: This procedure describes the separation of tert-butyl 13-cyclohexyl-6-(3-ethoxy-3-oxopropanoyl)-3-methoxy-7H-indolo[2,1-a][2]benzazepine-10-carboxylate (desired product) from the starting material ketone, tert-butyl 6-acetyl-13-cyclohexyl-3-methoxy-7H-indolo[2,1-a][2]benzazepine-10-carboxylate. Total weight of materials was estimated to be 2.6g. 3.9g of an the sample mixture as an orange oil was dissolved in dichloromethane and adsorbed on 10g of silica gel. The volatiles were removed in *vacuuo* using a rotoary evaporator. The sample adsorbed onto silica gel was applied to a column of 294g of silica gel which was slurry packed in 30% diethyl ether in hexanes. A bed of sand was placed on top of the adsorbed sample to aid in solvent addition. The approximate size of the silica gel column packing was 75mm diameter x 175mm in height. The product was eluted using a gradient of 30% diethyl ether in hexanes to 35% diethyl ether in hexanes to 40% diethyl ether in hexanes. Elute with approximately 1L of 30%Et2O/hexanes, then 1L of 35%Et2O/hexanes, and finally using 40%Et2O/hexanes. The volumes of fractions collected were approximately 125ml to 150ml. The product fractions were combined and volatiles removed and the title keto-ester dried in *vacuuo* to yield 1.63g of a yellow-orange amorphous solid. 1H NMR (500 MHz, CHLOROFORM-D) δ ppm 1.12 (t, *J*=7.02 Hz, 2.5 H) 1.15-1.28 (m, 1.6 H) 1.32(q, *J*=7.12Hz, 1.0 H) 1.35-1.43 (m, 1.5 H) 1.59 (s, 1.5 H) 1.61 - 1.69 (m, 9.4 H) 1.72 - 1.82 (m, 2.0 H) 1.94 (s, 1.2 H) 2.04 (s, 2.9 H) 2.76 - 2.85 (m, 1.0 H) 3.64 - 3.79 (m, 0.8 H) 3.88 (s, 0.7 H) 3.90 (s, 0.7 H) 3.92 (s, 2.6 H) 3.94 - 4.06 (m, 0.7 H) 4.12 (q, *J*=7.22 Hz, 1.9 H) 4.21 - 4.33 (m, 0.7 H) 4.39 (q, *J*=7.12 Hz, 0.2 H) 5.67 g, 0.2 H) 5.71 - 5.90 (m, 0.7 H) 6.95 - 7.02 (m, 1.0 H) 7.06 (dd, *J*=8.55, 2.75 Hz, 0.2 H) 7.12 (dd, *J*=8.70, 2.59 Hz, 0.82 H) 7.46 - 7.57 (m, 1.2 H) 7.64 (s, 0.8 H) 7.69 (d, *J*=8.55 Hz, 0.9 H) 7.76 - 7.85 (m, 0.9 H) 8.14 (s, 0.2 H) 8.22 (s, 0.8 H) 12.40 (s, 0.1 H). LC-MS retention time 2.78 min; 556 m/z (MH-). LC data was recorded on a Shimadzu LC-10AS liquid chromatograph equipped with a Waters Xterra MS 7u C18 3.0x50mm column using a SPD-10AV UV-Vis detector at a detector wave length of 220nM. The elution conditions employed a flow rate of 5 ml/min, a gradient of 100% solvent A /0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

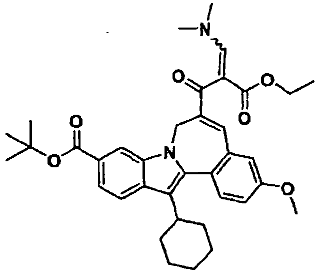

### tert-Butyl 13-cyclohexyl-6-((2E,Z)-3-(dimethylamino)-2-(ethoxycarbonyl)-2-propenoyl)-3-methoxy-7H-indolo[2,1-a][2]benzazepine-10-carboxylate.

tert-Butyl 13-cyclohexyl-6-(3-ethoxy-3-oxopropanoyl)-3-methoxy-7H-indolo[2,1-a][2]benzazepine-10-carboxylate (1.63 g, 2.92 mmol) was dissolved in N,N-dimethylformamide dimethyl acetal (7.0 mL, 52.7 mmol) in a 50ml round bottom flask. The reaction was maintained under a nitrogen atmosphere and heated in oil bath under refluxing conditions (110°C) for 2.75hrs. The reaction was then cooled reaction under a nitrogen atmosphere and the volatiles were evaporated *in vacuuo* to give an orange colored foam. TLC analysis (SiO2 plate, elution- 50% diethyl ether in hexanes) confirmed that the reaction had gone to completion. This material was dried *in vacuuo* at room temperature overnight to yield 1.87g of the enamine intermediate as an orange amber foam which was used in the next step without any further purification. LC-MS of intermediate enamine: LC-MS retention time 2.81 min; 613 m/z (MH+). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A/100% solvent B, a gradient time of 2 min, a hold time of 1 min, and an analysis time of 3 min where solvent A was 10% MeOH / 90% H2O / 0.1% trifluoroacetic acid and solvent B was 10% H2O / 90% MeOH / 0.1% trifluoroacetic acid. MS data was determined using a Micromass Platform for LC in electrospray mode.

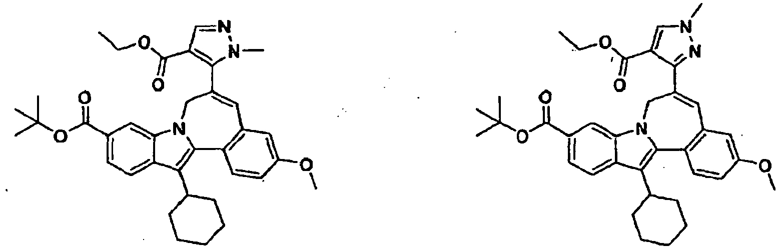

### 7H-indolo[2,1-a][2]benzazepine-10-carboxylic acid, 13-cyclohexyl-6-[4-(ethoxycarbonyl)-1-methyl-1H-pyrazol-5-yl]-3-methoxy-, 1,1-dimethylethyl ester and 7H-indolo[2,1-a][2]benzazepine-10-carboxylic acid, 13-cyclohexyl-6-[4-(ethoxycarbonyl)-1-methyl-1H-pyrazol-3-yl]-3-methoxy-, 1.1-dimethylethyl ester.

The intermediate enamine, tert-butyl 13-cyclohexyl-6-((2E,Z)-3-(dimethylamino)-2-(ethoxycarbonyl)-2-propenoyl)-3-methoxy-7H-indolo[2,1-a][2]benzazepine-10-carboxylate, (1.83 g, 2.99 mmol) was dissolved in absolute ethanol (10 mL). Within a few minutes of dissolution in ethanol the reaction became heterogeneous with a fine yellow/orange precipitate forming. Methylhydrazine (0.173 mL, 3.29 mmol) was added to the reaction and the reaction was placed under a nitrogen atmosphere with a condenser. The reaction was heated to 80C and remained heterogeneous at 80C. An additional 5ml of absolute ethanol was added and the reaction stirred at 80C for 15minutes. The reaction was still heterogeneous and 1,4-dioxane (5ml) was added and the reaction slowly became homogeneous. The reaction was heated for 2 hours then cooled and the volatiles removed *in vacuuo* to obtain an orange amber foam. The residue was dissolved in benzenc and then volatiles removed in vacuuo and the sample was dried at room temperature overnight *in vacuuo* to yield 1.79g of a yellow amorphous solid/foam. LC-MS analysis indicated two possible isomeric products. 102mg of the crude product was purified by reverse phase prep HPLC. The sample was purified on a Shimadzu high pressure liquid chromatography system employing Discovery VP software interfaced with a SCL-10A controller, SIL-10A autosampler and FRC-10A fraction collector. The sample was dissolved in acetonitrile / DMF (1:1) (4ml) purified using a Waters Sunfire Prep C18 OBD, 5uM 19mm x 1.00mm column and monitored using a SPD-10AV UV-Vis detector at a detector wave length of 220nM. The elution conditions employed a flow rate of 25 mL / min, a gradient of 25% solvent A / 75% solvent B to 0% solvent A / 100% solvent B, a gradient time of 10 minutes with a run time of 20 minutes using %A= 10% acetonitrile, 90% water, 0.1% TFA %B= 90% acetonitrile, 10% water, 0.1 % TFA solvent system. First eluting product is 7H-indolo[2,1-a][2]benzazepine-10-carboxylic acid, 13-cyclobexyl-6-[4-(ethoxycarbonyl)-1-methyl-1H-pyrazol-5 -yl]-3-methoxy-, 1,1-dimethylethyl ester with retention time of 11.7minutes with the minor component product being 7H-indolo[2,1-a][2]benzazepine-10-carboxylic acid, 13-cyclohexyl-6-[4-(ethoxycarbonyl)-1-methyl-1H-pyrazol-3-yl]-3-methoxy-, 1,1-dimethylethyl ester with a retention of 14.5 minutes.

*7H-indolo[2,1-a][2]benzazepine-10-carboxylic acid, 13-cyclohexyl-6-[4-(ethoxycarbonyl)-1-methyl-1H-pyrazol-5-yl]-3-methoxy-, 1,1-dimethylethyl ester.* 1H NMR (500 MHz, CHLOROFORM-D) δ ppm 1.13 - 1.27 (m, 1 H) 1.30 (t, *J*=7.02 Hz, 3 H) 1.34 - 1.55 (m, 3 H) 1.58 (s, 9 H) 1.61 - 1.84 (m, 3 H) 1.85 - 2.00 (m, 1 H) 1.99 - 2.18 (m, 3 H) 2.86 (t, *J*=11.75 Hz, 1 H) 3.28 (s, 3 H) 3.90 (s, 3 H) 4.27 (d, *J*=4.27 Hz, 2 H) 4.71 (s, 1 H) 4.97 (s, 1 H) 6.77 (s, 1 H) 6.93 (d, *J*=2.44 Hz, 1 H) 7.07 (dd, *J*=8.55, 2.44 Hz, 1 H) 7.65 (d, *J*=8.55 Hz, 1 H) 7.79 - 7.88 (m, 2 H) 7.97 (s, 1 H). LC-MS retention time 3.92min; 596 m/z (MH+). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min , a gradient of 70% solvent A / 30% solvent B to 0% solvent A / 100% solvent B, a gradient time of 5min, a hold time of I min, and an analysis time of 6 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

*7H-indolo[2,1-a][2]benzazepine-10-carboxylic acid, 13-cyclohexyl-6-[4-(ethoxycarbonyl)-1-methyl-1H-pyrazol-3-yl]-3-methoxy-, 1,1-dymethylethyl ester.* 1H NMR (500 MHz, CHLOROFORM-D) δ ppm 1.13 -1.26 (m, 1 H) 1.30 (t, *J*=7.17 Hz, 3 H) 1.33 - 1.60 (m, 3 H) 1.61 (s, 9 H) 1.64-1.83 (m, 3 H) 1.91 (s, 2 H) 1.98-2.16 (m, 3 H) 2.82 - 2.91 (m, 1 H) 3.90 (s, 3 H) 3.94 (s, 3 H) 4.20 - 4.30 (m, 2 H) 4.41 (d, *J*=14.04 Hz, 1 H) 5.76 (d, *J*=14.04 Hz, 1 H) 6.96 - 7.03 (m, 2 H) 7.49 - 7.54 (m, 1 H) 7.64 (dd, *J*=8.39, 1.37 Hz, 1 H) 7.79 (d, *J*=8.55 Hz, 1 H) 7.84 (s, 1 H) 7.90 (s, 1 H) 8.26 (s, I H). LC-MS retention time 4.26min; 596 m/z (MH+). LC data was recorded on a Shimadzu LC-10AS liquid chromatograph equipped with a Phenomenex-Luna 10u C18 3.0x50mm column using a SPD-10AV UV-Vis detector at a detector wave length of 220nM The elution conditions employed a flow rate of 5 ml/min , a gradient of 70% solvent A / 30% solvent B to 0% solvent A / 100% solvent B, a gradient time of 5min, a hold time of 1 min, and an analysis time of 6 min where solvent A was 5% acetonitrile 95% H20 / 10 mM ammonium acetate and solvent B was 5% H₂O / 95% acetonitrile / 10 mM ammonium acetate. MS data was determined using a Micromass Platform for LC in electrospray mode. The remainder of the sample (1.615g) was adsorbed onto 4g of silica gel using dichloromethane and chromatographed on 50g of silica gel slurry packed using 2% ethyl acetate in dichloromethane and eluting with 2% ethyl acetate in dichloromethane. The first component to elute is the minor product, 7H-indolo[2,1-a][2]benzazepine-10-carboxylic acid, 13-cyclohexyl-6-[4-(ethoxycarbonyl)-1-methyl-1H-pyrazol-3-yl]-3-methoxy-, 1,1-dimethylethyl ester, 179mg isolated. The second component, 7H-indolo[2,1-a][2]benzazepine-10-carboxylic acid, 13-cyclohexyl-6-[4-(ethoxycarbonyl)-1-methyl-1H-pyrazol-5-yl]-3-methoxy-, 1,1-dimethylethyl ester was the major component to elute and yielded 966mg.

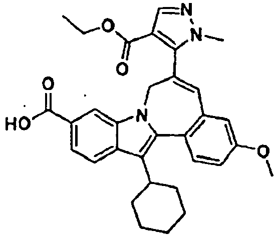

*7H-indolo[2,1-a][2]benzazepine-10-carbozylic acid, 13-cyclohexyl-6-[4-(ethoxycarbonyl)-1-methyl-1H-pyrazol-5-yl]-3-methoxy-.*

7H-indolo[2,1-a][2]benzazepine-10-carboxylic acid, 13-cyclohexyl-6-[4-(ethoxycarbonyl)-1-methyl-1H-pyrazol-5-yl]-3-methoxy-, 1,1-dimethylethyl ester (53 mg, 0.089 mmol) was dissolved in 1,2-dichloroethane (2 mL), under a nitrogen atmosphere. Tifluoroacetic acid was then added (2 ml, 26.0 mmol), and the reaction was stirred at room temperature for 2.5 hours. The volatiles were then removed in vacuuo and the residue dissolved in benzene and the resultant solution was evaporated under reduced pressure to remove residual TFA. This process was repeated once. This gave the title compound as a yellow solid, (46.2mg). 1H NMR (500 MHz, CHLOROFORM-D) δ ppm 1.12 - 1.26 (m, 1 H) 1.31 (t, *J*=7.02 Hz, 3 H) 1.40 (s, 2 H) 1.51 - 1.89 (m, 3 H) 1.87 - 2.23 (m, 4 H) 2.87 (t, *J*=11.29 Hz, 1 H) 3.29 (s, 3 H) 3.91 (s, 3 H) 4.27 (br.s, 2 H) 4.73 (br.s, 1 H) 4.97 (br.s, I H) 6.78 (s, 1 H) 6.94 (d, *J*=2.14 Hz, 1 H) 7.08 (dd, *J*=8.55, 2.44 Hz, 1 H) 7.54 (d, *J*=8.85 Hz, 1 H) 7.77 (d, *J*=8.24 Hz, 1 H) 7.86 - 7.95 (m, 2 H) 7.98 (s, 1 H) 8.36 (br.s, 2 H). LC-MS retention time 1.84 min; 538 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min , a gradient of 70% solvent A / 30% solvent B to 0% solvent A / 100% solvent B, a gradient time of 5min, a hold time of 0 min, and an analysis time of min where solvent A was 5% acetonitrile / 95% H2O / 10 mM ammonium acetate and solvent B was 5% H₂O / 95% acetonitrile / 10 mM ammonium acetate. MS data was determined using a Micromass Platform for LC in electrospray mode.

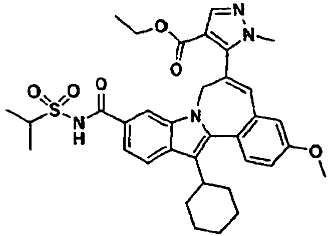

### 1H-pyrazole-4-carboxylic acid 5-[13-cyclohexyl-3-methoxy-10-[[[(1-methylethyl)sulfonyl]amino]carbonyl]-1H-indolo[2,1-a][2]benzazepin-6-yl]-1-methyl-, ethyl ester.

7H-indolo[2,1-a][2]benzazepine-10-carboxylic acid, 13-cyclohexyl-6-[4-(ethoxycarbonyl)-1-methyl-1H-pyrazol-5-yl]-3-methoxy- (981mg, 1.818 mmol) was dissolved in THF (18 mL). Carbonyldiimidazole (649 mg, 4.00 mmol) was added to the reaction. The reaction was placed under a nitrogen atmosphere and stirred at room temperature for 45minutes then heated to reflux for 1 hour. The reaction was cooled under a nitrogen atmosphere and propane-2-sulfonamide (1164 mg, 9.45 mmol) was added to the reaction followed by DBU (0.548 mL, 3.64 mmol). The reaction was then immersed in oil bath at 80°C under nitrogen atmosphere and heated overnight at 70-80°C. The reaction was then diluted with ethyl acetate and the organic layer washed sequentially with 1.0 N aqueous hydrochloric acid, 0.1 M aqueous NaH₂PO₄ and brine. The organic layer was dried over MgSO₄, filtered and volatiles removed *in vacuuo* to yield a yellow foam which was dried *in vacuuo* at room temperature overnight to yield 1.170g of a yellow amorphous solid. Proton NMR analysis revealed the presence of propane-2-sulfonamide (1.41ppm, d, 500MHz, CDC13) in the sample. The crude sample was dissolved in approximately 200ml of dichloromethane and washed 2 x with 125ml of water, then sequentially with 1N aqueous hydrochloric acid, 2 x with 0.1M NaH2PO4 then again with 1N aqueous hydrochloric acid. The dichloromethane solution was dried over sodium sulfate, filtered and volatiles removed and the product dried *in vacuuo* at room temperature overnight to yield 1.046g (89%) of the title compound as a yellow amorphous solid. 1H NMR (500 MHz, CHLOROFORM-D) δ ppm 0.80 - 0.90 (m, 1 H) 1.20 - 1.27 (m, 4 H) 1.30 (t, *J*=7.02 Hz, 4 H) 1.32-1.43 (m, 3 H) 1.46 (d, *J*=7.02 Hz, 6 H) 1.53 - 1.64 (m, 3 H) 1.79 (d, *J*=9.77 Hz, 3 H) 1.85 - 2.13 (m, 5 H) 2.81 - 2.91 (m, 1 H) 3.90 (s, 3 H) 4.00 - 4.10 (m, 1 H) 4.24 (s, 2 H) 4.71 (d, *J*=12.21 Hz, 1 H) 4.99 (d, *J*=16.48 Hz, 1 H) 6.77 (s, 1 H) 6.94 (d, *J*=2.44 Hz, 1 H) 7.08 (dd, *J*=8.55, 2.75 Hz, I H) 7.38 (dd, *J*=8.55, 1.22 Hz, 1 H) 7.52 (d, *J*=8.85 Hz, 1 H) 7.76 (d, *J*=1.22 Hz, 1 H) 7.86 - 7.92 (m, 2 H) 8.26 (br.s, I H). LC-MS retention time 1.79 min; 643 m/z (MH-). LC data was recorded on a Shimadzu LC-10AS liquid chromatography equipped with a Waters Xterra MS 7u C 18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

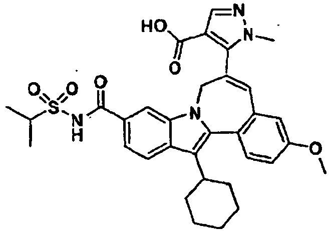

### 1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-methyl-.

1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-methyl-, ethyl ester (1.044 g, 1.619 mmol) was dissolved in THF (L 1 ml) with heating. DMF was then added (5 mL) and the mixture was gently heated to ensure dissolution. The mixture was then cooled to room temperature and tetrabutylammonium hydroxide (6.5 ml, 6.50 mmol) (1.0M in methanol) was added. The resultant mixture was then stirred at room temperature under nitrogen for 2.5hrs and progress was monidered by LC-MS. Results showed predominately methyl and ethyl esters. The mixture was then stirred for an additional 3hrs, after which a further 2.0mL (2mmol) of tetrabutylammonium hydroxide(1.0M in methanol) was added and the reaction was stirred at room temperature under nitrogen for a further 2 days. Subsequent analysis shows reaction had proceeded to approximately 22% conversion to the desired acid product. Volatiles (methanol, ethanol, THF) were removed from the reaction *in vacuuo* (30°C) using a rotary evaporator. To the reaction was added 200uL of 10N aqueous sodium hydroxide and 4 ml of THF. The reaction was mixed by placing on rotary evaporator, gently heated (25°C, 3hrs) then add approximately 6ml ofDMF and heat at 35C for 3hrs and using house vacuum to remove volatiles generated from hydrolysis. Analysis of the reaction by LC-MS indicated approximately 83% conversion to acid product. The reaction was stirred overnight at room temperature and subsequent analysis showed no additional conversion to product. An additional 4.0ml of 1.0M tetrabutylammonium hydroxide in methanol was added to the reaction. The reaction was placed on a rotary evaporator and volatiles removed *in vacuuo* and the reaction continued to heat at 40-45C for approximately 6 hours. The reaction was then worked up by adding 1N aqueous hydrochloric acid (100ml) into the reaction and then the mixture was rinsed into a 500ml sepratory funnel using ethyl acetate bring the organic volume to approx. 200ml. Partition the reaction between ethyl acetate and 1.0N aqueous hydrochloric acid. The organic layer was wash 3x (∼30ml each) with 1.0N aqueous hydrochloric acid, then brine and dried over MgS04, filtered and the volatiles removed *in vacuuo* to yield 1.03g of a yellow film foam. The sample was dried in vacuuo at room temperature to 0.92g (92%) of the title compound. LC-MS retention time 1.48 min; 615 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min , a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

An alterantive procedure for the preparation of the title compound is described below.

Dissolve 1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-methyl-, ethyl ester (1.044 g, 1.619 mmol) in a pre-mixed solution of THF(20 mL), MeOH (20 mL) and sodium hydroxide (20 mL, 20.00 mmol). The reaction was homogenous and was stirred at room temperature under a nitrogen atmosphere for 26hrs then concentrated in vacuuo using a rotary evaporator with a bath temperature at 20C. The reaction was poured into 1N aqueous hydrochloric acid and extract using ethyl acetate. The combined organic layers were washed sequentially with 1N aqueous hydrochloric acid and brine, then dried over magnesium sulfate, filtered and solvent removed *in vacuuo.* The crude product dried *in vacuuo* at room temperature to yield 1.68g of an orange amorphous solid. The crude product was dissolved in chloroform (approximately 50mL) with heating and hexanes were added until some material starts to precipitate but re-dissolves on swirling (approximately 10-12ml of hexanes). The mixture was allowed to slowly cool to room temperature and then allowed to stand at room temperature for a few hours. The very fine particulate yellow precipitate was filtered using a Buchner funnel and dried in vacuuo at room temperature to yield 819mg (45%) of purified product as a bright yellow amorphous solid. The title compound 4.6mg was dissolved in CDCl3 (2ml) with the addition of approximately 5 drops of CD3OD to aid in dissolution for 1H NMR acquisition.
1H NMR (500 MHz, CHLOROFORM-D / CD3OD) δ ppm 1.11 - 1.39 (m, 3 H) 1.41 (d, *J*=7.02 Hz, 6 H) 1.47 - 1.65 (m, 1 H) 1.75 (d, *J*=8.85 Hz, 2 H) 1.82 - 227 (m, 13 H) 2.77 - 2.90 (m, 1 H) 3.28 (s, 3 H) 3.88 (s, 3 H) 3.97 - 4.06 (m, 1 H) 4.66 (s, 1 H) 5.01 (s, 1 H) 6.76 (s, 1 H) 6.92 (d, *J*=2.75 Hz, 1 H) 7.04 (dd, *J*=8.70, 2.59 Hz, 1 H) 7.47 - 7.54 (m, 2 H) 7.81 (d, *J*=1.22 Hz, 1 H) 7.86 (d, *J*=8.54 Hz, 1 H) 7.91 (s, 1 H).

LC-MS retention time 1.39 min; 615 m/z (MH-). LC data was recorded on a Shimadzu LC-10AS liquid chromatograph equipped with a Phenomenex-Luna 10u C183.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where. solvent A was 5% acetonitrile / 95% H2O / 10 mM ammonium acetate and solvent B was 5% H2O / 95% acetonitrile /10 mM ammonium acetate. MS data was determined using a Micromass Platform for LC in electrospray mode.

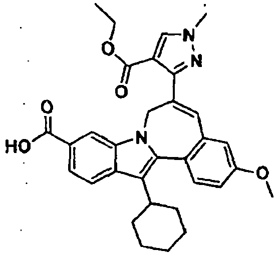

### 7H-indolo[2,1-a][2]benzazepine-10-carboxylic acid, 13-cyclohexyl-6-[4-(ethoxycarbonyl)-1-methyl-1H-pyrazol-3-yl]-3-methoxy-.

Dissolve 7H-indolo[2,1-a][2]benzazepine-10-carboxylic acid, 13-cyclohexyl-6-[4-(ethoxycarbonyl)-1-methyl-1H-pyrazol-3-yl]-3-methoxy-, 1,1-dimethylethyl ester (176 mg, 0.295 mmol) in 1,2-dichloroethane (3 mL), place reaction under a nitrogen atmosphere, then add trifluoroacetic acid (3 ml, 38.9 mmol). Stir the reaction at room temperature under a nitrogen atmosphere for 2hr 20min then remove volatiles *in vacuuo.* Dissolve product in a mixture of benzene and dichloromethane then remove volatiles *in vacuuo.* Repeat dissolution in benzene and dichloromethane and remove volatiles to aid in removal of trace trifluoroacetic acid. The title compound was dried *in vacuuo* at room temperature to yield 164mg of a yellow amorphous solid. 1H NMR (500 MHz, CHLOROFORM-D) δ ppm 1.01 - 1.29 (m, 3 H) 1.32 (t, *J*=7,17 Hz, 3 H) 1.35 - 1.53 (m, 2 H) 1.52-1.85 (m, 3 H) 1.86-2.20 (m, 4 H) 2.79 - 2.95 (m, 1 H) 3.91 (s, 3 H) 3.97 (s, 3 H) 4.27 (q, *J*=7.22 Hz, 2 H) 4.41 (d, *J*=14.34 Hz, 1 H) 5.84 (d, *J*=14.34 Hz, 1 H) 6.97 - 7.08 (m, 2 H) 7.54 (d, *J*=8.24 Hz, 1 H) 7.76 (d, *J*=8.55 Hz, 1 H) 7.85 (d, *J*=8.54 Hz, 1 H) 7.91 (s, 1 H) 7.95 (s, 1 H) 8.50 (s, 1 H). LC-MS retention time 2.08 min; 538 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM The elution conditions employed a flow rate of 5 ml/min , a gradient of 70% solvent A / 30% solvent B to 0% solvent A / 100% solvent B, a gradient time of 5min, a hold time of 1 min, and an analysis time of 6 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

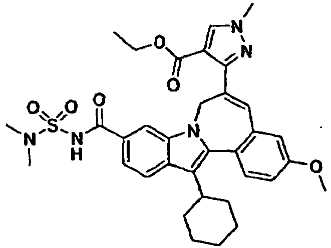

### 1H-pyrazole-4-carboxylic acid, 3-[13-cyclohexyl-10-[[[(dimethylamino)sulfonyl]amino]carbonyl]-3-methoxy-7H-indolo[2,1-a][2]benzazepin-6-yl]-1-methyl-, ethyl ester.

Dissolve 7H-indolo[2,1-a][2]benzazepine-10-carboxylic acid, 13-cyclohexyl-6-[4-(ethoxycarbonyl)-1-methyl-1H-pyrazol-3-yl]-3-methoxy- (60mg, 0.111 mmol) in THF (1.0 mL), then add carbonyl diimidazole (42 mg, 0.259 mmol) to the reaction. The reaction was stirred under a nitrogen atmosphere for 1 hour at room temperature. The reaction was then heated at 70C for 1 hour then cooled and N,N-dimethylsulfamide (70 mg, 0.564 mmol) was added to the reaction followed by DBU (0.034 mL, 0.222 mmol). The reaction was again capped under a nitrogen atmosphere and heated at 70C for 16.5 hours. The reaction was diluted with ethyl acetate and washed sequentially with 1.0N aqueous hydrochloric acid, 0.1M aqueous NaH2PO4 and again with 1.0N aqueous hydrochloric acid, and finally with brine. The organic phase was dried over magnesium sulfate, filtered, solvent removed and the product dried *in vacuuo* at room temperature to yield 70mg of an amorphous yellow solid. 1H NMR (500 MHz, CHLOROFORM-D) δ ppm 0.76 - 0.95 (m, 2 H) 1.12 - 1.26 (m, 5 H) 1.32 (t, *J*=7.17 Hz, 3 H) 1.38 (s, 2 H) 1.51 - 1.61 (m, 4 H) 1.67 - 1.81 (m, 3 H) 1.87 - 2.01 (m, 2 H) 2.01-2.11 (m, 3 H) 2.82 - 2.92 (m, 1 H) 3.06 (s, 6 H) 3.91 (s, 3 H) 3.96 (s, 3 H) 4.26 (d, *J*=7.02 Hz, 2 H) 4.42 (d, *J*=14.34 Hz, 1 H) 5.75 (d, *J*= 14.04 Hz, 1 H) 6.99 - 7.05 (m, 2 H) 7.38 (d, *J*=8.24 Hz, 1 H) 7.52 (d, *J*=8.24 Hz, 1 H) 7.85 (d, *J*=8.55 Hz, 1 H) 7.89 - 7.96 (m, 2 H) 8.18 (s, 1 H) 8.36 (br.s, 1 H). LC-MS retention time 2.34 min; 644 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LG in electrospray mode.

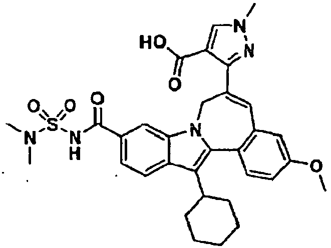

### 1H-pyrazole-4-carboxylic acid, 3-[13-cyclohexyl-10-[[[(dimethylamino)sulfonyl]amino]carbonyl]-3-methoxy-7H-indolo[2,1-a][2]benzazepin-6-yl]-1-methyl-.

1*H*-pyrazole-4-carboxylic acid, 3-[13-cyclohexyl-10-[[[(dimethylamino)sulfonyl]amino]carbonyl]-3-methoxy-7*H*-indolo[2,1-*a*][2]benzazepin-6-yl]-1-methyl-, ethyl ester (66mg, 0.102 mmol) was dissolved in THF (1 mL) and a 1.0M solution of tetrabutylammonium hydroxide (0.41 mL, 0.410 mmol) in methanol was added to the reaction. The reaction was stirred at room temperature under a nitrogen atmosphere for 4hrs then analyzed by LC-MS. The reaction was concentrated in vacuuo on a rotary evaporator then 1 ml of THF was added to the reaction and the reaction once again capped under a nitrogen atmosphere. The reaction was placed in a water bath at 40C and allowed to stir overnight Sometime overnight the heating element on the water bath failed and the reaction was observed to at room temperature the next morning. LC-MS analysis of the reaction indicated the reaction was complete. The reaction was diluted with ethyl acetate and washed sequentially with 1.0N aqueous hydrochloric acid, then again with 1.0N aqueous hydrochloric acid and finally with brine. The organic layer was dried over magnesium sulfate, filtered and solvent removed *in vacuuo.* The title compound was dried *in vacuuo* to yield 63mg of an amorphous yellow solid. 1H NMR (500 MHz, CHLOROFORM-D) δ ppm 0.81- 0.97 (m, 3 H) 1.18 - 1.30 (m, 4 H) 1.30 - 1.65 (m, 6 H) 1.66 - 1.83 (m, 3 H) 1.84 - 2.10 (m, 6 H) 2.84 - 2.92 (m, 3 H) 3.04 (s, 6 H) 3.88 (s, 3 H) 3.97 (s, 3 H) 4.43 (d, *J*=14.34 Hz, 1 H) 5.68 (d, *J*=13.73 Hz, 1 H) 6.99 (d, *J*=2.75 Hz, 1 H) 7.02 (dd, *J*=8.55, 2.44 Hz, 1H) 7.35 (d, *J*=8.24 Hz, 1 H) 7.52 (d, *J*=8.55 Hz, 1 H) 7.77 (s, 1 H) 7.83 (d, *J*=8.24 Hz, 1 H) 7.98 (s, 1 H) 8.15 (s, 1 H) 8.71 (s, 1 H). LC-MS retention time 1.58 min; 616 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min , a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

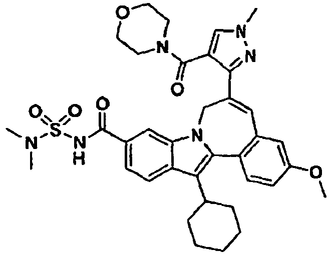

### 7H-indolo[2,1-a][2]benzazepine-10-carboxamide, 13-cyclohexyl-N-[(dimethylamino)sulfonyl]-3-methoxy-6-[1-methyl-4-(4-morpholinylcarbonyl)-1H-pyrazol-3-yl]-.

1*H*-pyrazole-4-carboxylic acid, 3-[13-cyclohexyl-10-[[[(dimethylamino)sulfonyl]amino]carbonyl]-3-methoxy-7*H*-indolo[2,1-*a*][2]benzazepin-6-yl]-1-methyl- (57mg, 0.092 mmol) was dissolved in DMF (1.0ml) and TBTU (55.8 mg, 0.174 mmol) added to the reaction. The reaction was capped under a nitrogen atmosphere and stir at room temperature for 1 hour then DMAP (47 mg, 0.385 mmol) was added to the reaction followed by morpholine (16 µL, 0.184 mmol). The reaction was capped under a nitrogen atmosphere and stirred at room temperature overnight. The reaction was diluted with ethyl acetate and washed sequentially with 1.0N aqueous hydrochloric acid, 0.1M aqueous NaH2PO4 and brine. The organic phase was dried over magnesium sulfate, filtered and solvent removed *in vacuuo.* The title compound was dried overnight *in vacuuo* at room temperature to yield 63 mg of a yellow amorphous solid. The title compound was further purified on a Shimadzu high pressure liquid chromatography system employing Discovery VP software interfaced with a SCL-10A controller, SIL-10A autosampler and FRC-10A fraction collector. The sample was dissolved in acetonitrile / DMF (1:1) (total volume 2ml) purified using a Waters Sunfire Prep C 18 OBD, 5uM 19mm x 100mm column and monitored using a SPD-10AV UV-Vis detector at a detector wave length of 220nM. The elution conditions employed a flow rate of 25 mL / min , a gradient of 60% solvent A / 40% solvent B to 0% solvent A / 100% solvent B, a gradient time of 12 minutes with a run time of 25 minutes using %A= 10% acetonitrile, 90% water, 0.1% TFA %B= 90% acetonitrile, 10% water, 0.1% TFA solvent system. The sample was run as two 1ml injections. The run time of the second Prep HPLC run was truncated to 15 minutes base on data from the first run. The product fractions (retention time= 8.75min.) were combined and solvent removed *in vacuuo.* The compound was dried at room temperature *in vacuuo* to yield 38mg of the title compound as a yellow amorphous solid. 1H NMR (500 MHz, CHLOROFORM-D) δ ppm 1.23 (q, *J*=12.21 Hz, I H) 1.30 - 1.56 (m, 3 H) 1.77 (d, *J*=10.38 Hz, 2 H) 1.90 - 2.14 (m, 4 H) 2.54 - 2.81 (m, 4 H) 2.82 - 2.95 (m, 2 H) 2.95 - 3.05 (m, 2 H) 3.07 (s, 6 H) 3.09 - 3.39 (m, 5 H) 3.90 (s, 3 H) 3.97 (s, 3 H) 4.45 (d, *J*=14.65 Hz, 1 H) 5.45 (d, *J*=14.65 Hz, 1 H) 6.95 (d, *J*=2.75 Hz, 1 H) 6.97 (s, 1 H) 7.04 (dd, *J*=8.70, 2.59 Hz, 1 H) 7.46 (dd, *J*=8.55, 1.53 Hz, 1 H) 7.52 (d, *J*=8.55 Hz, 1 H) 7.55 (s, 1 H) 7.59 (d, *J*=8.55 Hz, I H) 7.94 (d, *J*=1.22 Hz, 1 H) 9.13 (br.s, 1 H). LC-MS retention time 1.82 min; 685 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The clution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

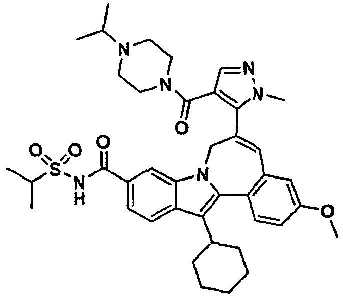

### 7H-indolo[2,1-a][2]benzazepine-10-carboxamide, 13-cyclohexyl-3-methoxy-N-[(1-methylethyl)sulfonyl]-6-[1-methyl-4-[[4-(1-methylethyl)-1-piperazinyl]carbonyl]-1H-pyrazol-5-yl]-.

1*H*-pyrazole-4-carboxylic acid, 5-[13-cyclohexyl-3-methoxy-10-[[[(1-methylethyl)sulfonyl]amino]carbonyl]-7*H*-indolo[2,1-*a*][2]benzazepin-6-yl]-1-methyl- (0.92g, 1.5mmol) was dissolved in DMF (25ml) and HATU (1141 mg, 3.0mmol) was added followed by diisopropylethylamine (1306uL, 7.5mmol). A 1mL solution of the reaction solution containing 1*H*-pyrazole-4-carboxylic acid, 5-[13-cyclohexyl-3-methoxy-10-[[[(1-methylethyl)sulfonyl]amino]carbonyl]-7*H-*indolo[2,1-*a*][2]benzazepin-6-yl]-1-methyl- / HATU / DIEA was transferred a 16x100mm wheaton vial containing 1-isopropylpiperazine (36.9mg, 0.288mmol). The reaction was capped and shaken at room temperature overnight. The reaction was transferred into a 96well plate and the reaction vial rinsed with DMF (800uL) to bring the final reaction volume to 1.800mL. The reaction mixture was purified by reverse phase HPLC under the following conditions. The Prep HPLC was controlled by Dionex Chromeleon 6.70 spl LC software using Varian a prostar binary pump with 50 mL/min pump head and detection using a Sedex 75 ELS detector for fraction collection. A Dionex UVD340U UV spectrometer was used to observe the UV trace of the HPLC run. A Waters Sunfire C18 19mm x 25mm 10u was employed for the isolation using a solvent system of A= water and 20mM ammonium acetate, B= acetonitrile. The following gradient was used: 80%A and 20%B hold for 3 minutes followed by a 19 minute gradient to 5%A and 95%B with a final bold at 5%A and 95%B of 5 minutes. The flow rate for purification was 20ml/ min. The fractions of interest were collected and concentrated to dryness using a Zymark Turbo Vap Evaporator. NMR analysis was performed on the peaks of interest (analytical retention time 5.69 minutes and 6.30minutes). The title compound was determined to be the peak with analytical retention time of 6.30 minutes by NMR analysis (rotomers). 1H NMR (500 MHz, CHLOROFORM-D) δ ppm 0.17 (s, 0.5 H) 0.91 (t, *J*=6.87 Hz, 3.3 H) 1.10 (t, *J*=14.95 Hz, 1.2 H) 1.17 - 1.32 (m, 2.6 H) 1.33 - 1.59 (m, 13.1 H) 1.68 - 1.83 (m, 3.5 H) 1.88 - 2.18 (m, 8.7H) 2.20 - 2.67 (m, 7.8 H) 2.76 - 3.05 (m, 3.3 H) 3.23 (d, *J*=10.99 Hz, 1.3 H) 3.29 - 3.53 (m, 1.6 H) 3.63 - 3.86 (m, 2.7 H) 3.88 - 3.98 (m, 6.0 H) 4.02 - 4.10 (m, 1.0 H) 4.61 (d, *J*=14.65 Hz, 1.3 H) 4.89 (d, *J*=14.95 Hz, 0.9 H) 6.76 - 6.86 (m, 0.8 H) 6.91 - 7.00 (m, 1.9 H) 7.06 - 7.13 (m, 1.3 H) 7.51 - 7.57 (m, 1.4 H) 7.57 - 7.66 (m, 1.6 H) 7.66 - 7.75 (m, 1.9 H) 7.89 (d, *J*=8.55 Hz, 1.5 H). LCMS analysis using MassLynx 4.0 SP4 LC-MS software, a CTC-Leap HTS-PAL autosampler, with an Agilent 1100 binary pump, Agilent 1100 photodiode array UV detector, Polymer Lab 2100 ELS detector (Temp=45C, nebulizer temp 3 5C) and a Waters ZQ With ESCi mass spectrometer. The analysis was performed using a Phenomenex Gemini 4.6x 150mm C18 3u column with a mobile phase solvent system of A= water and 20mM ammonium acetate, B= acetonitrile with a flow rate of 1.0ml/min and a gradient starting at 70%A and 30%B and a final composition 5%A and 95%B, gradient time of 11minutes and a bold time of 2 minutes to give an analysis runtime of 13 minutes.

Retention time = 6.63 minutes, m/z= 725 (MH+).

The following compounds were synthesized by an analogous method as described above for 7*H*-indolo[2,1-*a*][2]benzazepine-10-carboxamide, 13-cyclohexyl-*N-*[(dimethylamino)sulfonyl]-3-methoxy-6-[1-methyl-4-(4-morpholinylcarbonyl)-1*H-*pyrazol-3-yl]- and 7*H*-indolo[2,1-*a*][2]benazepine-10carboxamide, 13-cyclohexyl-3-methoxy-*N*-[(1-methylethyl)sulfonyl]-6-[1-methyl-4-[[4-(1-methylethyl)-1-piperazinyl]carbonyl]-1*H*-pyrazol-5-yl]-. Analytical LCMS data on the following examples were acquired using the hollowing column and conditions: Gradient: 6 minutes; Flow rate: 4 mL/min; Stop time: 6 minutes; Eluent A: 5% CH₃CN / 95% H₂O with 10 mM NH₄OAc; Eluent B: 95% CH₃CN / 5% H₂O with 10 mM NH₄OAc; Initial %B= 0; Final % B=100; Column: Phenomenex Luna 4.6mm x 50mm S5.

### 13-cyclohexyl-6-(1-ethyl-4-(((2R)-2-(methoxymethyl)-4-morpholinyl)carbonyl)-1H-pyrazol-5-yl)-N-(isopropylsulfonyl)-3-methoxy-7H-indolo[2,1-a][2]benzazepine-10-carboxamide:

LCMS: m/e 745 (M+H), ret time 4.90 min.

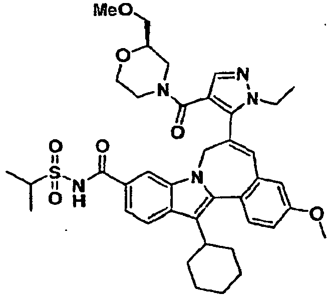

### 7H-indolo[2,1-a][2]benzazepine-10-carboxamide, 13-cyclohexyl-6-[1-ethyl-4-(3-oxa-9-azabicyclo[3.3.1]non-9-ylcarbonyl)-1H-pyrazol-5-yl]-3-methoxy-N-[(1-methylethyl)sulfonyl]-:

LCMS: m/e 741 (M+H), ret time 5.80 min.

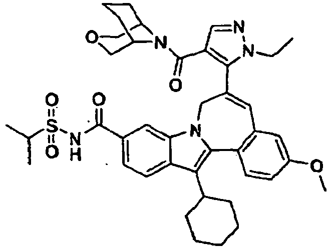

### 7H-indolo[2,1-a][2]benzazepine-10-carboxamide, 13-cyclohexyl-6-[1-ethyl-4-(2-oxa-5-azabicyclo[2.2.1]hept-5-ylcarbonyl)-1H-pyrazol-5-yl]-3-methoxy-N-[(1-methylethyl)sulfonyl]-:

LCMS: m/c 713 (M+H), ret time 4.81 min.

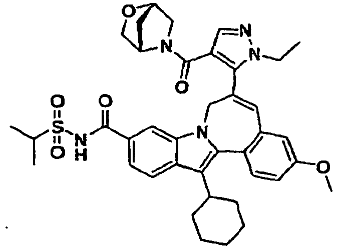

### 7H-indolo[2,1-a][2]benzazepine-10-carboxamide, 13-cyclohexyl-6-[1-ethyl-4-[[(3R,5S)-3,4,5-trimethyl-1-piperazinyl]carbonyl]-1Hpyrazol-5-yl]-3-methoxy-N-[(1-methylethyl)sulfonyl]-:

LCMS: m/e 742 (M+H), ret time 4.64 min.

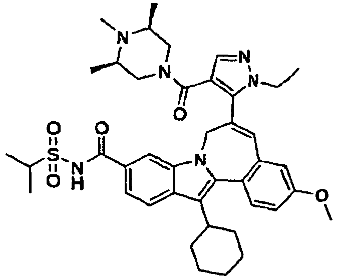

### 7H-indolo[2,1-a][2]benzazepine-10-carboxamide, 13-cyclohexyl-6-[1-ethyl-4-[(3-methyl-3,8-diazabicyclo[3.2.1]oct-8-yl)carbonyl]-1H-pyrazol-5-yl]-3-methoxy-N-[(1-methylethyl)sulfonyl]-:

LCMS: m/e 740 (M+H), ret time 5.25 min.

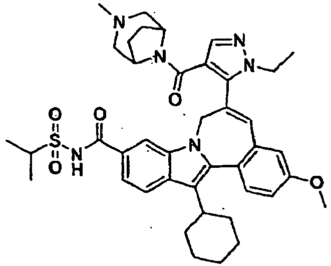

### 7H-indolo[2,1-a][2]benzazepine-10-carboxamide, 13-cyclohexyl-6-[1-ethyl-4-[(5-ethyl-2,5-diazabicyclo[2.2.1]hept-2-yl)carbonyl]-1H-pyrazol-5-yl]-3-methoxy-N-[(1-methylethyl)sulfonyl]-:

LCMS: m/e 740 (M+H), ret time 4.44 min.

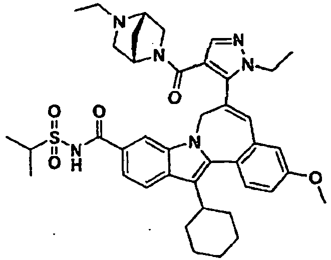

### 7H-indolo[2,1-a][2]benzazepine-10-carboxamide, 13-cyclohexyl-3-methoxy-N-[(1-methylethyl)sulfonyl]-6-[4-(8-oxa-3-azabicyclo[3.2.1]oct-3-ylcarbonyl)-1-(2,2,2-trifluoroethyl)-1H-pyrazol-5-yl]-:

LCMS: m/e 780 (M+H), ret time 5.49 min.

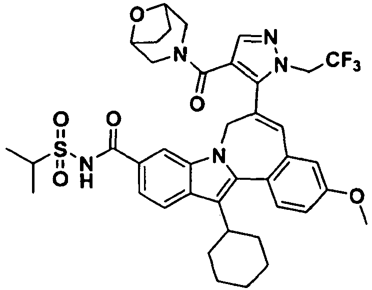

### 7H-indolo[2,1-a][2]benzazepine-10-carboxamide, 13-cyclohexyl-6-[4-(3,7-dioxa-9-azabicyclo[3.3.1]non-9-ylcarbonyl)-1-(2,2,2-trifluoroethyl)-1Hpyrazol-5-yl]-3-methoxy-N-[(1-methylethyl)sulfonyl]-:

LCMS: m/e 796 (M+H), ret time 5.12 min.

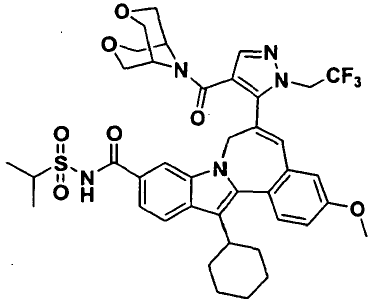

### 7H-indolo[2,1-a][2]benzazepine-10-carboxamide, 13-cyclohexyl-3-methoxy-N-[(1-methylethyl)sulfonyl]-6-[4-(2-oxa-5-azabicyclo[2.2.1]hept-5-ylcarbonyl)-1-(2,2,2-trifluoroethyl)-1H-pyrazol-5-yl]-:

LCMS: m/e 766 (M+H), ret time 5.40 min.

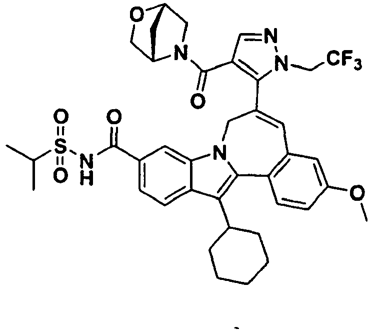

### 7H-indolo[2,1-a][2]benzazepine-10-carboxamide, 13-cyclohexyl-3-methoxy-N-[(1-methylethyl)sulfonyl]-6-[4-[(4-methyl-1-piperazinyl)carbonyl]-1-(2,2,2-trifluoroethyl)-1H-pyrazol-5-yl]-:

LCMS: m/e 767 (M+H), ret time 4.86 min.

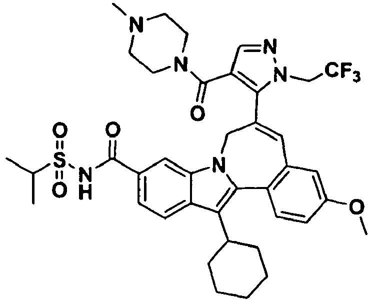

### 13-cyclohexyl-6-(1-isopropyl-4-(8-oxa-3-azabicyclo[3.2.1]oct-3-ylcarbonyl)-1H-pyrazol-5-yl)-N-(isopropylsulfonyl)-3-methoxy-7H-indolo-[2,1-a][2]benzazepine-10-carboxamide:

LCMS: m/e 740 (M+H), ret time 5.17 min.

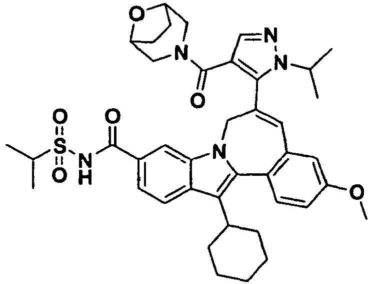

### 13-cyclohexyl-6-(1-isopropyl-4-(3-oxa-9-azabicyclo[3.3.1]non-9-ylcarbonyl)-1H-pyrazol-5-yl)-N-(isopropylsulfonyl)-3-methoxy-7H-indolo[2,1-a][2]benzazepine-10-carboxamide:

LCMS: m/e 754 (M+H), ret time 5.94 min.

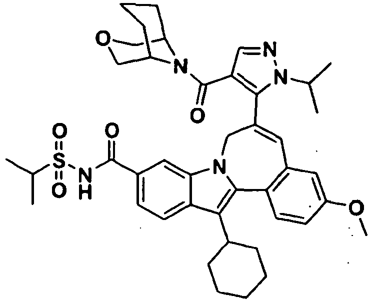

### 13-cyclohexyl-6-(1-isopropyl-4-(2-oxa-5-azabicyclo[2.2.1]hept-5-ylcarbonyl)-1H-pyrazol-5-yl)-N-(isopropylsulfonyl)-3-methoxy-7H-indolo[2,1-a][2]benzazepine-10-carboxamide:

LCMS: m/e 726 (M+H), ret time 4.96 min.

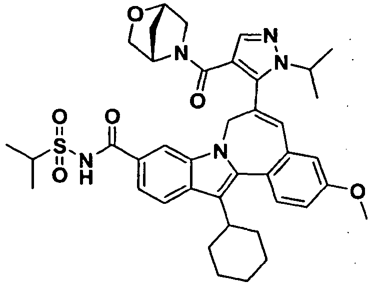

### 13-cyclohexyl-6-(1-isopropyl-4-((4-methyl-1-piperazinyl)carbonyl)-1H-pyrazol-5-yl)-N-(isopropylsulfonyl)-3-methoxy-7H-indolo[2,1-a][2]benzazepine-10-carboxamide:

LCMS: m/e 727 (M+H), ret time 4.61 min.

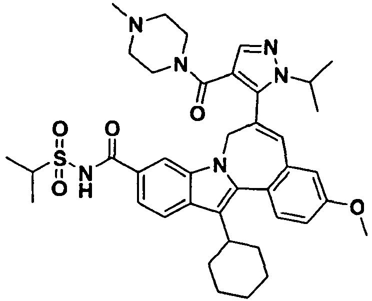

### 13-cyclohexyl-6-(1-isopropyl-3-methyl-4-(8-oxa-3-azabicyclo[3.2.1]oct-3-ylcarbonyl)-1H-pyrazol-5-yl)-N-(isopropylsulfonyl)-3-methoxy-7H-indolo[2,1-a][2]benzazepine-10-carboxamide:

LCMS: m/e 754 (M+H), ret time 5.20 min.

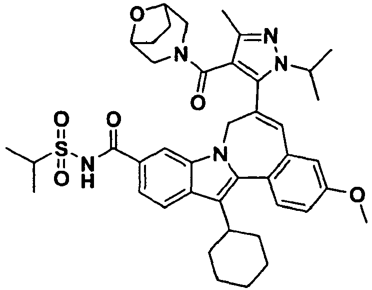

### 13-cyclohexyl-6-(1-isopropyl-3-methyl-4-(2-oxa-5-azabicyclo[2.2.1]hept-5-ylcarbonyl)-1H-pyrazol-5-yl)-N-(isopropylsulfonyl)-3-methoxy-7H-indolo[2,1-a][2]benzazepine-10-carboxamide:

LCMS: m/e 740 (M+H), ret time 5.10 min.

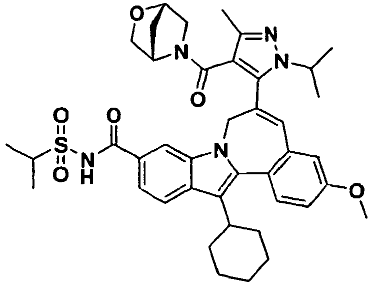

### 7H-indolo[2,1-a][2]benzazepine-10-carboxamide, 13-cyclohexyl-3-methoxy-N-[(1-methylethyl)sulfonyl]-6-[3-methyl-4-[(3-methyl-3,8-diazabicyclo[3.2.1]oct-8-yl)carbonyl]-1-(1-methylethyl)-1H-pyrazol-5-yl]-:

LCMS: m/e 767 (M+H), ret time 5.70 min. 1H NMR (500 MHz, DMSO/CHLOROFORM-D) □ ppm 1.10-1.57 (m, 17 H) 1.75 - 2.16 (m, 9 H) 2.35 (s, 3 H) 2.65 (m, 2 H) 2.91 (m, I H) 2.96 (s, 3 H) 3.24 (m, 4 H) 3.92-3.95 (m, 5 H) 4.67 (br d, 1 H) 5.02 (br d, 1 H) 7.10 (br m, 1 H) 7.16 (s, 1 H) 7.25 (m, 1 H) 7.59 (d, *J*=8.24 Hz, 1 H) 7.67 (d, *J*=8.55 Hz, 1 H) 7.94 (d, *J*=8.24 Hz, 1 H) 8.00 (s, 1 H) 8.17 (s, 1 H).

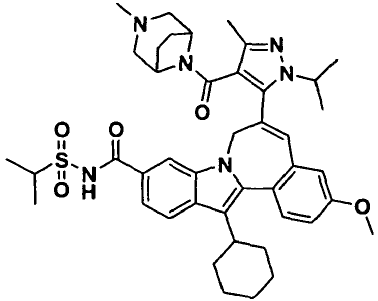

### 13-cyclohexyl-6-(4-((5-ethyl-2,5-diazabicyclo[2.2.1]hept-2-yl)carbonyl)-1-isopropyl-3-methyl-1H-pyrazol-5-yl)-N-(isopropylsulfonyl)-3-methoxy-7H-indolo[2,1-a][2]benzazepine-10-carboxamide:

LCMS: m/e 767 (M+H), ret time 4.44 min. 1H NMR (500 MHz, DMSO/CHLOROFORM-D) □ ppm 1.10-1.57 (m, 18 H) 1.75 - 2.16 (m, 9 H) 2.29 (m, 4 H) 2.91 (m, 1 H) 2.96 (s, 3 H) 3.24 (m, 4 H) 3.92-3.96 (m, 5 H) 4.66 (br d, 1 H) 5.05 (br d, 1 H) 7.10 (br m, 1 H) 7.30 (m, 2 H) 7.58 (d, *J*=8.24 Hz, 1 H) 7.67 (br m, 1 H) 7.95 (br d,1 H) 8.00 (s, 1 H) 8.14 (br s, 1 H).

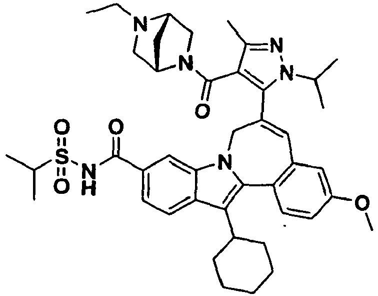

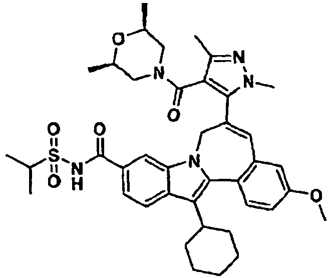

*7H-indolo[2,1-a][2]benzazepine-10-carboxamide, 13-cyclohexyl-6-[1,3-dimethyl-4-[[cis-2,6-dimethyl- 4-morpholinyl]carbonyl]-1H-pyrazol-5-yl]-3-methoxy-N-[(1-methylethyl)sulfonyl]-.* To a solution of 1*H*-pyrazole-4-carboxylic acid, 5-[13-cyclohexyl-3-methoxy-10-[[[(1-methylethyl)sulfonyl]amino]carbonyl]-7*H*-indolo[2,1-*a*][2]benzazepin-6-yl]-1,3-dimethyl- (13 mg, 0.021 mmol) in DMSO (1 mL), TBTU (13.24 mg, 0.041 mmol) and DIPEA (0.018 mL, 0.103 mmol) were added. The reaction mixture was stirred at RT for 15 min. Then cis-2,6-dimethylmorpholine (3.56 mg, 0.048 mmol) was added and the resultant mixture was stirred at RT overnight The crude reaction mixture was purified by preparative HPLC using CH₃CN-H₂O-TFA as a solvent system. Homogeneous fractions were combined and concentrated under vacuum to afford the title compound as a yellow colored solid, (10.1 mg, 0.014 mmol, 67.3 % yield). MS m/z 326(M-H⁻), Retention time: 1.958 min. (basic). 1H NMR (500 MHz, CHLOROFORM-D) δ ppm 0.57 - 2.33 (m, 25 H) 2.81 - 3.60 (m, 5 H) 3.77 - 3.99 (m, 8 H) 4.03 - 4.12 (m, 1 H) 4.57 (d, *J*=15.26 Hz, 1 H) 4.76 - 4.94 (m, 1 H) 6.75 - 6.88 (m, 1 H) 6.95 (s, 1 H) 7.12 (dd, *J*=8.55, 2.14 Hz, 1 H) 7.50 - 7.67 (m, 2 H) 7.71 (s, 1 H) 7.91 (d, *J*=8.24 Hz, 1 H).

The following compounds were synthesized by an analogous method as described above for 7H-indolo[2,1-a][2]benzazepine-10-carboxamide, 13-cyclohexyl-6-[1,3-dimethyl-4-[[cis-2,6-dimethyl-4-morpholinyl]carbonyl]-1H-pyrazol-5-yl]-3-methoxy-N-[(1-methylethyl)sulfonyl]-:

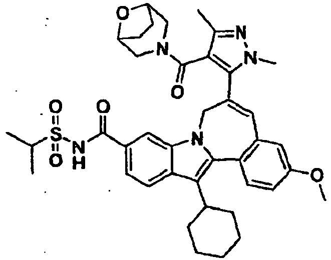

### 7H-indolo[2,1-a][2]benzazepine-10-carboxamide, 13-cyclohexyl-6-[1,3-dimethyl-4-(8-oxa-3-azabicyclo[3.2.1]oct-3-ylcarbonyl)-1H-pyrazol-5-yl]-3-methoxy-N-[(1-methylethyl)sulfonyl]-.

MS m/z 726(M+H). 1H NMR (500 MHz, CHLOROFORM-D) δ ppm 0.70 - 2.32 (m, 23 H) 2.75 - 3.69 (m, 5 H) 3.81 - 3.99 (m, 8 H) 4.03 (m, 1 H) 4.55 (br d, 1 H) 4.90 (br d, 1 H) 6.75 (s, 1 H) 6.96 (s, 1 H) 7.14 (m, 1 H) 7.51 - 7.65 (m, 2 H) 7.72 (s, 1 H) 7.87 (d, *J*=8.24 Hz, 1 H) 10.12 (br s, 1 H).

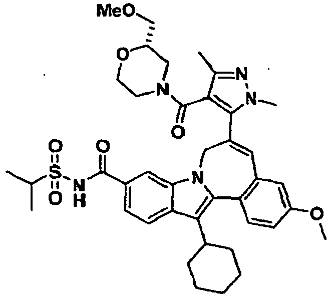

### 7H-indolo[2,1-a][2]benzazepine-10-carboxamide, 13-cyclohexyl-3-methoxy-6-[4-[[(2S)-2-(methoxymethyl)-4-morpholinyl]carbonyl]-1,3-dimethyl-1H-pyrazol-5-yl]-N-[(1-methylethyl)sulfonyl]-.

MS m/z 744 (M+H). 1H NMR (500 MHz, CHLOROFORM-D) δ ppm 1.19 - 2.52 (m, 21 H) 2.85 - 3.59 (m, 11H) 3.85 - 3.95 (m, 6 H) 4.07 (m, I H) 4.60 (br d, I H) 4.93 (br d, 1 H) 6.86 (br s, 1 H) 6.97 (s, 1 H) 7.16 (m, 1 H) 7.53 - 7.69 (br m, 2 H) 7.22 (br m, 1 H) 7.94 (br m, 1 H) 10.20 (br s, 1 H).

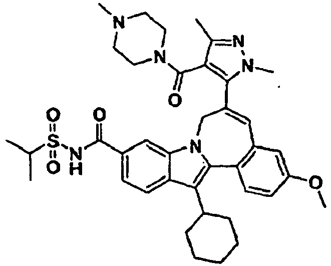

### 7H-indolo[2,1-a][2]benzazepine-10-carboxamide, 13-cyclohexyl-6-[1,3-dimethyl-4-[(4-methyl-1-piperazinyl)carbonyl]-1H-pyrazol-5-yl]-3-methoxy-N-[(1-methylethyl)sulfonyl]-.

MS m/z 713 (M+H). 1H NMR (500 MHz, CHLOROFORM-D) δ ppm 1.13 - 2.26 (m, 19 H) 2.39 - 3.71 (m, 12 H) 3.75 - 3.86 (m, 6 H) 4.03 (m, 1 H) 4.61 (br d, 1 H) 4.83 (br d, 1 H) 6.96 (m, 2 H) 7.13 (m, 1 H) 7.53 (d, *J*=8.24 Hz, 1 H) 7.61 - 7.82 (m, 2 H) 7.92 (d, *J*=8.24 Hz, 1 H) 10.20 (br s, 1 H).

### 7H-indolo[2,1-a][2]benzazepine-10-carboxamide, 13-cyclohexyl-3-methoxy-N-[(1-methylethyl)sulfonyl]-6-[1-methyl-3-(1-methylethyl)-4-(4-morpholinylcarbonyl)-1H-pyrazol-5-yl]-

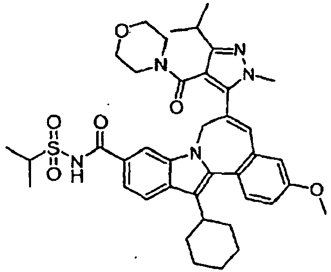

To a solution of 1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-methyl-3-(1-methylethyl)- (50 mg, 0.076 mmol) in DMSO (0.760 mL), TBTU (49 mg, 0.152 mmol) and DIPEA (0.039 mg, 0.30 mmol) were added. The reaction mixture was stirred at RT for 15 min. Then morpholine (26 mg, 0.304 mmol) was added. The solution was stirred at RT for overnight. The reaction fixture was purified by prep HPLC column using CH₃CN/H₂O/TFA as solvent system. Fractions were collected and concentrated under speedvac overnight to yield the title compound as a yellow solid (26 mg, 0.035 mmol, 47 % yield).
MS m/z 728 (M-H⁺)

1H NMR (500 MHz, CHLOROFORM-D) δ ppm 1.15 - 1.62 (m, 16 H) 1.72 - 2.25 (m, 7 H) 2.53 - 3.36 (m, 9 H) 3.90 (s, 3 H) 3.94 (s, 3 H) 4.07 (m, 1 H) 4.60 (br d, 1 H) 4.94 (br d, 1 H) 6.82 (d, *J*=2.44 Hz, I H) 6.96 (s, 1 H) 7.14 (dd, *J*=8.55, 2.75 Hz, 1 H) 7.57 (d, *J*=8.55 Hz, 1 H) 7.64 (m, 1 H) 7.76 (s, 1 H) 7.96 (d, *J*=8.55 Hz, 1 H) 10.56 (br s, 1 H).

The following compounds were synthesized by an analogous method as described above for 7H-indolo[2,1-a][2]benzazepine-14-carboxamide, 13-cyclohexyl-3-methoxy-N-[(1-methylethyl)sulfonyl]-6-[1-methyl-3-(1-methylethyl)-4-(4-morpholinylcarbonyl)-1H-pyrazol-5-yl]-:

### 7H-indolo[2,1-a][2]benzazepine-10-carboxamide, 13-cyclohexyl-6-[4-[[(2R,6S)-2,6-dimethyl-4-morpholinyl]carbonyl]-1-methyl-3-(1-methylethyl)-1H-pyrazol-5-yl]-3-methoxy-N-[(1-methylethyl)sulfonyl]-

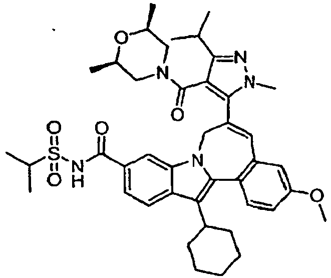

MS m/z 756 (MH⁺). 1H NMR (500 MHz, CHLOROFORM-D) δ ppm 0.70 (m, 6 H) 1.05 - 1.62 (m, 16 H) 1.72 - 2.28 (m, 7 H) 2.83 - 3.21 (m, 5 H) 3.90-3.94 (m, 8 H) 4.08 (m, 1 H) 4.59 (br d, 1 H) 4.96 (br d, 1 H) 6.79 (d, *J*=2.44 Hz, 1 H) 6.96 (s, 1 H) 7.14 (dd, *J*=8.55, 2.75 Hz, 1 H) 7.58 (d, *J*=8.55 Hz, 1 H) 7.69 (m, 1 H) 7.81 (s, 1 H) 7.96 (d, *J*=8.55 Hz, 1 H) 10.70 (br s, 1 H).

### 7H-indolo[2,1-a][2]benzazepine-10-carboxamide, 13-cyclohexyl-3-methoxy-N-[(1-methylethyl)sulfonyl]-6-[1-methyl-3-(1-methylethyl)-4-[(4-methyl-1-piperazinyl)carbonyl]-1H-pyrazol-5-yl]-

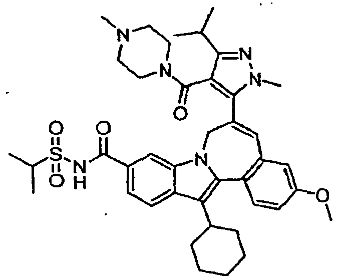

MS m/z 741 (MH⁺). 1H NMR (500 MHz, CHLOROFORM-D) δ ppm 1.12- 1.70 (m, 16 H) 1.70 - 2.12 (m, 7 H) 233 (br s, 3 H) 2.70 - 3.32 (m, 7 H) 3.45-4.07 (m, 9 H) 4.61 (br s, 1 H) 4.97 (br s, 1 H) 6.89 (br s, 1 H) 7.96 (s, 1 H) 7.13 (br m, 1 H) 7.50-7.86 (br m, 3 H) 7.94 (d, *J*=8.55 Hz, 1 H) 10.30 (br s, 1 H).

### 7H-indolo[2,1-a][2]benzazepine-10-carboxamide, 13-cyclohexyl-3-methoxy-N-[(1-methylethyl)sulfonyl]-6-[1-methyl-3-(1-methylethyl)-4-[[(3R,5S)-3,4,5-trimethyl-1-piperazinyl]carbonyl]-1H-pyrazol-5-yl]-

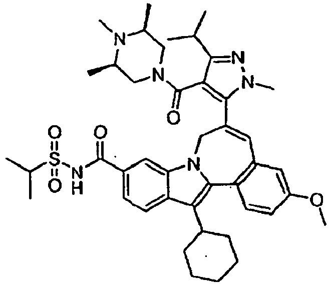

MS m/z 769 (MH⁺). 1H NMR (500 MHz, CHLOROFORM-D) δ ppm 1.05 (m, 6 H) 1.05 - 1.62 (m, 16 H) 1.72 - 2.13 (m, 7 H) 2.28 (br s, 3 H) 2.83 - 3.48 (m, 5 H) 3.79-4.11 (m, 9 H) 4.62 (br d, 1 H) 4.99 (br d, 1 H) 6.86 (br s, 1 H) 6.97 (s, 1 H) 7.18 (br s, 1 H) 7.60-7.81 (br m, 3 H) 7.95 (d, *J*=8.55 Hz, 1 H) 10.32 (br s, 1 H).

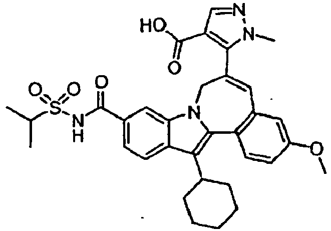

### 1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-methyl-

Dissolve 1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-methyl-, ethyl ester (1.044 g, 1.619 mmol) in a pre-mixed solution of THF(20 mL), MeOH (20 mL) and sodium hydroxide (20 mL, 20.00 mmol). The reaction was homogenous and was stirred at room temperature under a nitrogen atmosphere for 26brs then concentrated in vacuuo using a rotary evaporator with a bath temperature at 20C. The reaction was poured into 1N aqueous hydrochloric acid and extract using ethyl acetate. The combined organic layers were washed sequentially with 1N aqueous hydrochloric acid and brine, then dried over magnesium sulfate, filtered and solvent removed *in vacuuo.* The crude product dried *in vacuuo* at room temperature to yield 1.68g of an orange amorphous solid. The crude product was dissolved in chloroform (approximately 50mL) with heating and hexanes were added until some material starts to precipitate but re-dissolves on swirling (approximately 10-12ml of hexanes). The mixture was allowed to slowly cool to room temperature and then allowed to stand at room temperature for a few hours. The very fine particulate yellow precipitate was filtered using a Buchner funnel and dried in vacuuo at room temperature to yield 819mg (45%) of purified product as a bright yellow amorphous solid. The title compound 4.6mg was dissolved in CDCl3 (2ml) with the addition of approximately 5 drops of CD30D to aid in dissolution for 1H NMR acquisition.

1H NMR (500 MHz, CHLOROFORM-D / CD3OD) δ ppm 1.11 - 1.39 (m, 3 H) 1.41 (d, *J*=7.02 Hz, 6 H) 1.47 - 1.65 (m, 1 H) 1.75 (d, *J*=8.85 Hz, 2 H) 1.82 - 2.27 (m, 13 H) 2.77 - 2.90 (m, 1 H) 328 (s, 3 H) 3.88 (s, 3 H) 3.97 - 4.06 (m, 1 H) 4.66 (s, 1 H) 5.01 (s, 1 H) 6.76 (s, 1 H) 6.92 (d, *J*=2.75 Hz, 1 H) 7.04 (dd, *J*=8.70, 2.59 Hz, 1 H) 7.47 - 7.54 (m, 2 H) 7.81 (d, *J*=1.22 Hz, 1 H) 7.86 (d, *J*=8.54 Hz, 1 H) 7.91 (s, 1 H).

LC-MS retention time 1.39 min; 615 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

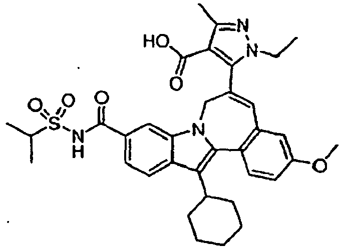

### 1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-

1*H*-pyrazole-4-carboxylic acid, 5-[13-cyclohexyl-3-methoxy-10-[[[(1-methylethyl)sulfonyl]amino]carbonyl]-7*H*-indolo[2,1-*a*][2]benzazepin-6-yl]-1-ethyl-3-methyl-, methyl ester (127mg, 0.193 mmol) was dissolved in THF (3ml) and methanol (3 ml) added to the solution.. Sodium hydroxide (3 ml, 3.00 mmol, 1.0N aqueous solution) was added to the homogeneous yellow solution reaction. The reaction turned to a rose-reddish cloudy color after complete addition of sodium hydroxide solution. The reaction was stirred at room temperature under a nitrogen atmosphere and eventually became a clear rose colored solution. The reaction was stirred at room temperature under a nitrogen atmosphere. HPLC analysis after 40hours revealed 78% conversion to product. Sodium hydroxide (1.0ml, 1.00mmol, 1.0N aqueous solution) was added to the reaction and the reaction stirred under a nitrogen atmosphere for an additional 24hrs. The reaction was concentrated to dryness *in vacuuo* using a rotary evaporator with bath at ambient temperature. The pink solid residue was partitioned between ethyl acetate and 1.0N aqueous hydrochloric acid. The aqueous phase was extracted with ethyl acetate and the organic layers combined and washed sequentially with 1.0N aqueous hydrochloric acid and brine. The organic layer was dried over magnesium sulfate, filtered and solvents removed *in vacuuo* to give a yellow solid which was dried *in vacuuo* at room temperature to give 108mg of the title compound as a amorphous yellow solid.

1H NMR (500 MHz, CHLOROFORM-D) δ ppm 0.71 (s, 3 H) 0.81 - 0.94 (m, 2 H) 0.96 - 1.20 (m, 3 H) 1.23 - 1.27 (m, 3 H) 1.27 - 1.37 (m, 3 H) 1.41 (t, *J*=7.17 Hz, 6 H) 1.45 - 1.53 (m, 2 H) 1.60 - 1.73 (m, 1 H) 1.77 (d, *J*=11.90 Hz, 2 H) 1.86 - 2.02 (m, 2 H) 2.04 - 2.08 (m, 1 H) 2.47 (s, 2 H) 2.77 - 2.89 (m, 1 H) 3.43 - 3.69 (m, 4 H) 3.70 - 3.86 (m, 2 H) 3.99 - 4.06 (m, 2 H) 4.61 (d, *J*=10.07 Hz, 1 H) 4.95 (d, *J*=13.73 Hz, 1 H) 6.75 (s, 1 H) 6.94 (d, *J*=2.44 Hz, 1 H) 7.03 - 7.10 (m, 1 H) 7.42 (d, *J*=8.24 Hz, 1 H) 7.47 - 7.54 (m, 1 H) 7.79 - 7.91 (m, 2 H) 8.76 (s, 1 H).

LC-MS retention time 1.50 min; 643 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzazepine-10-carboxylic acid, 13-cyclohexyl-6-[4-(ethoxycarbonyl)-1-ethyl-3-methyl-1H-pyrazol-5-yl]-3-methoxy-

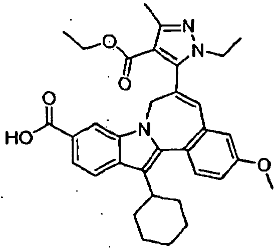

7H-indolo[2,1-a][2]benzazepine-10-carboxylic acid, 13-cyclohexyl-6-[4-(ethoxycarbonyl)-1-ethyl-3-methyl-1H-pyrazol-5-yl]-3-methoxy-, 1,1-dimethylethyl ester (2.134 g, 3.42 mmol) was dissolved in 1,2-dichloroethane (17 mL) then TFA (17 mL) was added and the reaction stirred at room temperature under a nitrogen atmosphere for 2hrs. Volatiles were removed from the reaction *in vacuuo* using a rotary evaporator. The orange-amber reaction residue was dissolved in dichloromethane and benzene added. The solvents and residual TFA was removed in vacuuo using the rotary evaporator. The product was dried in vacuuo at room temperature to give 2.309g of an amorphous yellow solid. Analysis of the product by 1H NMR and 19F NMR indicated TFA still present The product was then dissolved in ethyl acetate and washed with 1.0N aqueous hydrochloric acid. The aqueous phases were combined and back extracted with ethyl acetate. The organic extracts were combined and washed with brine, dried over magnesium sulfate, filtered and volatiles removed *in vacuuo* to yield to give a yellow foam which was dissolved in dichloromethane and benzene and volatiles again removed *in vacuuo* using the rotary evaporator. The product, as a light yellow amorphous solid, was dried in *vacuuo* to give 1.962 g of product The product was sufficiently pure to use without further purification.

1H NMR showed a broad baseline shift (4.5 to 5ppm, water, H+ from acid function group) causing larger integration values for the bridge methylene protons.

1H NMR (500 MHz, CHLOROFORM-D) δ ppm 0.63 (s, 3 H) 1.12 - 1.60 (m, 9 H) 1.78 (d, *J*=9.16 Hz, 2 H) 1.95 (s, 1 H) 2.00 - 2.19 (m, 3 H) 2.50 (s, 3 H) 2.86 (t, *J*=11.90 Hz, I H) 3.51 (d, *J*=34.79 Hz, 2 H) 3.90 (s, 3 H) 4.19 - 4.40 (m, 2 H) 4.68 (d, *J*=14.65 Hz, 2 H) 4.96 (d, *J*=12.21 Hz, 2 H) 6.73 (s, I H) 6.93 (d, *J*=2.14 Hz, 1 H) 7.07 (dd, *J*=8.5, 2.14 Hz, 1 H) 7.53 (d, *J*=8.85 Hz, 1 H) 7.76 (d, *J*=8.55 Hz, 1 H) 7.88 (d, *J*=8.55 Hz, 1 H) 7.96 (s, 1 H).

LC-MS retention time2.94 min; 568m/z (MH+). LC data was recorded on a Shimadzu LC-10AS liquid chromatograph equipped with a Waters Sunfire C18 5u 4.6x50mm column using a SPD-10AV UV-Vis detector at a detector wave length of 220nM. The elution conditions employed a flow rate of 4 ml/min , a gradient of 100% solvent A / 0% solvent B to 0% solvent A / 100% solvent B, a gradient time of 3 min, a hold time of 2 min, and an analysis time of 5 min where solvent A was 10% acetonitrile / 90% H2O / 0.1% TFA and solvent B was 90% H2O / 10% acetonitrile / 0.1% TFA. MS data was determined using a Micromass Platform for LC in electrospray mode.

### 1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-, ethyl ester

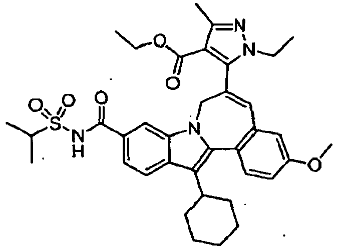

7H-indolo[2,1-a][2]benzazepine-10-carboxylic acid, 13-cyclohexyl-6-[4-(ethoxycarbonyl)-1-ethyl-3-methyl-1H-pyrazol-5-yl]-3-methoxy- (1.965g, 3.46 mmol) was dissolved in dichloromethane (35 mL). Propane-2-sulfonamide (1.415 g, 11.49 mmol) and DMAP (1.355 g, 11.09 mmol) were added to the reaction followed by EDC (1.046g, 5.46 mmol). The reaction was stirred under a nitrogen atmosphere for 2 days.

Remove volatiles/solvent from reaction *in vacuuo* using a rotary evaporator. Partition the reaction between ethyl acetate and 1.0N aqueous hydrochloric acid. Wash organic layer sequentially with 1.0N aqueous hydrochloric acid, 0.1N aqueous NaH2PO4 and brine. Dry organic layer over magnesium sulfate, filter and remove solvent *in vacuuo* to obtain a yellow foam which was dried *in vacuuo* at room temperature to give 2.12g of crude product as an amber-orange amorphous foam. Dissolve the crude product in dichloromethane and adsorb onto 5.3g of silica gel. The product was purified by column chromatography on 71g of silica gel slurry loaded in 2% methanol in dichloromethane. The product was eluted with 2% methanol in dichloromethane and after collecting 40 18x150mm fraction tubes 3% methanol in dichloromethane. The pure product fractions were combined and solvent removed in vacuuo using a rotary evaporator. The title compound (1.482g) was obtained as a amorphous yellow solid.

1H NMR (500 MHz, CHLOROFORM-D) δ ppm 0.66 (s, 3 H) 1.12 - 1.44 (m, 6 H) 1.46 (d, *J*=7.02 Hz, 6 H) 1.49 - 1.55 (m, *J*=19.99, 6.56 Hz, 1 H) 1.55 - 1.63 (m, 4 R) 1.78 (d, *J*=9.16 Hz, 2 H) 1.87 - 2.17 (m, 4 H) 2.48 (s, 3 H) 2.80 - 2.92 (m, 1 H) 3.37 - 3.66 (m, 2 H) 3.90 (s, 3 H) 3.99 - 4.09 (m, 1 H) 4.15 - 4.35 (m, 1 H) 4.68 (d, *J*=14.04 Hz, 1 H) 4.95 (d, *J*=13.73 Hz, 1 H) 6.74 (s, 1 H) 6.94 (d, *J*=2.44 Hz, I H) 7.07 (dd, *J*=8.55, 2.44 Hz, 1 H) 7.37 (d, *J*=8.24 Hz, 1 H) 7.51 (d, *J*=8.54 Hz, 1 H) 7.77 (s, 1 H) 7.88 (d, *J*=8.24 Hz, 1 H) 8.29 (s, 1 H).

LC-MS retention time 2.02min; 671m/z (MH-). LC data was recorded on a Shimadzu LC- 10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 1H-pyrazole-4-carboxylic acid, 5-[10-(aminocarbonyl)-13-cyclohexyl-3-methoxy-7H-indolo[2,1-a][2]benzazepin-6-yl]-1-ethyl-3-methyl-, ethy/ ester

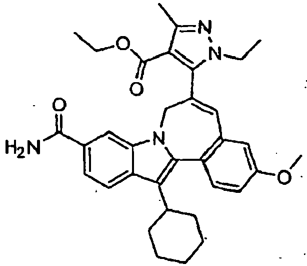

The title compound was isolated as a minor impurity in the purification of 1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-, ethyl ester in the above example. 1H NMR had indicated that ammonium chloride as an impurity contained in reactant propane-2-sulfonamide was most likely responsible for the formation to the title compound. The title compound, 1H-pyrazole-4-carboxylic acid, 5-[10-(aminocarbonyl)-13-cyyclohexyl-3-methoxy-7H-indolo[2,1-a][2]benzazepin-6-yl]-1-ethyl-3-methyl-, ethyl ester, trailed the major product, 4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-, ethyl ester of the reaction in the above chromatography conditions. The title compound (153mg) was isolated as a light yellow solid.

1H NMR (500 MHz, CHLOROFORM-D) δ ppm 0.64 (s, 2 H) 0.80 - 0.97 (m, 1 H) 1.13 - 1.57 (m, 7 H) 1.60 (s, 3 H) 1.78 (d, *J*=10.07 Hz, 2 H) 1.95 (s, 1 H) 2.08 (s, 3 H) 2.47 (s, 3 H) 2.80 - 2.92 (m, 1 H) 3.50 (d, *J*=39.67 Hz, 2 H) 3.90 (s, 3 H) 4.28 (s, 2 H) 4.67 (d, *J*=13.43 Hz, 1 H) 4.98 (d, *J*=12.21 Hz, 1 H) 5.58 (s, 1 H) 6.01 (s, 1 H) 6.72 (s, 1 H) 6.93 (d, *J*=2.14 Hz, 1 H) 7.06 (dd, *J*=8.55, 2.44 Hz, 1 H) 7.34 (d, *J*=8.24 Hz, I H) 7.52 (d, *J*=8.85 Hz, 1 H) 7.80 (s, 1 H) 7.85 (d, *J*=8.55 Hz, 1 H).

NMR results for sample in DMSO were not satisfactory. All peaks were broad (including solvent peak). Likewise NMR taken in acetonirtile exhibited broad peak shape.

1H NMR (500 MHz, ACETONITRILE-D3) δ ppm 0.55 (s, 2 H) 0.78 - 1.00 (m, 1 H) 1.10 - 1.35 (m, 4 H) 1.38 - 1.58 (m, 3 H) 1.69 - 1.83 (m, 2 H) 2.03 - 2.13 (m, 5 H) 2.15 (s, 3 H) 2.41 (s, 3 H) 2.78 - 2.92 (m, 1 H) 3.48 (s, 2 H) 3.90 (s, 3 H) 4.21 (d, *J*=30.21 Hz, 2 H) 4.62 (s, 1 H) 5.01 (s, 1 H) 5.82 (s, 1 H) 6.67 (s, 1 H) 6.84 (s, 1 H) 7.05 (d, *J*=2.75 Hz, 1 H) 7.13 (dd, *J*=8.55, 2.75 Hz, 1 H) 7.47 (dd, *J*=8.39, 1.37 Hz, 1 H) 7.59 (d, *J*=8.55 Hz, 1 H) 7.74 (s, 1 H) 7.89 (d, *J*=8.24 Hz, 1 H).

LC-MS retention time 2.19 min; 567 m/z (MH+). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzazepine-10-carboxamide, 13-cyclohexyl-6-[1-ethyl-3-methyl-4-[(3-methyl-3,8-diazabicyclo[3.2.1]oct-8-yl)carbonyl]-1H-pyrazol-5-yl]-3-methoxy-N-[(1-methylethyl)sulfonyl]-

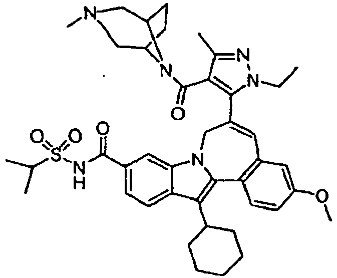

1*H*-pyrazole-4-carboxylic acid, 5-[13-cyclobexyl-3-methoxy-10-[[[(1-methylethyl)sulfonyl]amino]carbonyl]-7*H*-indolo[2,1-*a*][2]benzazepin-6-yl]-1-ethyl-3-methyl- (40.8 mg, 0.063 mmol) was dissolved in DMF (633 µL) and HATU (49.2 mg, 0.129 mmol) added to the reaction. The reaction was stirred for 1hr at room temperature under a nitrogen atmosphere then DMAP (39.2 mg, 0.321 mmol) was added to the reaction followed by the amine reagent, 3-methyl-3,8-diazabicyclo[3.2.1]octane dibydrochloride (29.5 mg, 0.148 mmol). The reaction was capped under a nitrogen atmosphere and stirred at room temperature for 19hr. The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile purified using a Waters Sunfire Prep C18 OBD, 5uM 19mm x 150mm column and monitored using a SPD-10AV UV -Vis detector at a detector wave length of 220nM. The elution conditions employed a flow rate of 25 mL / min, a gradient of 50% solvent A / 50% solvent B to 0% solvent A / 100% solvent B, a gradient time of 25 minutes with a run time of 30 minutes using %A= 10% acetonitrile, 90% water, 0.1% TFA %B= 90% acetonitrile, 10% water, 0.1% TFA solvent system. Retention time of product was 4.34min. Volatiles were removed from the product fraction in vacuuo using a Speed-vac using a medium heat setting. The product was transferred to a vial in acetonitile, solvent removed using a nitrogen sweep then the sample was dried *in vacuuo* at room temperature to yield 32.3mg of the title compound as a yellow amorphous solid. 1H NMR exhibits broad peaks characteristic of salt formation and/or restricted rotation.

1H NMR (500 MHz, CHLOROFORM-D) δ ppm 0.87 (s, 0.2 H) 1.24 (s, 2 H) 1.32 - 1.63 (m, 9 H) 1.79 (d, *J*=10.99 Hz, 2 H) 1.88 - 2.15 (m, 4 H) 2.19 - 2.75 (m, 12 H) 2.80 - 2.99 (m, 2 H) 3.11 (s, 2 H) 3.42 (d, *J*=70.19 Hz, 3 H) 3.80 - 3.90 (m, 1 H) 3.94 (s, 3 H) 4.03 (s, 1 H) 4.20 (s, 2 H) 4.57 (d, *J*=15.56 Hz, 1 H) 4.81 (s, 1 H) 6.98 (s, 1 H) 7.12 (d, *J*=8.55 Hz, 1 H) 7.54 (d, *J*=7.93 Hz, 1 H) 7.63 (s, 1 H) 7.91 (d, *J*=8.24 Hz, 1 H) 10.16 (s, 1 H).

LC-MS retention time2.04 min; 751m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzazepine-10-carboxamide, 13-cyclohexyl-6-[1-ethy/-3-methyl-4-[[(3R,5S)-3,4,5-trimethyl-1-piperazinyl]carbonyl]-1H-pyrazol-5-yl]-3-methoxy-N-[(1-methylethyl)sulfonyl]-

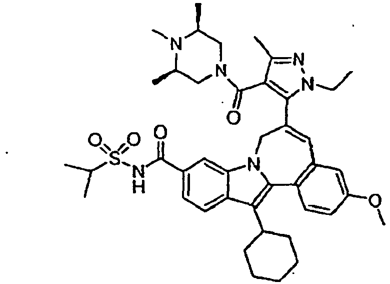

1*H*-pyrazole-4-carboxylic acid, 5-[13-cyclohexyl-3-methoxy-10-[[[(1-methylethyl)sulfonyl]amino]carbonyl]-7*H*-indolo[2,1-*a*][2]benzazepin-6-yl]-1-ethyl-3-methyl- (39.8 mg, 0.062 mmol) was dissolved in DMF (617 µL) and HATU (47.9 mg, 0.126 mmol) added to the reaction. The reaction was stirred for 1hr at room temperature under a nitrogen atmosphere then DMAP (47.7 mg, 0.390 mmol) was added to the reaction followed by the amine reagent, (2R,6S)-1,2,6-trimethylpiperazine dihydrochloride (29.4 mg, 0.146 mmol). The reaction was capped under a nitrogen atmosphere and stirred at room temperature for 19hr. The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min, a gradient of 70 solvent A / 30% solvent B to 0% solvent A / 100% solvent B, a gradient time of 25 minutes with a run time of 30 minutes using %A= 10% acetonitrile, 90% water, 0.1% TFA %B= 90% acetonitrile, 10% water, 0.1% TFA solvent system.

Retention time of product is 8.08min, Remove volatiles from product fractions *in vacuuo* overnight using speed-vac at medium heating setting. The product fraction were combined in acetonitrile, transferred into a vial and solvent removed using a nitrogen sweep. The product was dried overnight at room temperature to yield 32.5mg of the title compound as a yellow amorphous solid

1H NMR (500 MHz, CHLOROFORM-D) δ ppm 0.27 (s, 1.5 H) 1.04 (s, 6 H) 1.21 - 1.46 (m, 6 H) 1.50 (s, 6 H) 1.81 (d, *J*=9.77 Hz, 2 H) 1.90 - 2.13 (m, 4 H) 2.25 (s, 3 H) 2.36 (s, 2 H) 2.92 (s, 8 H) 3.08 - 3.65 (m, 5 H) 3.84 (s, 1 H) 3.94 (s, 3 H) 4.00 - 4.10 (m, 1 H) 4.15 (s, 2 H) 4.60 (d, *J*=14.34 Hz, 1 H) 4.72 - 4.98 (m, 2 H) 6.89 (s, 1 H) 6.95 (d, *J*=2.44 Hz, 1 H) 7.13 (d, *J*=7.63 Hz, 1 H) 7.56 (d, *J*=7.32 Hz, 1 H) 7.61 - 7.88 (m, 2 H) 7.94 (d, *J*=8.55 Hz, 1 H) 10.19 (s, 1 H).

LC-MS retention time1.26 min; 753 m/z (MH-). LC data was recorded on a Shimadzu LC- 10AS liquid chromatograph equipped with a Phenomenex-Luna 10u C183.0x50mm column using a SPD-10AV UV-Vis detector at a detector wave length of 220nM The elution conditions employed a flow rate of 5 ml/min , a gradient of 100% solvent A / 0% solvent B to 0% solvent A / 100% solvent B, a gradient time of 2 min, a hold time of 1 min, and an analysis time of 3 min where solvent A was 5% acetonitrile / 95% H2O / 10 mM ammonium acetate and solvent B was 5% H2O 95% acetonitrile / 10 mM ammonium acetate. MS data was determined using a Micromass Platform for LC in electrospray mode.

### 7H-indolo[2,1-a][2]benzazepine-10-carboxamide, 13-cyclohexyl-6-[1-ethyl-3-methyl-4-[(1S,4S)-4-2-oxa-5-azabicyclo[2.2.1]hept-5-ylcarbonyl]-1H-pyrazol-5-yl]-3-methoxy-N-[(1-methylethyl)sulfonyl]-

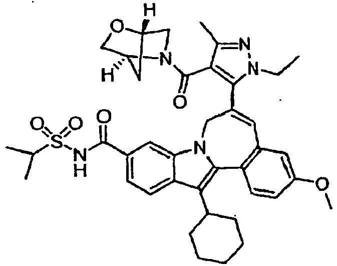

1*H*-pyrazole-4-carboxylic acid, 5-[13-cyclohexyl-3-methoxy-10-[[[(1-methylethyl)sulfonyl]amino]carbonyl]-7*H*-indolo[2,1-*a*][2]benzazepin-6-yl]-1-ethyl-3-methyl- 41.7mg, 0.065 mmol) was dissolved in DMF (647 µL) and HATU (49.8 mg, 0.131 mmol) added to the reaction. The reaction was stirred for 1hr at room temperature under a nitrogen atmosphere then DMAP (53.5 mg, 0.438 mmol) was added to the reaction followed by the amine reagent, (1S,4S)-(+)-2-Aza-5-oxabicyclo(2.2.1)-heptane hydrochloride (22.6 mg, 0.167 mmol). The reaction was capped under a nitrogen atmosphere and stirred at room temperature for 19hr. The product was purified on a Shimadzu high pressure liquid chromatography system employing Discovery VP software interfaced with a SCL-10A controller, SIL-10A autosampler and FRC-10A fraction collector. The reaction was diluted to 2ml with

acetonitrile and a few drops of water purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min, a gradient of 70 solvent A /30% solvent B to 0% solvent A / 100% solvent B, a gradient time of 25 minutes with a run time of 35 minutes using %A= 10% acetonitrile, 90% water, 0.1 % TFA %B= 90% acetonitrile, 10% water, 0.1% TFA solvent system. Retention time of product is 18.9min. Remove volatiles from product fractions *in vacuuo* overnight using speed-vac at medium heating setting. The product fraction was dissolved in dichloromethane and transferred into a vial and solvent removed using a nitrogen sweep. The product was dried overnight at room temperature to yield 3 1.0mg of the title compound as a yellow amorphous solid.

1H NMR data shows characteristics of restricted rotation with broadening and splitting of some peaks.

1H NMR (500 MHz, CHLOROFORM-D) δ ppm 0.15 (s, 0.6 H) 0.94 (s, 0.4 H) 1.08 - 1.34 (m, 2.6 H) 1.34 - 1.60 (m, 12.4 H) 1.79 (d, *J*=10.38 Hz, 2.1 H) 1.97 (d, *J*=9.77 Hz, 1.3 H) 2.00 = 2.16 (m, 3.0 H) 2.29 (s, 3.0 H) 2.71 (d, *J*=6.71 Hz, 0.2 H) 2.81 - 2.94 (m, 1.4 H) 3.03 (d, *J*=11.90 Hz, 1.0 H) 3.09 - 3.67 (m, 8.7 H) 3.83 - 4.02 (m, 4.4 H) 4.01 - 4.11 (m, 1.4 H) 4.13 - 4.31 (m, 2.2 H) 4.53 - 4.65 (m, 1.0 H) 4.92 (dd, *J*=15.26, 5.49 Hz, 0.9 H) 6.84 (d, *J*=18.62 Hz, 0.6 H) 6.89 - 6.97 (m, 1.5 H) 7.12 (t, *J*=8.09 Hz, 1.0 H) 7.55 (q, *J*=7.73 Hz, 1.0 H) 7.59 - 7.66 (m, 0.8 H) 7.67 - 7.76 (m, 0.9 H) 7.83 (s, 0.3 H) 7.86 - 7.99 (m, 1.0 H) 10.41 10.82 (m, 0.8 H).

LC-MS retention time 1.71 min; 724 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile /10 mM ammonium acetate. MS data was determined using a Micromass Platform for LC in electrospray mode.

The compounds below were synthesized by identical coupling methodology used in the above examples.

### 7H-indolo[2,1-a][2]benzozepine-10-carboxamide, 13-cyclohexyl-6-[1-ethyl-4-[(5-ethyl-2,5-diazabicyclo[2.2.1]hept-2-yl)carbonyl]-3-methyl-1H-pyrazol-5-yl]-3-methoxy-N-[(1-methylethyl)sulfonyl]-

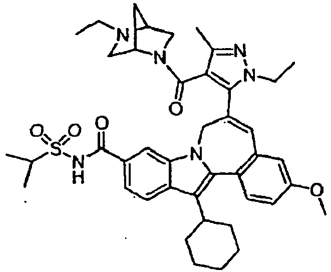

### HPLC purification method:

The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile with a couple drops of TFA and water, filtered through a 0.45uM syringe filter and purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min , a gradient of 70 solvent A / 30% solvent B to 0% solvent A / 100% solvent B, a gradient time of 20 minutes with a run time of 20 minutes using %A= 10% acetonitrile, 90% water, 0.1% TFA %B= 90% acetonitrile, 10% water, 0.1% TFA solvent system. The product was collected between 6.62minutes and 8.81 minutes.

The title compound was isolated (86.7mg) as a yellow amorphous solid.

1H NMR is characteristic of restricted rotation and/or salt formation with splitting/broadening of peaks- appears rotomeric.

1H NMR (500 MHz, CHLOROFORM-D) δ ppm 0.29 - 0.58 (m, 0.5 H) 0.87 (s, 0.2 H) 0.96 - 1.07 (m, 0.2 H) 1.17 (s, 2.1 H) 1.23 - 1.34 (m, 1.2 H) 1.34 - 1.57 (m, 10.7 H) 1.58 - 1.74 (m, 1.0 H) 1.81 (d, *J*=9.46 Hz, 1.9. H) 1.96 (d, *J*=14.34 Hz, 1.1H) 2.04 (d, *J*=8.55 Hz, 2.8 H) 2.19 - 2.34 (m, 3.1 H) 2.60 - 2.98 (m, 8.3 H) 3.03 - 3.57 (m, 3.7 H) 3.59 - 3.87 (m, 1.9 H) 3.94 (s, 3.0 H) 4.03 (s, 0.8 H) 4.09 - 4.36 (m, 2.3 H) 4.58 (t, *J*=15.26 Hz, 1.0 H) 4.78 (d, *J*=14.34 Hz, 0.9 H) 6.90 - 7.23 (m, 3.0 H) 7.45 - 7.67 (m, 2.6 H) 7.92 (d, *J*=7.63 Hz, 0.9 H) 8.32 (s, 0.1 H) 9.29 (s, 0.1 H) 9.92 - 10.87 (m, 0.95 H).

LC-MS retention time 1.73 min; 751m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A /0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzazepine-10-carboxamide, 13-cyclohexyl-6-[1-ethyl-3-methyl-4-[(8-methyl-3,8-diazabicyclo[3.2.1]oct-3-yl)carbonyl]-1H-pyrazol-5yl]-3-methoxy-N-[(1-methylethyl)sulfonyl]-

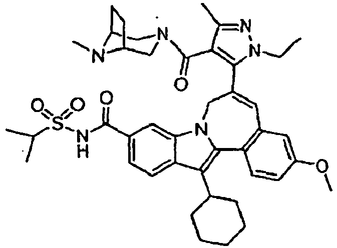

### HPLC purification method:

The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile with the addition of a couple drops of TFA and water then filtered through a 0.45uM syringe filter and purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min, a gradient of 70 solvent A / 30% solvent B to 0% solvent A /100% solvent B, a gradient time of 20 minutes with a run time of 20 minutes using %A= 10% acetonitrile, 90% water, 0.1% TFA %B= 90% acetonitrile, 10% water, 0.1% TFA solvent system- The product fractions were collected between 6.79 and 8.69 minutes.

The title compound was isolated (80.0mg) as a yellow amorphous solid.

1NMR exhibits characteristics of restricted rotation and/or salt formation.

1H NMR (500 MHz, CHLOROFORM-D) δ ppm 0.87 (s, 2.0 H) 1.23 (s, 2.2 H) 1.30 -1.62 (m, 10.5 H) 1.76 (s, 2.7 H) 1.86 - 2.10 (m, 5.5 H) 2.14 - 2.47 (m, 4.6 H) 2.55 - 2.93 (m, 7.7 H) 3.14 - 3.86 (m, 5.2 H) 3.85 - 3.94 (m, 3.6 H) 3.98 (dd, *J*=12.67, 6.56 Hz, 1.8 H) 4.08 - 4.40 (m, 1.4 H) 4.44 - 5.09 (m, 2.0 H) 6.95 (s, 1.5 H) 7.01 - 7.14 (m, 1.2 H) 7.50 (d, *J*=7.63 Hz 1.0 H) 7.58 (d, *J*=7.93 Hz, 1.0 H) 7.66 - 7.82 (m, 0.7 H) 7.87 (d, *J*=8.55 Hz, 1.0 H) 8.24 (s, 0.1 H) 8.67 (s, 0.1 H) 10.18 (s, 0.6 H) LC-MS retention time 1.80min; 751 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min , a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzaepine-10-carboxamide, 13-cyclohexyl-6-[1-ethyl-3-methyl-4-(4-morpholinylcarbonyl)-1H-pyrazol-5-yl]-3-methoxy-N-[(1-methylethyl)sulfonyl]-

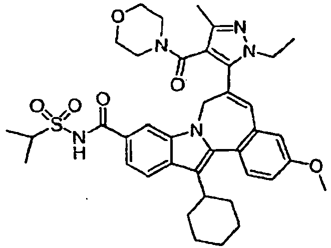

### HPLC purification method:

The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile with a few drops of TFA and water, then filtered through a 0.45uM syringe filter and purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min , a gradient of 70 solvent A / 30% solvent B to 0% solvent A /100% solvent B, a gradient time of 20 minutes with a run time of 30 minutes using %A= 10% acetonitrile, 90% water, 0.1% TFA %B= 90% acetonitrile, 10% water, 0.1% TFA solvent system. The product fractions were collected between 15.62 and 16.66 minutes.

The title compound was isolated (67.0mg) as a yellow amorphous solid.

1H NMR (500 MHz, CHLOROFORM-D) δ ppm 1.14 - 1.29 (m, 1 H) 1.34 - 1.46 (m, 5 H) 1.51 (dd, *J*=22.89, 6.71 Hz, 7 H) 1.79 (d, *J*=10.38 Hz, 2 H) 1.91 - 2.01 (m, 2 H) 2.02 - 2.20 (m, 7 H) 2.27 (s, 3 H) 2.50 - 2.77 (m, 2 H) 2.78 - 2.93 (m, 3 H) 3.08 (s, 2 H) 3.94 (s, 3 H) 4.01 - 4.09 (m, 1 H) 4.11 - 4.24 (m, 2 H) 4.57 (d, *J*=15.26 Hz, 1 H) 4.84 (d, *J*= 14.95 Hz, 1 H) 6.81 (s, 1 H) 6.93 (d, *J*=2.44 Hz, 1 H) 7.12 (dd, *J*=8.70, 2.59 Hz, 1 H) 7.57 (d, *J*=8.55 Hz, 1 H) 7.62 (d, *J*=8.24 Hz, 1 H) 7.67 (s, 1 H) 7.92 (d, *J*=8.24 Hz, 1 H) 10.60 (s, 1 H).

LC-MS retention time 1.83 min; 712 m/z (MH-). LC data was recorded on a Shimadzu LC-10AS liquid chromatograph equipped with a Phenomenex-Luna 10u C18 3.0x50mm column using a SPD-10Av UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzazepine-10-carboxamide, 13-cyclohexyl-6-[1-ethyl-3-methyl-4-(3-oxa-9-azabicyclo[3.3.1]non-9-ylcarbonyl)-1H-pyrazol-5-yl]-3-methoxy-N-[(1-methylethyl)sulfonyl]-

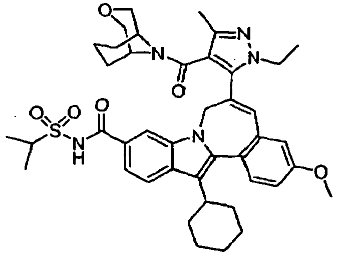

### HPLC purification method:

The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile with a few drops of TFA and water, then filtered through a 0.45uM syringe filter and purified using a Waters Sunfire Prep C18 OBD, 5uM 19mm x 150mm column and monitored using a SPD-10Av UV=Vis detector at a detector wave length of 220nM. The elution conditions employed a flow rate of 25 mL / min, a gradient of 70 solvent A / 30% solvent B to 0% solvent A / 100% solvent B, a gradient time of 20 minutes with a run time of 30 minutes using %A= 10% acetonitrile, 90% water, 0.1% TFA %B= 90% acetonitrile, 10% water, 0.1% TFA solvent system. The product fractions were collected between 18.65 and 19.75minutes.

The title compound was isolated (63.2mg) as a yellow amorphous solid.

1H NMR exhibits some characteristics of restricted rotation with the splitting of some peaks- rotomeric.

1H NMR (500 MHz, CHLOROFORM-D) δ ppm 1.14 - 1.27 (m, 2.0 H) 1.28 - 1.51 1 (m, 12.8 H) 1.51 - 1.58 (m, *J*=6.56, 6.56 Hz, 3.3 H) 1.68 (t, *J*=13.73 Hz, 0.9 H) 1.77 (d, *J*=10.38 Hz, 2.0 H) 1.90 - 2.27 (m, 8.6 H) 2.30 (s, 3.2 H) 2.32 - 2.45 (m, 1.1 H) 2.83 - 2.92 (m. 1.0 H) 3.05 (d, *J*=9.16 Hz, 0.7 H) 3.16 (s, 0.8 H) 3.23 (s, 0.8 H) 3.32 (d, *J*=10.99 Hz, 1.2 H) 3.43 - 3.80 (m, 1.2 H) 3.94 (s, 3.0 H) 3.99 - 4.09 (m. 1.1 H) 4.10 - 4.26 (m, 2:0 H) 4.58 (d, *J*=15.26 Hz, 1.0 H) 4.89 (d, *J*=14.95 Hz, 0.7 H) 4.95 (d, *J*=14.65 Hz, 0.3 H) 6.75 - 6.85 (m, 1.0 H) 6.91 (d, *J*=2.14 Hz, 1.0 H) 7.08 - 7.14 (m 1.0 H) 7.57 (t, *J*=8.70 Hz, 1.0 H) 7.65 (d, *J*=8.24 Hz, 1.0 H) 7.74 (s, 0.7 H) 7.81 (s, 0.3 H) 7.89 (t, *J*=8.24 Hz, 1.0 H) 10.86 (s, 0.8 H).

LC-MS retention time 2.15 min; 752 m/z (MH-). LC data was recorded on a Shimadzu LC- 10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min , a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzazepine-10-carboxamide, 13-cyclohexyl-6-[1-ethyl-4-[[(2S)-2-(methoxymethyl)-4-morpholinyl] carbonyl]-3-methyl-1H-pyrozol-5-yl]-3-methoxy-N-[(1-methylethyl)sulfonyl]-

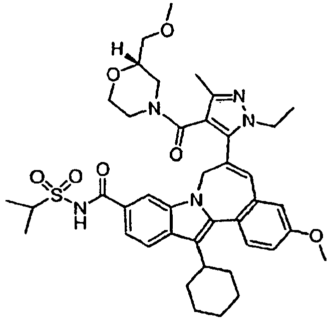

### HPLC purification method:

The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile with a few drops of TFA and water, then filtered through a 0.45uM syringe filter and purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min , a gradient of 70 solvent A / 30% solvent B to 0% solvent A / 100% solvent B, a gradient time of 20 minutes with a run time of 30 minutes using %A= 10% acetonitrile, 90% water, 0.1% TFA %B= 90% acetonitrile, 10% water, 0.1% TFA solvent system. The product fractions were collected between 15.58 and 16.76 minutes.

The title compound was isolated (65.1mg) as a yellow amorphous solid.

1H NMR exhibits some charasteristics of restricted rotation with the splitting of some peaks- rotomeric.

1H NMR (500 MHz, CHLOROFORM-D) δ ppm 1.15 - 1.30 (m, 1.3 H) 1.31 - 1.46 (m, 4.9 H) 1.49 (d, *J*=6.41 Hz, 3.9 H) 1.53 (d, *J*=6.41 Hz, 3.3 H) 1.79 (d, *J*=9.77 Hz, 2.4 H) 1.83 - 2.03 (m, 4.9 H) 2.02 - 2.18 (m, 3.1 H)2.26 (s, 3.0 H) 2.29 - 2.52 (m, 1.3 H) 2.68 (s, 0.5 H) 2.80 - 3.02 (m, 3.8 H) 3.05 (s, 1.9 H) 3.12 - 3.30 (m, 3.3 H) 3.29 - 3.51 (m, 1.4 H) 3.94 (s, 2.9 H) 4.00 - 4.10 (m, 1.1 H) 4.10 - 4.24 (m, 1.9 H) 4.56 (d, *J*=15.26 Hz, 1.0 H) 4.74 - 4.92 (m, 0.9 H) 6.81 (d, *J*=17.09 Hz, 1.0 H) 6.92 (s, 1.0 H) 7.11 (dd, *J*=8.55,2.14 Hz, 1.0 H) 7.50 - 7.77 (m, 3.0 H) 7.89 (t, *J*=9.00 Hz, 1.0 H) 10.74 (s, 0.6 H).

LC-MS retention time 1.88 min; 756 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O /10 mM ammonium acetate and solvent B was 5% H2O /95% acetonitrile /10 mM ammonium acetate. MS data was determined using a Micromass Platform for LC in electrospray mode.

### 7H-indolo[2,1-a][2]benzazepine-10-carboxylic acid, 13-cyclohexyl-6-[4-(ethoxycarbonyl)-1-ethyl-1H-pyrazol-5-yl]-3-methoxy-, 1,1-dimethylethyl ester

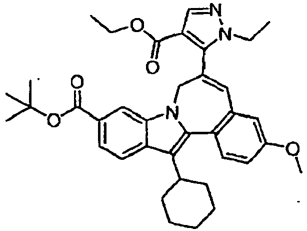

In a 20ml microwave vessel suspend tert-butyl 13-cyclobexyl-6-((2E,Z)-3-(dimethylamino)2-(ethoxycarbonyl)-2-propenoyl)-3-methoxy-7H-indolo[2,1-a][2]benzazepine-10-carboxylate (2.056 g, 3.36 mmol) in ethanol (9 mL) and dioxane (2.2 mL). To the reaction was added ethylhydrazine oxalate (0.533 g, 3.55 mmol) followed by triethyl amine (1.4 mL, 10.04 mmol). The reaction was capped under a nitrogen atmosphere and heated using microwave to 160C for 40min. The reaction was diluted with ethyl acetate and washed with 1.0N aqueous hydrochloric acid and the aqueous layer extracted with ethyl acetate. The organic extracts were combined and washed sequentially with saturated aqueous sodium bicarbonate and then 1.0N aqueous hydrochloric acid and brine. The organic phase was dried over magnesium sulfate, filtered and solvent removed *in vacuuo* using a rotary evaporator to give 2.02g of crude product. The crude product was combined with 500mg of crude product from an analogous reaction in dichloromethane and adsorbed onto 6.3g of silica gel. The title compound was purified by silica column chromatography (89g slurry loaded in dichloromethane), eluting with dichloromethane then 2% ethyl acetate in dichloromethane. Note the first major UV adsorbing spot is that of the regio-ethyl pyrazole isomer and the second larger spot on TLC is that of the title compound.

The pure product fractions were collected and solvent removed *in vacuuo* to give 1.006g of the title compound as a yellow amorphous solid.

1H NMR (500 MHz, CHLOROFORM-D) δ ppm 0.70 (s, 3 H) 1.28 - 1.51 1 (m, 7 H) 1.57 (s, 9 H) 1.77 (d, *J*=9.16 Hz, 2 H) 1.94 (s, 1 H) 2.07 (s, 3 H) 2.79 - 2.94 (m, 1 H) 3.53 - 3.71 (m, 2 H) 3.90 (s, 3 H) 4.21 - 4.33 (m, 2 H) 4.69 (d, *J*=11.90 Hz, 1 H) 4.99 (d, *J*=12.51 Hz, H) 6.76 (s, 1 H) 6.93 (d, *J*=2.44 Hz, 1H) 7.06 (dd, *J*=8.55_{.} 2.44 Hz, 1 H) 7.52 (d, *J*=8.85 Hz, 1 H) 7.63 (d, *J*=8.55 Hz, 1 H) 7.79 - 7.84 (m, 2 H) 7.93 (s, 1 H).

LC-MS retention time2.91 min; 610m/z (MH+). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O /10 mM ammonium acetate and solvent B was 5% H2O / 95% acetonitrile /10 mM ammonium acetate. MS data was determined using a Micromass Platform for LC in electrospray mode.

### 7H-indolo[2,1-a][2]benzazepine-10-carboxylic acid, 13-cyclohexyl-6-[4-(ethoxycarbonyl)-1-ethyl-1H-pyrazol-3-yl]-3-methoxy-, 1,1-dimethylethyl ester

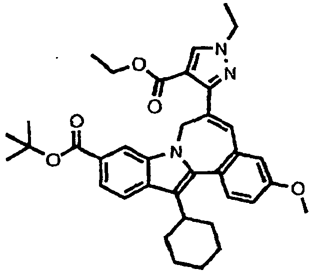

The above compound was isolated as a minor component of the reaction mixture for the above experimental for 7H-indolo[2,1-a][2]benzazepine-10-carboxylic acid, 13-cyclohexyl-6-[4-(ethoxycarbonyl)-1-ethyl-1H-pyrazol-5-yl]-3-methoxy-, 1,1-dimethylethyl ester and is described in the text as the regio-ethyl pyrazole isomer. 1H NMR (500 MHz, CHLOROFORM-D) δ ppm 1.17 1.27 (m, 2 H) 1.31 (t, *J*=7.17 Hz,3H)1.34-1.51(m,3H) 1.51-1.57(m,7H) 1.61 (s, 9 H) 1.61 - 1.66 (m, I H) 1.75 (d, *J=*11.29 Hz, 2 H) 1.93 (d, *J*=7.02 Hz, 1 H) 1.97 - 2.16 (m, 3 H) 2.81- 2.91 (m, 1 H) 3.90 (s, 3 H) 4.13 - 4.31 (m, 4 H) 4.39 (d, *J*=14.34 Hz, 1 H) 5.84 (d, *J*=14.04 Hz, 1 H) 6.96 - 7.06 (m, 2 H) 7.51 (d, *J*=8.24 Hz, 1. H) 7.64 (d, *J*=8.24 Hz, 1 H) 7.79 (d, *J*=8.55 Hz, 1 H) 7.94 (d, *J*=7.32 Hz, I H) 8.29 (s, I H).

LC-MS retention time 3.06 min; 610m/z (MH+). LC data was recorded on a Shimadzu LC- 10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A 10% solvent B to 0% solvent A /100% solvent B, a gradient time of 3 min, a hold time of 1 min, and an analysis time of 4 min where solvent A was 5% acetonitrile / 95% H2O / 10 mM ammonium acetate and solvent B was 5% H2O /95% acetonitrile /10 mM ammonium acetate. MS data was determined using a Micromass Platform for LC in electrospray mode.

### 7H-indolo[2,1-a][2]benzazepine-10-carboxylic acid, 13-cyclohexyl-6-[4-(ethoxycarbonyl)-1-ethyl-1H-pyrazol-5-yl]-3-methoxy-

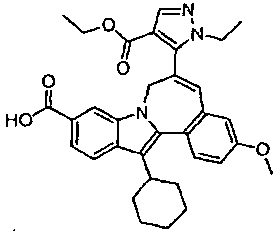

7H-indolo[2,1-a][2]benzazepine-10-carboxylic acid, 13-cyclohexyl-6-[4-(ethoxycarbonyl)-1-ethyl-1H-pyrazol-5-yl]-3-methoxy-, 1,1-dimethylethyl ester (0.984 g, 1.614 mmol) was dissolved in 1,2-dichloroethane (10 mL) and TFA (10.00 mL) added to the reaction. The reaction was placed under a nitrogen atmosphere and stirred at room temperature for 3.5 hours. Volatiles were removed from the reaction in vacuuo using a rotary evaporator. Dissolve product in ethyl acetate and wash sequentially with 1.0N aqueous hydrochloric acid and brine, dry over magnesium sulfate, filter and remove solvents in vacuuo. The title compound was dried in vacuuo to yield 978mg as a amorphous yellow solid.

1H NMR (500 MHz, CHLOROFORM-D) δ ppm 0.69 (s, 3 H) 1.14 - 1.28 (m, 2 H) 1.28 - 1.39 (m, 4 H) 1.37 - 1.61 (m, 3 H) 1.78 (d, *J*=9.46 Hz, 2 H) 1.95 (s, 1 H) 2.08 (s, 3 H) 2.80 - 2.95 (m, 1 H) 3.65 (s, 2 H) 3.91 (s, 3 H) 4.19 - 4.36 (m, 2 H) 4.73 (d, *J*=12.21 Hz, 1 H) 4.99 (d, *J*=10.38 Hz, 1 H) 6.78 (s, 1 H) 6.94 (d, *J*=2.75 Hz, 1 H) 7.08 (dd, *J*=8.55, 2.75 Hz, 1 H) 7.53 (d, *J*=8.55 Hz, 1 H) 7.75 (dd, *J*=8.55, 1.22 Hz, 1 H) 7.88 (d, *J*=8.55 Hz, 1 H) 7.93 (s, 1 H) 7.99 (s, 1 H).

LC-MS retention time 2.03min; 552m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM: The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O 10 mM ammonium acetate and solvent B was 5% H2O / 95% acetonitrile /10 mM ammonium acetate. MS data was determined using a Micromass Platform for LC in electrospray mode.

### Ethyl 5-(13-cyclohexyl-10-((isopropylsulfonyl)carbamoyl)-3-methoxy-7H-indoln[2,1-a][2]benzazepin-6-yl)-1-ethyl-1H-pyrazole-4-carboxylate

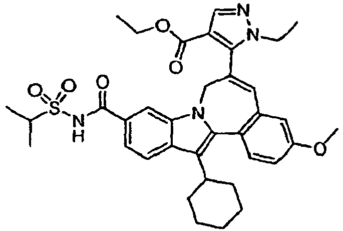

In a 100ml round bottom flask dissolve 13-cyclohexyl-6-(4-(ethoxycarbonyl)-1-ethyl-1H-pyrazol-5-yl)-3-methoxy-7H-indolo[2,1-a][2]benzazepine-10-carboxylic acid (950mg, 1.680 mmol), propane-2-sulfonamide (652 mg, 5.29 mmol) and DMAP (655 mg, 5.36 mmol) in dichloromethane (16.8 mL). To the reaction add EDC (508 mg, 2.65 mmol). Place the reaction under a nitrogen atmosphere and stir a room temperature for 64 hours.

Remove solvent in *vacuuo* using a rotary evaporator and partition the reaction between ethyl acetate and 1.0N aqueous hydrochloric acid. Wash the organic extract sequentially with 1.0N aqueous hydrochloric acid and brine then dry over magnesium sulfate. Filter off the magnesium sulfate from the organic extract rinsing with ethyl acetate then remove the solvent in *vacuuo* from the filtrate using a rotary evaporator. The product was dried in *vacuuo* at room temperature to yield 1.009g of the title compound as a yellow amorphous solid.

1H NMR (500 MHz, CHLOROFORM-D) δ ppm 0.69 (s, 3 H) 1.18 - 1.36 (m, 6 H) 1.41 (d, *J*=6.71 Hz, 2 H) 1.45 (d, *J*=6.71 Hz, 6 H) 1.48 - 1.61 (m, 2 H) 1.79 (d, *J*=9.46 Hz, 2 H) 1.90 - 2.15 (m, 4 H) 2.46 (s, 4 H) 2.82 - 2.93 (m, 1 H) 3.66 (s, 2 H) 3.90 (s, 3 H) 4.00 - 4.08 (m, 1 H) 4.26 (dd, *J*=13.73, 6.7.1 Hz, 2 H) 4.72 (d, *J*= 13.73 Hz, 1 H) 4.99 (d, *J*=12.21 Hz, 1 H) 6.79 (s, 1 H) 6.94 (d, *J*=1.83 Hz, 1 H) 7.08 (dd, *J*=8.39, 1.98 Hz, 1 H) 7.37 (d, J=8.55 Hz, 1 H) 7.52 (d, J=8.55 Hz, 1 H) 7.76 (s, 1 H) 7.88 (d, *J*=8.55 Hz, 1 H) 7.95 (s, 1 H) 8.31 (s, I H).

LC-MS retention time 1.95min; 657m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O /10 mM ammonium acetate and solvent B was 5% H2O 95% acetonitrile /10 mM ammonium acetate. MS data was determined using a Micromass Platform for LC in electrospray mode.

### 5-(13-cyclohexyl-10-((isopropylsulfonyl)carbamoyl))-3-methoxy-7H-indolo[2,1-a][2]benzazepin-6-yl)-1-ethyl-1H-pyrazole-4-carboxylic acid

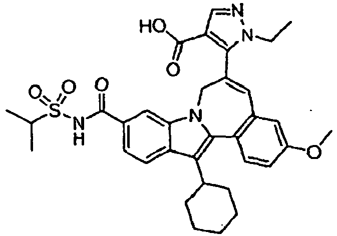

Ethyl 5-(13-cyclohexyl-10-((isopropylsulfonyl)carbamoyl)-3-methoxy-7H-indolo[2,1-a][2]benzazepin-6-yl)-1-ethyl-1H-pyrazole-4-carboxylate was dissolved in THF (14 mL) and methanol (14 mL) then sodium hydroxide(15 mL, 15.00 mmol) (1.0N aqueous) was added to the reaction. The reaction was placed under a nitrogen atmosphere and stirred at room temperature for 16.5hrs. The reaction was concentrated ***in** vacuuo* using a rotary evaporator (bath temp equal to less than 26C) then partitioned between ethyl acetate and 1.0N aqueous hydrochloric acid. The aqueous layer was extracted with ethyl acetate and the organic extracts combined, washed with brine and dried over magnesium sulfate. The organic solution was filtered and solvents removed in *vacuuo* to give amorphous amber-orange solid (902mg) after drying at room temperature *in vacuuo..*

1H NMR (500 MHz, CHLOROFORM-D) δ ppm 0.79 (s, 3 H) 1.12 - 1.21 (m, 1 H) 1.28 - 1.38 (m, 2 H) 1.41 (d, *J=*6.71 Hz, 6 H) 1.43 -1.57 (m, 2 H) 1.66 - 1.83 (m, 2 H) 1.87 - 2.07 (m, 4 H) 2.77 - 2.89 (m, 1 H) 3.71 (s, 3 H) 3.90 (s, 3 H) 3.98 - 4.07 (m, 1 H) 4.63 (d, *J*=13.43 Hz, 1 H) 4.99 (d, *J*=12.82 Hz, I H) 6.79 (s, 1 H) 6.94 (d, *J*=2.44 Hz, 1 H) 7.07 (dd, *J*=8.70, 2.29 Hz, 1 H) 7.43 (d, *J*=8.24 Hz, 1 H) 7.51 (d, *J*=8.85 Hz, 1 H) 7.80 - 7.87 (m, 2 H) 7.95 (s, 1 H) 8.86 (s, 1 H).

LC-MS retention time 1.35 min; 629m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% Solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 13-cyclohexyl-6-(4-(((2R,6S)-2,6-dimethyl-4-morpholinyl)carbonyl)-1-ethyl-1H-pyrazol-5-yl)-N-(isopropylsulfonyl)-3-methoxy-7H-indolo[2,1-a][2]benzazepine-10-carboxamide

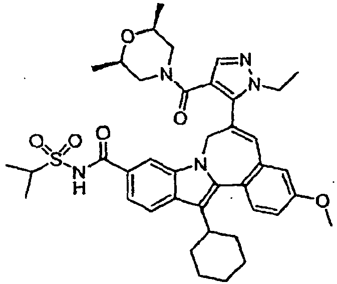

5-(13-cyclohexyl-10-((isopropylsulfonyl)carbamoyl)3-methoxy-7H-indolo[2,1-a][2]benzazepin-6-yl)-1-ethyl-1H-pyrazole-4-carboxylic acid (75 mg, 0.119 mmol) was dissolved in DMF (1.2ml) and HATU (103 mg, 0.271 mmol) added to the reaction. The reaction was capped and stirred at room temperature for 1.5hrs then DMAP (44.3 mg, 0.363 mmol) and the amine reagent (2R,6S)-2,6-dimethylmorpholine (0.044 ml, 0.357 mmol) were added to the reaction. The reaction was capped under a nitrogen atmosphere and stirred at room temperature for 2 days. The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile with 2 drops of water added to aid solubility then purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min, a gradient of 70 solvent A / 30% solvent B to 0% solvent A / 100% solvent B, a gradient time of 20 minutes with a run time of 30 minutes using %A= 10% acetonitrile, 90% water, 0.1% TFA %B= 90% acetonitrile, 10% water, 0.1% TFA solvent system. Product eluted between 17.39 and 18.20 minutes. Volatiles from the product fraction were removed *in vacuuo* overnight using a Speed-Vac set at medium heat. The title compound (54.3mg) was isolated as a yellow amorphous solid.

1H NMR (500 MHz, CHLOROFORM-D) δ ppm 0.69 (s, 4 H) 0.82 (d, *J*=33.57 Hz, 2 H) 1.15 - 1.31 (m, 1 H) 1.31 - 1.59 (m, 12 H) 1.79 (d, *J*=10.68 Hz, 2 H) 1.88 - 2.32 (m, 13 H) 2.88 (t, *J*=11.14 Hz, 1 H) 2.96 - 3.31 (m, 2 H) 3.95 (s, 3 H) 4.04 - 4.12 (m, 1 H) 4.22 (s, 2 H) 4.60 (d, J=14.95 Hz, 1 H) 4.90 (d, *J*=15.56 Hz, 1 H) 6.77 - 6.86 (m, 1 H) 6.94 (s, 1 H) 7.10 - 7.17 (m, 1 H) 7.61 (dd, *J*=27.62, 7.17 Hz, 2 H) 7.70 (d, *J*=19.23 Hz, 2 H) 7.92 (d, *J*=7.93 Hz, 1 H) 10.49 (s, 1 H).

LC-MS retention time 1.93min; 726m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of I min, and an analysis time of 4 min where solvent A was 5% acetonitrile / 95% H2O /10 mM ammonium acetate and solvent B was 5% H2O /95% acetonitrile /10 mM ammoniutn acetate. MS data was determined using a Micromass Platform for LC in electrospray mode.

The following compounds were prepared from 5-(13-cyclohexyl-10-((isopropylsulfonyl)carbamoyl)-3-methoxy-7H-indolo[2,1-a][2]benzazepin-6-yl)-1-ethyl-1H-pyrazole-4-carboxylic acid by identical coupling methodology used in the above example for 13-cyclohexyl-6-(4-(((2R,6S)-2,6-dimethyl-4-morpholinyl)carbonyl)-1-ethyl-1H-pyrazol-5-yl)-N-(isopropylsulfonyl)-3-methoxy-7H-indolo[2,1-a][2]benzazepine-10-carboxamide.

### 7H-indolo[2,1-a][2]benzazepine-10-carboxamide, 13-cyclohexyl-6-[1-ethyl-4-(8-oxa-3-azabicyclo[3.2.1]oct-3-ylcarbonyl)-1H-pyrazol-5-yl]-3-methoxy-N-[(1-methylethyl)sulfonyl]-

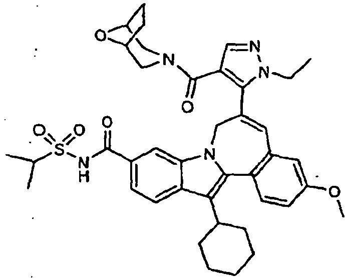

### HPLC purification method:

The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile and a few drops of water added to solubilize. The sample was then filtered through a 0.45uM syringe filter and purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min, a gradient of 70 solvent A /30% solvent B to 0% solvent A /100% solvent B, a gradient time of 20 minutes with a run time of 30 minutes using %A= 10% acetonitrile, 90% water, 4.1% TFA %B= 90% acetonitrile, 10% water, 0.1% TFA solvent system. The product fraction was collected between 16.30min and 17.11min.

The title compound was isolated (61.7mg) as a yellow amorphous solid.

1H NMR (500 MHz, CHLOROFORM-D) δ ppm 1.24 (dd, *J*=21.67, 10.38 Hz, 2 H) 1.39 (s, 4 H) 1.46 (d, *J*=5.80 Hz, 6 H) 1.52 - 1.72 (m, 4 H) 1.78 (d, *J*=8.85 Hz, 2 H) 1.90 - 2.03 (m, 2 H) 2.08 (s, 3 H) 2.27 (s, 1 H) 2.85 (t, *J*=11.60 H:z, 1 H)2.97 - 3.13 (m, 1 H) 3.31 (s, 1 H) 3.86 (s, 1 H) 3.93 (s, 3 H) 3.98 - 4.09 (m, 2 H) 4.22 (s, 2 H) 4.57 (d, *J=*15.26 Hz, 1 H) 4.81-5.07 (m, 1 H) 6.71-6.92 (m, 3 H) 6.94 (s, 1H) 7.11 (d, *J*=7.93 Hz, 1 H) 7.54 (d, *J*=8.55 Hz, 1 H) 7.72 (s, 1 H) 7.82 - 8.02 (m, 1 H) 9.63 - 10.11 (m, 1H).

LC-MS retention time 1.80min; 724 m/z (MH-). LC data was recorded on a Shimadzu LC-10AS liquid chromatography equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A /0% solvent B to 0% solvent A /100% solvent B, a gradient time of 3 min, a hold time of 1 min, and an analysis time of 4 min where solvent A was 5% acetonitrile /95% H2O /10 mM ammonium acetate and solvent B was 5% H2O / 95% acetonitrile /10 mM ammonium acetate. MS data was determined using a Micromass Platform for LC in electrospray mode.

### 7H-indolo[2,1-a][2]benzarepine-10-carboxamide, 13-cyclohexyl-6-[1-ethyl-4-[(8-methyl-3,8-diazabicyclo[3.2.1]oct-3-yl)carbonyl]-1H-pyrazol-5-yl]-3-methoxy-N-[(1-methylethyl)sulfonyl]-

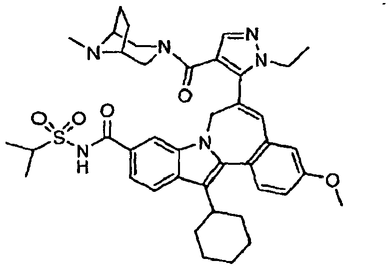

### HPLC purification method:

The product was purified on a Shimadzu high pressure liquid chromatography system employing Discovery VP software interfaced with a SCL-10A controller, SIL-10A autosampler and FRC-10A fraction collector. The reaction was diluted to 4ml with acetonitrile / DMF (1:1) and a couple drops of TFA added, the sample was filtered through a 0.45uM syringe filter and the filtrate injected in two injections and purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min, a gradient of 70 solvent A / 30% solvent B to 0% solvent A /100% solvent B, a gradient time of 20 minutes with a run time of 20 minutes using %A= 10% methanol, 90% water, 0.1% trifluoroacetic acid %B= 90% methanol, 10% water, 0.1% trifluoroacetic acid solvent system.

The title compound was isolated (55.5mg) as a yellow-greenish amorphous solid. 1H NMR (500 MHz, CHLOROFORM-D) d ppm 0.74 (s, 3 H) 1.12 - 1.29 (m, 2 H) 1.30 - 1.41 (m, 4 H) 1.42 - 1.57 (m, 4 H) 1.77 (d, *J*=8.55 Hz, 2 H) 1.86 - 2.10 (m, 4 H) .2.18 (s, 1 H) 2.24 - 2.50 (m, 2 H) 2.78 - 2.95 (m, 4 H) 3.03 (s, 5 H) 3.73 (s, 1 H) 3.81 - 3.88 (m, 1 H) 3.90 (s, 3 H) 3.93 (s, 1 H) 3.99 - 4.08 (m, 2 H) 4.10 - 4.37 (m, 3 H) 4.40 - 4.69 (m, 3 H) 6.90 (s, 1 H) 6.96 (d, *J*=2.44 Hz, 1 H) 7.07 (dd, *J*=8.70, 2.29 Hz, 1 H) 7.49 - 7.55 (m, 2 H) 7.57 - 7.65 (m, 1 H) 7.67 (s, 1 H) 7.86 (d, *J*=8-24 Hz, 1 H) 10.64 (s, 1 H) 11.24 (s, 1 H).

LC-MS retention time 1.74 min;737m/z (MH-). LC data was recorded on a Shimadzu LC-10AS liquid chromatograph equipped with a Phenomcnex-Luna 10u C18 3.0x50mm column using a SPD-10AV UV-Vis detector at a detector wave length of 220nM. The elution conditions employed a flow rate of 5 ml/min, a gradient of 100% solvent A /0% solvent B to 0% solvent A /100% solvent B, a gradient time of 3 min, a bold time of I min, and an analysis time of 4 min where solvent A was 5% acetonitrile / 95% H2O / 10 mM ammonium acetate and solvent B was 5% H2O / 95% acetonitrilc /10 mM ammonium acetate. MS data was determined using a Micromass Platform for LC in electrospray mode.

### ethyl 5-(13-cyclohexyl-10-((dimethylsulfamoyl)carbamoyl)-3-methoxy-7H-indolo[2,1-a][2]benzazepin-6-yl)-1-ethyl-3-methyl-1H-pyrazole-4-carboxylate

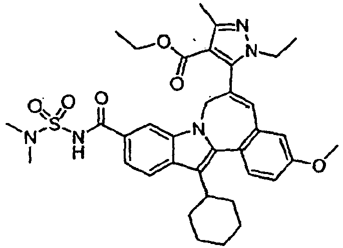

7H-indolo[2,1-a][2]benzazepine-10-carboxylic acid, 13-cyclohexyl-6-[4-(ethoxycarbonyl)-1-ethyl-3-methyl-1H-pyrazol-5-yl]-3-methoxy- (0.603 g, 1.062 mmol) was dissolved in dichloromethane (10.6 mL) with DMAP (0.417 g, 3.41 mmol) and N,N-dimethylsulfamide (0.394 g, 3.17 mmol). EDC (0.336 6 g, 1.753 mmol) was added to the reaction and the reaction was stirred under a nitrogen atmosphere at room temperature for 40 hours. Solvent was removed from the reaction in *vacuuo* using a rotary evaporator. The residue was partitioned between ethyl acetate and 1.0N aqueous hydrochloric acid. The organic phase was sequentially washed with 1.0N aqueous hydrochloric acid, a mixture of 0.1 M aqueous NaH2PO4 / 1.0N aqueous hydrochloric acid and again with 1.0N aqueous hydrochloric acid and finally with brine. The organic phase was dried over magnesium sulfate, filtered and solvent removed in *vacuuo* using a rotary evaporator. The yellow amorphous solid was dried *in vacuuo* at room temperature to yield 669g of the title compound_

1H NMR (500 MHz, CHLOROFORM-D) δ ppm 0.53 - 0.90 (m, 3 H) 1.15 1.46 (m, 7 H) 1.47 -1.66 (m, 4 H) 1.78 (d, *J=*8.85 Hz, 2 H) 1.91 - 2.15 (m, 4 H) 2.48 (s, 3 H) 2.86 (t, *J*=16.02 Hz, 1 H) 3.04 (s, 6 H) 3.52 (d, *J*=38.76 Hz, 2 H) 3.90 (s, 3 H) 4.26 (s, 2 H) 4.67 (d, *J*=14.34 Hz, 1 H) 4.95 (d, J=13.73 Hz, 1 H) 6.73 (s, 1 H) 6.93 (d, *J*=2.44 Hz, 1 H) 7.06 (dd, *J*=8.55, 2.44 Hz, 1 H) 7.33 (d, *J*=8.5 Hz, 1 H) 7.51 (d, *J*=8.55 Hz, 1 H) 7.78 (s, 1 H) 7.87 (d, *J*=8.55 Hz, 1 H) 8.46 (s, 1 H)

LC-MS retention time2.23 min; 672 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A /0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 1H-pyrazole-4-carboxylic acid 5-[13-cyclohexyl-10-[[[(dimethylamino)sulfonyl]amino]carbonyl]-3-methoxy-7H-indolo[2,1-a][2]benzazepin-6-yl]-1-ethyl-3-methyl-

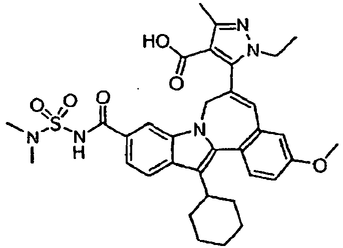

In a 100ml rb flask dissolve 1H-pyrazole-4-carboxylic acid, 5-[13-cyclohexyl-10-[[[(dimethylamino)sulfonyl]amino]carbonyl]-3-methoxy-7H-indolo[2,1-a][2]benzazepin-6-yl]-1-ethyl-3-methyl-, ethyl ester (652mg, 0.968 mmol) in THF (10 mL) and methanol (10.00 mL). Sodium hydroxide (10 mL, 10.00 mmol), 1.0N aqueous solution was added to the reaction and the reaction was stirred under a nitrogen atmosphere at room temperature for 7 days.

The reaction was concentrated using rotary evaporator keeping the water heating bath temperature at or below 25C. The remaining reaction solution was partitioned between ethyl acetate and 1.0N aqueous hydrochloric acid. Extract aqueous phase with ethyl acetate and combine the organic extracts and wash with brine, dry over magnesium sulfate, filter and remove solvent in *vacuuo* using a rotary evaporator. The amorphous film residue was dissolved in dichloromethane and the solvent and volatiles were removed in vacuuo using a rotary evaporator. The yellow amorphous solid was broken up into a more finely divided solid and dried in *vacuuo* at room temperature to yield 600mg of the title compound as a yellow amorphous solid.

1H NMR (500 MHz, CHLOROFORM-D) δ ppm 0.71 (s, 3 H) 0.86 (s, 1 H) 1.10 - 1.58 (m, 6 H) 1.69 (s, 1 H) 1.76 (d, *J*=10.99 Hz, 1 H) 1.86 - 2.15 (m, 5 H) 2.47 (s, 3 H) 2.84 (t, *J*=11.90 Hz, 1 H) 3.00 (s, 6 H) 3.59 (s, 2 H) 3.90 (s, 3 H) 4.64 (d, *J*=13.43 Hz, 1 H) 4.97 (d, *J*=113.73 Hz, 1 H) 6.75 (s, 1 H) 6.95 (d, *J*=2.75 Hz, 1 H) 7.06 (dd, *J*=8.55, 2.44 Hz, 1 H) 7.35 (d, *J*=8.24 Hz, 1 H) 7.50 (d, *J*=8.54 Hz, 1 H) 7.77 - 7.86 (m, 2 H) 8.68 (s, 1 H).

LC-MS retention time 1.75min; 644 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A /100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 13-cyclohexyl-6-(4-(((2R, 6S)-2,6-dimethyl-4-morpholinyl)carbonyl)-1-ethyl-3-methyl-1H-pyrazol-5-yl)-N-(dimethylsulfamoyl)-3-methoxy-7H indolo[21-a][2]benzapine-10-carboxamide

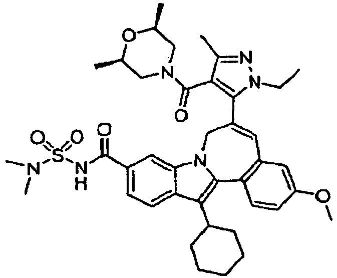

CAS Name for 77778-062 (126 mg, 0.195 mmol) was dissolved in DMF (1.951 mL) and
HATU (167 mg, 0.439 mmol) was added to the reaction. The reaction was capped and stirred at room temperature for 1.5hrs then DMAP (72.1 mg, 0.590 mmol) was added to the reaction followed by the amine reagent, (2R,6S)-2,6-dimethylmorpholine (72.5 µL, 0.585 mmol). The reaction was capped under a nitrogen atmosphere and stirred at room temperature for 2 days. The product was purified on a Shimadzu high pressure liquid chromatography system employing Discovery VP software interfaced with a SCL-10A controller, SIL-10A autosampler and FRC-10A fraction collector. The reaction was diluted to 4ml with acetonitrile with a few drops of water to dissolve then filtered through a 0.45uM syringe filter and purified (2 x 2ml injections) using a Waters Sunfire Prep C18 OBD, 5uM 19mm x 150mm column and monitored using a SPD-10AV UV-Vis detector at a detector wave length of 220nM. The elution conditions employed a flow rate of 25 mL / min, a gradient of 70 solvent A / 30% solvent B to 0% solvent A / 100% solvent B, a gradient time of 20 minutes with a run time of 30 minutes using %A= 10% acetonitrile, 90% water, 0.1% TFA %B= 90% acetonitrile, 10% water, 0.1 % TFA solvent system_ The product fractions were collected and volatiles removed *in vacuuo* overnight using a speed-vac set on medium heating. The product fractions were analyzed by LCMS and the pure fractions were combined in dichloromethane and transferred to a vial. The solvent was removed using a nitrogen sweep and the product dried *in vacuuo* at room temperature to yield 89.2mg of the title compound as amorphous yellow solid.

1H NMR (500 MHz, CHLOROFORM-D) δ ppm 0.68 (s, 3 H) 0.73 - 1.08 (m, 3 H) 1.15 - 1.29 (m, 1 H) 1.31 - 1.54 (m, 6 H) 1.79 (d, .*J*=10.99 Hz, 2 H) 1.88 - 2.18 (m, 6 H) 2.26 (s, 3 H) 2.58 - 2.99 (m, 7 H) 3.07 (s, 5 H) 3.10 - 3.27 (m, 2 H) 3.94 (s, 3 H) 4.19 (s, 2 H) 4.57 (d, *J=*14.95 Hz, 1 H) 4.90 (d, *J*=15.26 Hz, I H) 6.70 - 6.88 (m, 1 H) 6.93 (s, 1 H) 7.12 (d, *J*=7.63 Hz, 1 H) 7.45 - 7.67 (m, 2 H) 7.73 (s, 1 H) 7.91 (d, *J*=8.24 Hz, 1H) 10.61 (s, 1 H)

LC-MS retention time 2.13 min; 741m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 1H-pyrazole-4-carboxylic acid, 5-[13-cyclohexyl-10-[[(cyclopropylsulfonyl)amino]carbonyl]-3-methoxy-7H-indolo[2,1-a][2]benzazepin-6-yl]-1-ethyl-3-methyl-, ethyl ester.

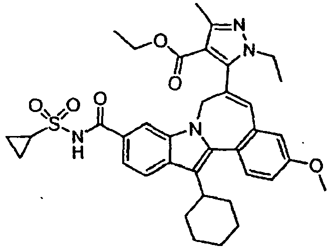

Dissolve 7H-indolo[2,1-a][2]benzazepine-10-carboxylic acid, 13-cyclohexyl-6-[4-(ethoxycarbonyl)-1-ethyl-3-methyl-1H-pyrazol-5-yl]-3-methxoy- (110 mg, 0.194 mmol) in THF (0.646 mL). Carbonyldiimidazole (94 mg, 0.581 mmol) was added to the reaction. The reaction was placed under a nitrogen atmosphere and heated to 60 deg C for 1 hour. The reaction was cooled under a nitrogen atmosphere and cyclopropanesulfonamide (94 mg, 0.775 mmol) was added to the reaction followed by DBU (0.088 mL, 0.581 mmol). The reaction was immerse in oil bath at 60 deg C under nitrogen atmosphere and heated overnight at 60 deg C. The reaction was diluted with chloroform (50 mL) and the organic layer washed sequentially with 1.0N aqueous hydrochloric acid (50 mL), 0.1M aqueous NaH2PO4 (50 mL) and brine (25 mL). The organic layer was dried over MgSO4, filtered and volatiles removed *in vacuuo* to yield a yellow foam which was dried *in vacuuo* at room temperature overnight to yield 130 mg (0.194 mmol, 90% crude) of the title compound as a yellow amorphous solid. 1H NMR (500 MHz, CHLOROFORM-D) δ ppm 0.67 (br.m, 3H) 1.12 - 1.59 (m, 9 H) 1.62 - 2.16 (m, 8 H) 2.49 (s, 3 H) 2.87 (m, 1 H) 3.91 (s, 3 H) 4.11 (m, 2 H) 4.27 (m, 3 H) 4.69 (br.d, 1 H) 4.97 (br.d, 1 H) 6.72 (s, 1 H) 6.94 (s, 1 H) 7.07 (dd, *J*=8.55, 2.14 Hz, 1 H) 7.37 (dd, *J*=8.55, 2.14 Hz, 1 H) 7.51 (d, *J*=8.24 Hz, 1 H) 7.82 (d, *J*=2.14 Hz, 1 H) 7.89 (d, *J*=8.24 Hz,1 H) 8.59 (br. s, 1 H). LCMS 671 m/z (MH+).

### 1H-pyrazole-4-carboxylic acid 5-[13-cyclohexyl-10-[[(clopropylsulfonyl)amino]carbonyl]-3-methoxy-7H-indolo[2,1a][2]benzazepin-6-yl]-1-ethyl-3-methyl-.

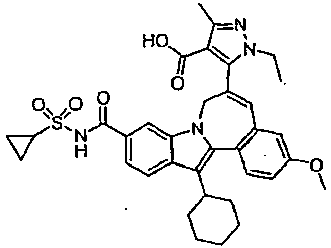

Dissolve 1*H-*pyrazole-4-carboxylic acid, 5-[13-cyclohexyl-10-[[(cyclopropylsulfonyl)amino]carbonyl]-3-methoxy-7*H*-indolo[2,1*a*]][2]benzazepin-6-yl]-1-ethyl-3-methyl-, ethyl ester (130 mg, 0.194 mmol) was dissolved in dioxane (0.969 mL) and methanol (0.969 mL) was added to the reaction followed by 1N aqueous sodium hydroxide (1.94 mL). The reaction was capped under a nitrogen atmosphere and stirred at room temperature for 72 hrs. The reaction was diluted with chloroform (50 mL) and washed with 1.0N aqueous hydrochloric acid (50 mL). The organic layer was concentrated *in vacuuo* using a rotary evaporator to yield the title compound as a yellow solid (125 mg, 100% crude). 1H NMR (500 MHz, CHLOROFORM-D) δ ppm 0.72 (br.m, 3H) 1.12 - 1.59 (m, 6 H) 1.62 - 2.16 (m, 8 H) 2.51 (s, 3 H) 2.87 (m, 3 H) 3.91 (s, 3 H) 4.12 (m, 1 H) 4.69 (br.d, 1 H) 5.01 (br.d, 1 H) 6.74 (s, 1 H) 6.96 (s, 1 H) 7.08 (dd, *J*=8.55,2.14 Hz, 1 H) 7.41 (dd, *J=*8.55, 2.14 Hz, 1 H) 7.52 (d, *J=*8.24 Hz, 1 H) 7.88 (m, 2 H) 8.92 (br. s, 1 H). MS m/z 643 (MH⁺).

### 7H-indolo[2,1-a][2]benzazepine-10-carboxamide, 13-cyclohexyl-N-(cyclopropylsulfonyl)-6-[4-[[(2R,6S)-2.6-dimethyl-4-morpholinyl]carbonyl]-1-ethyl-3-methyl-1H-pyrazol-5-yl]-3-methoxy-

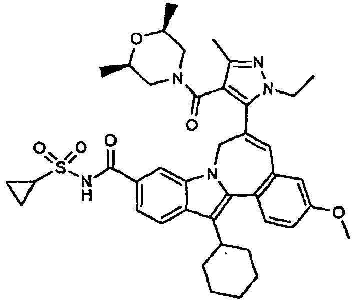

1*H*-pyrazole-4-carboxylic acid, 5-[13-cyclohexyl-10-[[(cyclopropylsulfonyl)amino]carbonyl]-3-methoxy-7*H-*indolo[2,1-*a*][2]benzazepin-6-yl]-1-elhyl-3-methyl- was dissolved in DMF (1.9mL) and HATU (160 mg, 0.421 mmol) added to the reaction. The reaction was stirred at room temperature for 1.5hrs. then DMAP (70.7 mg, 0.579 mmol) was added to the reaction followed by (2R, 6S)-2,6-dimethylmorpholine (71.1 µL, 0.574 mmol). The reaction was capped under a nitrogen atmosphere and stirred at room temperature for 17hrs. The product was purified on a Shimadzu high pressure liquid chromatography system employing Discovery VP software interfaced with a SCL-10A controller, SIL-10A autosampler and FRC-10A fraction collector. The reaction was diluted to 4ml with acetonitrile with a few drops of water to dissolve then filtered through a 0.45uM syringe filter and purified (2 x 2ml injections) using a Waters Sunfire Prep C 18 OBD, 5uM 19mm x 150mm column and monitored using a SPD-10AV UV-Vis detector at a detector wave length of 220mM. The elution conditions employed a flow rate of 25 mL / min , a gradient of 70 solvent A / 30% solvent B to 0% solvent A / 100% solvent B, a gradient time of 20 minutes with a run time of 30 minutes. using %A= 10% acetonitrile, 90% water, 0.1% TFA %B= 90% acetonitrile, 10% water, 0.1% TFA solvent system.

The title compound was dried *in vacuuo* at room temperature to yield 71.8mg of yellow amorphous solid.

1H NMR (500 MHz, CHLOROFORM-D) δ ppm 0.68 (s, 4 H) 0.81 (d, *J*=33.27 Hz, 2 H) 0.93 - -1.18 (m, 3 H) 1.20 1.30 (m, 1 H) 1.33 - 1.48 (m, 6 H) 1.50 - 1.61 (m, 1 H) 1.79 (d, *J*=10.99 Hz, 2 H) 1.97 (d, *J*=9.77 Hz, 3 H) 2.04 - 2.17 (m, 3 H) 2.26 (s, 3 H) 2.88 (t, *J*=11.29 Hz, 1 H) 3.05 - 3.29 (m, 3 H) 3.53 (s, 5 H) 3.95 (s, 3 H) 4.20 (s, 2 H) 4.57 (d, *J=*14.95 Hz, 1 H) 4.89 (d, *J*=15.56 Hz, 1 H) 6.75 - 6.87 (m, 1 H) 6.93 (s, 1 H) 7.13 (d, *J=*8.24 Hz, 1 H) 7.52 - 7.60 (m, 1 H) 7.63 - 7.77 (m, 2 H) 7.86 - 7.99 (m, 1 H) 10.72 (s, 1 H)

LC-MS retention time 1.50min; 738m/z (MH-). LC data was recorded on a Shimadzu LC-10AS liquid chromatograph equipped with a Phcnomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 0 min, and an analysis time of 3min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### methyl 1-isopropyl-5(3)-methyl-1H-pyrazole-4-carboxylate

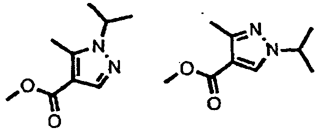

Dissolve methyl 3-methyl-1H-pyrazole-4-carboxylate (2.375g, 16.95 mmol) in DMF (85 mL), sodium hydride (0.471 g, 18.64 mmol) (95% in mineral oil) was added portion wise under nitrogen. The reaction was stirred for approximately 10 minutes and 2-iodopropane (1.861 mL, 18.64 mmol) was added to the reaction. The reaction was stirred at room temperature under a nitrogen atmosphere overnight (23hr). Solvent was removed from the reaction *in vacuuo* using a rotary evaporator. The orange residue was partitioned between 1.0N aqueous hydrochloric acid (∼30ml) and ethyl acetate. The yellow colored aqueous solution was extracted with ethyl acetate and the organic extracts were combined and washed 1x with saturated aqueous sodium bicarbonate solution. The aqueous hydrochloric acid washes were neutralized to pH of ∼4 with 10N aqueous sodium hydroxide then saturated aqueous sodium bicarbonate added to make the solution basic. The basic aqueous solutions were combined and extracted with ethyl acetate. The organic extracts were combined and washed with brine and dried over magnesium sulfate, filtered and solvent removed *in vacuuo* to yield a yellow oil (4.4g). The yellow oil was dissolved in benzene and volatiles removed *in vacuuo* using a rotary evaporator (bath temp= 40C) to obtain a yellow oil (weight= 2.964g). The material was dissolved in a small amount of dichlorornethane and applied to a silica gel column (105g) slurry loaded in dichloromethane. The product was eluted with dichloromethane to yield a mixture of isomers as a yellow oil (1.736g).

1H NMR (500 MHz, CHLOROFORM-D) δ ppm 1.46 (s, 2.29 H) 1.47 (s, 5.08 H) 1.48 (s, 3.03 H) 2.45 (s, 3.00 H) 2.54 (s, 1.99 H) 3.79 (s, 3.15 H) 3.79 (s, 1.91 H) 4.34 - 4.49 (m, 1.80 H) 7.83 (s, 1.02 H) 7.85 (s, 0.65 H).

### methyl 3(5)-iodo-1-isopropyl-5(3)-methyl-1H-pyrazole-4-carboaylate

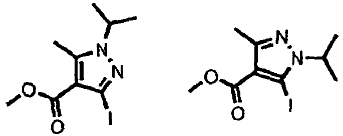

Vasilevskii, S.F.; Gerasimov, V.A; Shvartsbert, M.S.; Bulletin of the Academy of Sciences of the USSR, Division of Chemical Science (English Translation), 30 (4) 683-685 (1981), ISSN: 0568-5230; Izvestiya Akademii Nauk SSSR, Seriya Khimicheskaya, 4, 902-904 (1981). In a 25ml flask dissolve methyl 1-isopropyl-3(5)-methyl-1H-pyrazole-4-carboxylate mixture (550mg, 3.02 mmol) in acetic acid (3.2 ml), then add sulfuric acid (1.22ml, 6.87 mmol) (30% w/w) followed by iodine (620 mg, 2.443 mmol) and iodic acid (250 mg, 1.421 mmol). The reaction was heated at 95C under a nitrogen atmosphere for approximately 21hrs. The reaction was cooled and sodium acetate (750mg, 9.14 mmol) to the reaction. The reaction was transferred to a sepratory funnel in ethyl acetate and washed with 10% aqueous sodium sulfite. The organic layer was washed in an erlenmeyer with saturated aqueous sodium bicarbonate (Caution: CO2 evolution). The organic phase was decanted and washed with saturated aqueous sodium bicarbonate then brine. The organic extract was dried over magnesium sulfate, filtered and solvent removed *in vacuuo* using a rotary evaporator to give the product as a yellow oil (598mg). The crude mixture was used without further purification.

LC-MS retention time 1.4min unresolved peaks; 309m/z (MH+). LC data was recorded on a Shimadzu LC-10AS liquid chromatograph equipped with a Waters Sunfire C18 5u 4.6x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10mM ammonium acetate and solvent B was 95% H2O / 5% acetonitrile / 10mM ammonium acetate. MS data was determined using a Micromass Platform for LC in electrospray mode.

### 7H-indolo[2,1-a][2]benzazepine-10-carboxylic acid, 13-cyclohexyl-3-methoxy-6-[4-(methoxycarbonyl)-5-methyl-1-(1-methylethyl)-1H-pyrazol-3-yl]-, 1.1-dimethylethyl ester

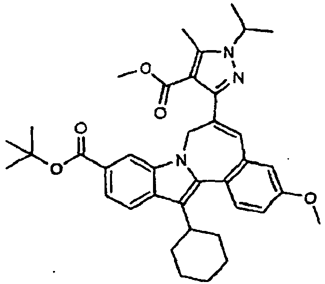

7H-indolo[2,1-a][2]benzazepine-10-carboxylic acid, 13-cyclohexyl-3-methoxy-6-(tributylstannyl}-, 1,1-dimethylethyt ester (679 mg, 0.927 mmol) was charged into a 20ml microwave vessel with copper(I) chloride (468 mg, 4.73 mmol), tetrakis(triphenylphosphine)palladium(0) (106.8 mg, 0.092 mmol) and lithium chloride (312 mg, 7.36 mmol) (lithium chloride flame dried and stored in vacuum at room temperature overnight). The reaction vessel was capped with a teflon lined septa and evacuated and back filled with nitrogen three times. A mixture containing methyl 3(5)-iodo-1-isopropyl-5(3)-methyl-1H-pyrazole-4-carboxylate (356 mg, 1.155 mmol) in DMSO (6.2mL) was added to the reaction vessel. The reaction was sparged for approximately 2 minutes with nitrogen and stirred for 5 minutes at room temperature then heated to 120C in an oil bath for 5hrs. Dilute reaction with ethyl acetate and wash sequentially with 5% aqueous ammonium hydroxide then brine. Back extract aqueous layer with diethyl ether. Combine organic extracts and wash sequentially with water then brine. Filter off a fine precipitate suspension in the organic layer then dry filtrate using magnesium sulfate. Filter drying agent and remove volatiles to yield the crude reaction products as a yellow oil (825mg). The crude product was adsorbed onto 2g of silica gel using dichloromethane and purified by column chromatography using 25.8g of silica gel slurry loaded in 30% hexanes in dichloromethane. The products were eluted from the column with 30% hexanes in dichloromethane then a gradient to dichloromethane, continued elution with 1% ethyl acetate in dichloromethane and finally a gradient to 5% ethyl acetate in dichloromethane. Pure fractions of the title compound were combined and volatiles removed to yield the title compound (165mg) a yellow film.

LC-MS retention time 3.91min; 624m/z (MH+). LC data was recorded on a Shimadzu LC-10AS liquid chromatograph equipped with a Waters Sunfire C18 5u 4.6x50mm column using a SPD-10AV UV-Vis detector at a detector wave length of 220nM. The elution conditions employed a flow rate of 5 ml/min, a gradient of 70% solvent A / 30% solvent B to 0% solvent A / 100% solvent B, a gradient time of 3 min, a hold time of 3 min, and an analysis time of 6 min where solvent A was 10% acetonitrile / 90% H2O / 0.1 % TFA and solvent B was 90% H2O /10% acetonitrile / 0.1% TFA. MS data was determined using a Micromass Platform for LC in electrospray mode.

1H NMR indicates some impurities present. The material was used without further purification.

1H NMR (500 MHz, CHLOROFORM-D) δ ppm 0.91 (t, *J=*7.48 Hz, 1.2 H) 1.16 - 1.41 (m, 5.6 H) 1.44 (d, *J*=6.71 Hz, 2.2 H) 1.46 -1.53 (m, 4.5 H) 1.55 (s, 6.8 H) 1.60 (s, 9.2 H) 1.61 - 1.67 (m, 1.7 H) 1.69 - 1.81 (m, 2.3 H) 1.93 (d, *J*=10.99 Hz, 1.2 H) 2.06 (s, 3.1 H) 2.48 - 2.50 (s, 0.7 H) 2.51 (s, 2.8 H) 2.77 - 2.83 (m, 0.4 H) 2.83 - 2.92 (m, 1.0 H) 3.70 (s, 2.9 H) 3.80 (s, 0.6 H) 3.90 (s, 3.0 H) 4.35 - 4.53 (m, 2.2 H) 5.59 (d, *J*=14.95 Hz, 0.9 H) 6.95 (d, *J=*2.75 Hz, 1.0 H) 6.99 (dd, *J*=8.55, 2.75 Hz, 1.0 H) 7.36 (s, 1.0 H) 7.50 (d, *J*=8.55 Hz, 1.1 H) 7.64 (dd, *J*=8.39, 1.37 Hz, 1.0 H) 7.80 (d, *J*=8.55 Hz, 1.0 H) 8.19 (s, 0.9 H).

### 7H-indolo[2.1-a][2]benzazepine-10-carboxylic acid, 13-cyclohexyl-3-methoxy-6-[4-(methoxycarbonyl)-5-methyl-1-(1-methylethyl)-1H-pyrazo/-3-y/]-

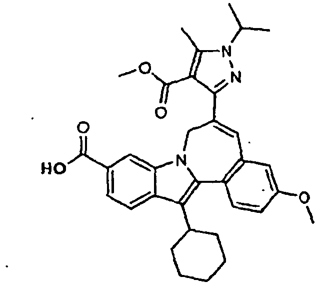

7*H*-indolo[2,1-a][2]benzazepine-10-carboxylic acid, 13-cyclohexyl-3-methoxy-6-[4-(methoxycarbonyl)-5-methyl-1-(1-methylethyl)-1*H*-pyrazol-3-yl]-, 1,1-dimethylethyl ester (152mg, 0.244 mmol) was dissolved in 1,2-dichloroethane (1.2 mL), then TFA (1.2 mL) was added to the reaction. The reaction was stirred at room temperature under a nitrogen atmosphere for 2hrs. Volatiles were removed from the reaction *in vacuuo* using a rotary evaporator. Dissolve residue in dichloromethane and benzene then remove volatiles *in vacuuo* using a rotary evaporator, repeat sequence. The resulting yellow foam was dissolved in dichloromethane and transferred to a tared round bottom flask The volatiles were removed *in vacuuo* an the resulting yellow foam was dried *in vacuuo* at room temperature to yield 147mg of the title compound. The material was used without further purification LC-MS retention time 2.24min; 566m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

LC-MS retention time3.09 min; 568m/z (MH+). LC data was recorded on a Shimadzu LC-10AS liquid chromatograph equipped with a Waters Sunfire C18 5u 4.6x50mm column using a SPD-10AV UV-Vis detector at a detector wave length of 220nM. The elution conditions employed a flow rate of 4 ml/min, a gradient of 100% solvent A / 0% solvent B to 0% solvent A /100% solvent B, a gradient time of 3 min, a hold time of 2 min, and an analysis time of 5 min where solvent A was 10% acetonitrile / 90% H2O / 0.1% TFA and solvent B was 90% H2O / 10% acetonitrile / 0.1% TFA. MS data was determined using a Micromass Platform for LC in electrospray mode.

### 1H-pyrazole-4-carboxylic acid, 3-[13-cyclohexyl-3-methoxy-10-[[[1-methylethyl)sulfonyl]amino]carbonyl]-7H-indolo[2,1-a][2]benzazepin-6-yl]-5-methyl-1-(1-methylethyl)-, methyl ester

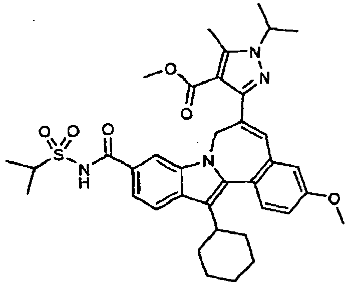

In a 2 dram vial suspend 7*H*-indolo[2,1-a][2]benzazepine-10-carboxylic acid, 13-cyclohexyl-3-methoxy-6-[4-(methoxycarbonyl)5-methyl-1-(1-methylethyl)-1*H-*pyrazol-3-yl]- in dichloromethane (2.5ml). To the reaction as a fine yellow suspension add propane-2-sulfonamide (96.7 mg, 0.785 mmol) and DMAP (105 mg, 0.859 mmol). Upon addition of DMAP, the reaction became a homogeneous clear yellow solution. EDC (77 mg, 0.402 mmol) was added to the reaction and the reaction was capped under a nitrogen atmosphere and stir at room temperature for 39hrs. The solvent was removed *in vacuuo* using a rotary evaporator and the reaction residue partitioned between ethyl acetate and 1.0N aqueous hydrochloric acid. The organic layer was washed sequentially with 1.0N aqueous hydrochloric acid, 0.1M aqueous NaH2P04 and 1.0N aqueous hydrochloric acid then brine. The organic layer was dried over magnesium sulfate, filtered and solvent remove *in vacuuo* to yield a yellow amorphous solid. The product was dried *in vacuuo* at room temperature. to give 154mg of the title compound as an amorphous yellow solid. The product was used without further purification.

LC-MS retention time 3.14min; 673m/z (MH+). LC data was recorded on a Shimadzu LC-10AS liquid chromatograph equipped with a Waters Sunfire C18 5u 4.6x50mm column using a SPD-10AV UV-Vis detector at a detector wave length of 220nM. The elution conditions employed a flow rate of 4 ml/min, a gradient of 100% solvent A / 0% solvent B to 0% solvent A / 100% solvent B, a gradient time of 3 min, a hold time of 2 min, and an analysis time of 5 min where solvent A was 10% acetonitrile / 90% H2O / 0.1% TFA and solvent B was 90% H2O / 10% acetonitrile / 0.1% TFA. MS data was determined using a Micromass Platform for LC in electrospray mode.

LC-MS retention time 2.16 min;671 ml) (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min , a gradient of 100% solvent A / 0% solvent B to 0% solvent A / 100% solvent B, a gradient time of 3 min, a hold time of 1 min, and an analysis time of 4 min where solvent A was 5% aectonitrile / 95% H2O / 10 mM ammonium acetate and solvent B was 5% H2O / 95% acetonitrile / 1.0 mM ammonium acetate. MS data was determined using a Micromass Platform for LC in electrospray mode.

### 1H-pyrazole-4-carboxylic acid 3-[13-cyclohexyl-3-methoxy-10-[[[(1-methylethyl)sulfonyl]amino]carbonyl]-7H-indolo[2,1-a][2]benzazepin-6-yl]-5-methyl-1-(1-methylethyl)-

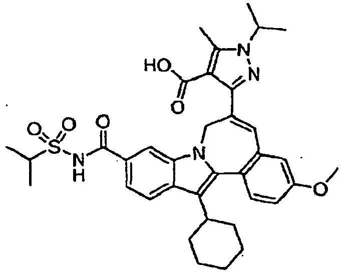

1*H-*pyazole-4-carboxylic acid, 3-[13-cyclohexyl-3-methoxy-10-[[[(1-methylethyl)sulfonyl]amino]carbonyl]-7*H*-indolo[2,1-*a*][2]benzazepin-6-yl]-5-methyl-1-(1-methylethyl)-, methyl ester (153mg, 0.227 mmol) was dissolved in THF (2.2 mL) and Methanol (2.2 mL). To the clear orange/amber solution aqueous 1.0N sodium hydroxide (2.2 mL, 2.200 mmol) was added. The reaction mixture turned a rose/red color and was slightly cloudy which became clear within 24hrs. The reaction was stirred under a nitrogen atmosphere at room temperature for 14 days. Progression of the reaction was measured at intervals of every few days by taking a 15ul aliquot diluting in acetonitrile and adding 1 to 2 drops of water to dissolve salts then analyzing the solution by LCMS.

The reaction was partitioned between 1.0N aqueous hydrochloric acid and ethyl acetate. The aqueous phase was extracted 1x using ethyl acetate and the organic extracts combined and washed with brine and dried over magnesium sulfate. Filtering and removing solvents *in vacuuo* using a rotary evaporator gave a orange amorphous solid. The crude product was dried *in vacuuo* at room temperature to yield 151mg of amorphous amber-orange solid. The crude product was used without further purification.

LC-MS retention time 2.85min; 659m/z (MH+). LC data was recorded on a Shimadzu LC-10AS liquid chromatograph equipped with a Waters Sunfire C 18 5u 4.6x50mm column using a SPD-10AV UV-Vis detector at a detector wave length of 220nM. The elution conditions employed a flow rate of 4 ml/min, a gradient of 100% solvent A / 0% solvent B to 0% solvent A / 100% solvent B, a gradient time of 3 min, a hold time of I min, and an analysis time of 4 min where solvent A was 10% acetonitrile / 90% H2O / 0.1% TFA and solvent B was 90% H2O / 10% acetonitrile / 0.1% TFA. MS data was determined using a Micromass Platform for LC in electrospray mode.

### 7H-indolo[2,1-a][2]benzazepine-10-carboxamide, 13-cyclohexyl-6-[4-[[(2R,6S)-2,6-dimethyl-4-morpholinyl]carbonyl]-5-methyl-1-(1-methylethyl)-1H-pyrazol-3-yl]-3-methoxy-N-[(1-melhylethyl)sulfonyl]-

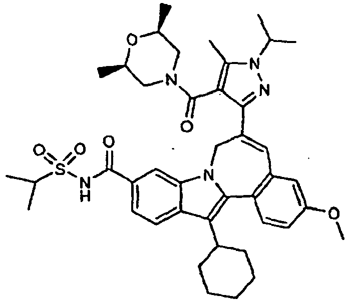

In a 2 diam vial, 1*H*-pyrazole-4-carboxylic acid,.3-[13-cyclohexyl-3-methoxy-10-[[[(1-methylethyl)sulfonyl]amino]carbonyl]-7*H*-indolo[2,1-*a*][2]benzazepin-6-yll-5-methyl-1-(1-methylethyl)- (50 mg, 0.076 mmol) was dissolved in DMF (759 µL) then HATU (66 mg, 0.174 mmol) was added to the reaction. The reaction was stirred at room temperature under a nitrogen atmosphere for 20 minutes and DMAP (30.3 mg, 0.248 mmol) was added to the reaction followed by the amine reagent, (2R,6S)-2,6-dimethylmorpholine (28.2 µL, 0.228 mmol). The reaction was capped under a nitrogen atmosphere and stirred at room temperature overnight (16hrs).

The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min, a gradient of 50% solvent A / 50% solvent B to 0% solvent A / 100% solvent B, a gradient time of 20 minutes with a run time of 30 minutes using %A= 10% acetonitrile, 90% water, 0.1% TFA %B= 90% acetonitrile, 10% water, 0.1% TFA solvent system. Volatiles from the product fraction at retention time 15.9 minutes were removed *in vacuuo* and the title compound was dried *in vacuuo* at room temperature to yield 25.9mg of a yellow amorphous solid.

1H NMR of sample shows characteristics of restricted rotation.

1H NMR (500 MHz, CHLOROFORM-D) δ ppm 0.80 (s, 1.2 H) 0.96 (d, *J*=4.58 Hz, 0.4 H) 1.07 (d, *J*=5.80 Hz, 2.3 H) 1.17 - 1.29 (m, 1.2 H) 1.31 - 1.40 (m, 1.1H) 1.39 - 1.45 (m, 1.1 H) 1.46 - 1.54 (m, 9.9 H) 1.56 (d, *J*=12.51 Hz, 0.6 H) 1.61 (d, *J*=6.41 Hz, 0.8 H) 1.65 (d, *J*=6.10 Hz, 0.5 H) 1.72 (t, *J*=6.41 Hz, 2.0 H) 1.78 (d, *J*=10.99 Hz, 2.0 H) 1.89 - 2.02 (m, 2.0 H) 2.02 - 2.17 (m, 2.8 H) 2.20 (s, 0.9 H) 2.27 (s, 2.0 H) 2.30 - 2.44 (m, 1.5 H) 2.49 (d, *J=*13.12 Hz, 0.5 H) 2.71 (br.s, 4.2 H) 2.81 - 3.01 (m, 2.3 H) 3.15 (s, 0.4 H) 3.27 (d, *J*=12.82 Hz, 0.5 H) 3.35 - 3.43 (m, 0.7 H) 3.47 - 3.57 (m, 0.5 H) 3.90 (d, *J*=4.58 Hz, 3.0 H) 4.01- 4.15 (m, 1.1 H) 432 (dd, *J*=20.29,14.19 Hz, 0.7 H) 4.38 - 4.55 (m, 2.0 H) 5.15 (d, *J*=15.56 Hz, 0.2 H) 5.46 (d, *J*=15.26 Hz, 0.1 H) 5.80 (d, *J*=14.34 Hz, 0.4 H) 5.93 (d, *J*=14.34 Hz, 0.3 H) 6.75 (s, 0.4 H) 6.83 - 6.93 (m, 0.6 H) 6.94 - 7.01 (m, *J*=22.89 Hz, 1.0 H) 7.03 (dd, *J*=8.55, 2.14 Hz, 1.0 H) 7.39 (t, *J*=7.17 Hz, 0.7 H) 7.52 (d, *J*=8.55 Hz, 1.1 H) 7.60 (d, *J*=8.24 Hz, 0.2 H) 7.82 - 7.98 (m, 1.5 H) 8.02 - 8.11 (m, 0.4 H) 8.34 (d, *J*=19.84 Hz, 0.6 H) 9.17 (s, 0.1 H) 10.02 (s, 0.2 H).

LC-MS retention time 1.96 min; 754m/z (MH-). LC data was recorded on a Shimadzu LC- 10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A / 100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzazepine-10-carboxamide, 13-cyclohexyl-3-methoxy-N-[(1-methylethyl)sulfonyl]-6-[5-methyl-4-[(3-methyl-3,8-diazabicyclo[3.2.1]oct-8-yl)carbonyl]-1-(1-methylethyl)-1H-pyrazol-3-yl]-

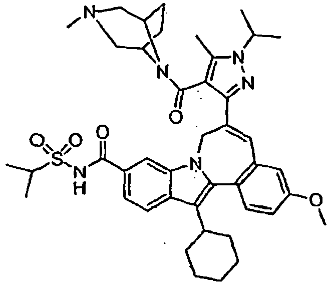

1*H*-pyrazole4-carboxylic acid, 3-[13-cyclohexyl-3-methoxy-10-[[[(1-methylethyl)sulfonyl]amino]carbonyl]-7*H*-indolo[2,1-*a*][2]benzazepin-6-yl]-5-methyl-1-(1-methylethyl)- (50 mg, 0.076 mmol) was dissolved in DMF (759 µL) and HATU (59.6 mg, 0.157 mmol) to the reaction. The reaction was stirred under a nitrogen atmosphere for 55 minutes then DMAP (52.0 mg, 0.426 mmol) was added to the reaction followed by the amine reagent, 3-methyl-3,8-diazabicyclo[3.2.1]octane dihydrochloride (37.8 mg, 0.190 mmol). The reaction was capped under a nitrogen atmosphere and stirred at room temperature for 63 hours. The product was purified on a Shimadzu high pressure liquid chromatography system employing Discovery VP software interfaced with a SCL-10A controller, SIL-10A autosampler and FRC-10A fraction collector. The reaction was diluted to 2ml with acetonitrile with 2 drops of trifluoroacetic acid added, filtered through a syringe filter and the filtrate purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min , a gradient of 70 solvent A / 30% solvent B to 0% solvent A / 100% solvent B, a gradient time of 20 minutes with a run time of 30 minutes using %A= 10% acetonitrile, 90% water, 0.1% TFA %B= 90% acetonitrile, 10% water, 0.1% TFA solvent system.

The retention time of the product was 8.84min. The volatiles/solvents were removed *in vacuuo* from the product fraction using a rotary evaporator and the product dried *in vacuuo* at room temperature to yield 27.4mg of the title compound as a yellow solid. The 1H NMR shows characteristics of restricted rotation and/or salt formation exhibiting broadening of peaks and multiple peak sets, integrals rounded to nearest whole number.

1H NMR (500 MHz, CHLOROFORM-D) δ ppm 0.59 (s, 1 H) 0.89 (s, 1 H) 1.22 (d, *J*=20.14 Hz, 1 H) 1.29 - 1.66 (m 12 H) 1.77 (d, *J*=10.38 Hz, 4 H) 1.87 - 2.14 (m, 5 H) 2.16 - 2.48 (m, 4 H) 2.79 - 3.07 (m, 3 H) 3.10 - 3.81 (m, 7 H) 3.90 (s, 3 H) 4.04 (d, *J*=39.06 Hz, 2 H) 4.48 (s, 2 H) 4.67 (s, 1 H) 4.99 (d, *J*=94.00 Hz, 1 H) 5.71 (s, 1 H) 5.98 (s, 1 H) 6.70 - 6.97 (m, 2 H) 7.04 (d, *J*=7.63 Hz, 1 H) 7.34 - 7.63 (m, 2 H) 7.76 - 7.94 (m, 2 H) 8.06 (s, 1 H) 9.34 (s, 1 H) 9.70 (s, 1 H)

LC-MS retention time 2.01 min;765 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A /100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzazepine-10-carboxamide, 13-cyclohexyl-3-methoxy-N-[(1-methylethyl)sulfonyl]-6-[5-methyl-1-(1-methylethyl)-4-[[(3R,5S)-3,4,5-trimethyl-1-piperazinyl]carbonyl]-1H-pyrazol-3-yl]-

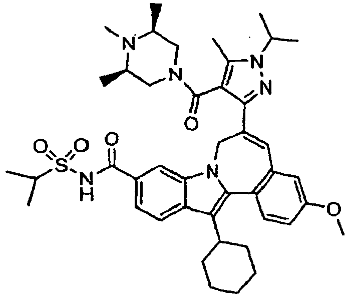

1*H*-pyrazole-4-carboxylic acid, 3-[13-cyclohexyl-3-methoxy-10-[[[(1-methylethyl)sulfonyl]amino]carbonyl]-7*H*-indolo[2,1-*a*][2]benzazepin-6-yl]-5-methyl-1-(1-methylethyl)- (46 mg, 0.070 mmol) was dissolved in DMF (698 µL) and HATU (61.6 mg, 0.162 mmol) was added to the reaction. The reaction was capped and stirred for 68 minutes at room temperature then DMAP (44.5 mg, 0.364 mmol) was added to the reaction followed by (2R,6S)-1,2,6-trimethylpiperazine dihydrochloride (35.9 mg, 0.178 mmol). The reaction was capped under a nitrogen atmosphere and stirred at room temperature for 17hrs.

The product was purified on a Shimadzu high pressure liquid chromatography system employing Discovery VP software interfaced with a SCL-10A controller, SCL-10A autosampler and FR-C-10A fraction collector. The reaction was diluted to 2ml with acetonitrile and 2 drops of TFA added to ensure protonation then filtered through a 0.45uM syringe filter and purified using a Waters Sunfire Prep C18 OBD, 5uM 19mm x 150mm column and monitored using a SPD-10AV UV-Vis detector at a detector wave length of 220nM. The elution conditions employed a flow rate of 25 mL / min , a gradient of 70 solvent A / 30% solvent B to 0% solvent A / 100% solvent B, a gradient time of 20 minutes with a run time of 20 minutes using %A= 10% acetonitrile, 90% water, 0.1% TFA %B= 90% acetonitrile, 10% water, 0.1 % TFA solvent system.

Volatiles were removed from the product fractions *in vacuuo* overnight using a speed-vac on the medium heating setting. The product fraction were combined in dichloromethane then solvent removed *in vacuuo* and the product was dried in vacuuo at room temperature to give 27.0mg of the title compound as a brown-tan amorphous solid.

1H NMR is characteristic of restricted rotation and or salt formation.

1H NMR (500 MHz, CHLOROFORM-D) δ ppm 0 - 0.61 (m, 2.7 H) 0.91 (t, *J*=7.32 Hz, 0.8 H) 0.98 - 1.14 (m, 2.6 H) 1.17 - 1.44 (m, 5.9 H) 1.48 (s, 10.7 H) 1.57 - 1.86 (m, 5.3 H) 1.88 - 2.12 (m, 4.2 H) 2.13 - 2.38 (m, 5.0 H) 2.40 - 2.82 (m, 9.1 H) 2.84 - 3.06 (m, 1.9 H) 3.09 - 3.80 (m, 2.7 H) 3.91 (d, *J*=10.07 Hz, 3.0 H) 4.07 (d, *J*=10.99 Hz, 1.3 H) 4.22 - 4.40 (m, 0.6 H) 4.42 - 4.51 (m, 1.0 H) 4.55 (d, *J*=13.73 Hz, 0.3 H) 4.68 (dd, *J*=30.98,14.80 Hz, 0.5 H) 5.04 (d, *J*=15.87 Hz, 0.3 H) 5.83 (d, *J*=15.26 Hz, 0.3 H) 6.01 (d, *J*=15.56 Hz, 0.2 H) 6.68 - 6.78 (m, 0.3 H) 6.91 (d, *J*=27.47 Hz, 0.7 H) 6.96 - 7.11 (m, 1.9 H) 7.38 (dd, *J*=1526, 7.93 Hz, 0.6 H) 7.48 - 7.58 (m, 1.0 H) 7.72 (d, *J*=7.93 Hz, 0.3 H) 7.86 (d, *J*=8.24 Hz, 0.8 H) 7.93 (s, 0.9 H) 8.08 (s, 0.3 H) 8.37 (d, *J*=33.57 Hz, 0.5 H) 9.94 (s, 0.3 H) 11.49 (s, 0.4 H).

LC-MS retention time 2.04min;767 m/z (MH-). LC data was recorded on a Shimadzu LC-10AS liquid chromatograph equipped with a Phenomenex-Luna 10u C18 3.0x50mm column using a SPD-10AV UV-Vis detector at a detector wave length of 220nM. The elution conditions employed a flow rate of 5 ml/min, a gradient of 100% solvent A / 0% solvent B to 0% solvent A /100% solvent B, a gradient time of 3 min, a hold time of 1 min, and an analysis time of 4 min where solvent A was 5% acetonitrile / 95% H2O / 10 mM ammonium acetate and solvent B was 5% H2O / 95% acetonitrile / 10 mM ammonium acetate. MS data was determined using a Micromass Platform for LC in electrospray mode.

### 7H-indolo[2,1-a][2]benzazepine-10-carboxylic acid,13-cyclohexyl-6-[1-ethyl-5-(methoxycarbonyl)-3-methyl-1H-pyrazol-4-yl]-3-methoxy-,1,1-dimethylethyl ester

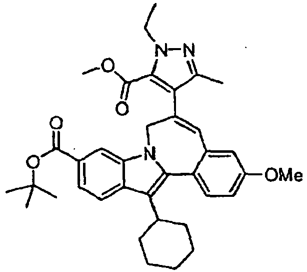

In a microwave tube, 7*H*-indolo[2,1-*a*][2]benzazepine-10-carboxylic acid, 13-cyclohexyl-3-methoxy-6-(tributylstannyl)-, 1,1-dimethylethyl ester (1.10g, 1.50 mmol), methyl 4-bromo-1-ethyl-3-methyl-1H-pyrazole-5-carboxylate (482 mg, 1.92 mmol) and bis(triphenylphosphine)palladium II chloride (105 mg, 0.150 mmol), LiCl (318 mg, 7.51 mmol), CuCl (624 mg, 6.31 mmol) were added. It was then sealed, degassed and flushed with nitrogen. 1,4-Dioxane (7.51 mL) was added. The reaction mixture was heated at 120°C under microwave condition for 1 hour. It was then filtered and the filtrate was concentrated. The residue was purified on a Shimadzu high pressure liquid chromatography system employing Discovery VP software interfaced with a SCL-10A controller, SIL-10A autosampler and FRC-10A fraction collector. The sample was dissolved in acetonitrile / DMF (1:1) (8ml) purified using a Waters Sunfire Prep C18 OBD, 5uM 19mm x 100mm column and monitored using a SPD-10AV UV-Vis detector at a detector wave length of 220aM. The elution conditions employed a flow rate of 25 mL / min , a gradient of 25% solvent A / 75% solvent B to 0% solvent A / 100% solvent B, a gradient time of 10 minutes with a run time of 20 minutes using %A= 10% acetonitrile, 90% water, 0.1% TFA %B= 90% acetonitrile, 10% water, 0.1% TFA solvent system.

The product-containing fractions were collected and concentrated to give title compound as a yellow oil (916 mg, 0.150 mmol, 40% yield).

MS m/z 610 (MH⁺).

### 7H-indolo[2,1-a][2]benzazepine-10-carboxylic acid, 13-cyclohexyl-6-[1-ethyl-5-(methoxycarbonyl)-3-methyl-1H-pyrazol-4-yl]-3-methoxy-

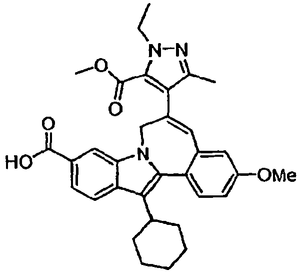

To a solution of 7H-indolo[2,1-a][2]benzazepine-10-carborylic acid, 13-cyclohexyl-6-[1-ethyl-5-(methoxycarbonyl)-3-methyl-1H-pyrazol-4-yl]-3-methoxy-, 1,1-dimethylethyl ester (290 mg, 0.476 mmol) in 1,2-dichloroethane (0.951 mL), TFA (0.951 mL) was added. The reaction mixture was stirred at RT for 2 hours. Volatiles were removed on a rotary evaporator to give the title compound as a brownish thick oil as crude product (263 mg, 0.476 mmol, 100 % yield).

MS m/z 568(MH⁺).

### 1H-pyrazole-5-carboxylic acid, 4-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-, methyl ester

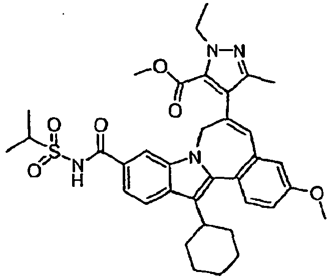

To a solution of 7H-indolo[2,1-a][2]benzazepine-10-carboxylic acid, 13-cyclohexyl-6-[1-ethyl-5-(methoxycarbonyl)-3-methyl-1H-pyrazol-4-yl]-3-methoxy-(260 mg, 0.470 mmol) in tetmhydrofuran (1.57 mL) was added carbonyldiimidazole (228 mg, 1.41 mmol). The reaction mixture was heated at 60°C for one hour. Propane-2-sulfonamide (231 mg, 1.88 mmol) and DBU (0.212 mL, 1.41 mmol) were added at room temperature. The reaction mixture was then heated at 60°C for 2 hours. The reaction mixture was diluted with 1N HCl (50 mL) solution and extracted with chloroform (50mL). The organic layers were combined and concentrated on a rotory evaporator to give an orange oil as crude product. It was purified through silica gel using 90/9/1 methylene chloride/MeOH/AcOH as the eluent to give the title compound (269 mg, 0.409 mmol, 87 % yield) as a yellow oil.

MS m/z 659 (MH⁺).

### 1H-pyrazole-5-carboxylic acid, 4-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-

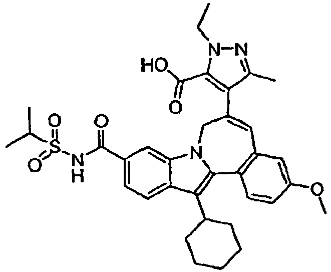

To a mixture of 1H-pyrazole-5-carboxylic acid, 4-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-, methyl ester (260 mg, 0.395 mmol) was dissolved in dioxane (1.97 mL) and methanol (1.97 mL) was added to the reaction followed by 1N aqueous sodium hydroxide (3.95 mL). The reaction was capped under a nitrogen atmosphere and stirred at room temperature for 72 hrs. The reaction was diluted with chloroform (50.0 mL) and washed with 1.0N aqueous hydrochloric acid (50ml). The organic layer was concentrated *in vacuuo* using a rotary evaporator to yield the title compound as a yellow solid (252 mg, 0.391 mmol, 99%).

MS m/z 645 (MH⁺).

### 7H-indolo[2,1-a][2]benzazepine-10-carboxamide, 13-cyclohexyl-6-[1-ethyl-3-methyl-5-(4-morpholinylcarbonyl)-1H-pyrazol-4-yl]-3-methoxy-N-[(1-methylethyl)sulfonyl]-

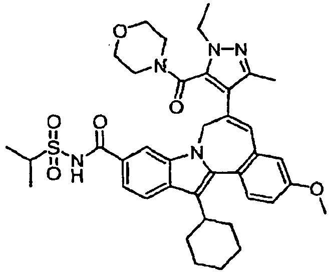

To a solution of 1H-pyrazole-5-carboxylic acid, 4-[13-cyclohexyl-3-methoxy-10-[[[(1-methylethyl)sulfonyl]amino]carbonyl]-7H-indolo[2,1-a][2]benzazepin-6-yl]-1-ethyl-3-methyl- (35 mg, 0.054 mmol) in DMF (0.54 mL), TBTU (35 mg, 0.109 mmol) and DIPEA (0.028 mg, 0.22 mmol) were added. The reaction mixture was stirred at RT for 15 min. Then morpholine (19 mg, 0.22 mmol) was added. The solution was stirred at RT for overnight. The reaction mixture was purified by prep HPLC column using CH₃CN/H₂O/TFA as solvent system. Fractions were collected and concentrated under speedvac overnight to yield the title compound as a beige solid (26 mg, 0.036 mmol, 81 % yield).

MS m/z 714 (MH⁺)

1H NMR (500 MHz, CHLOROFORM-D) δ ppm 1.19 - 1.62 (m, 13 H) 1.76 - 2.30 (m, 6 H) 2.41 (s, 3 H) 2.60 (m, 1H) 2.77 - 3.22 (m, 8 H) 3.94 (s, 3 H) 4.13 (m, 2 H) 4.30 (m 1 H) 4.63 (br d, 1 H) 4.83 (br d, 1 H) 6.72 (s, 1 H) 6.97 (s, 1 H) 7.12 (d, *J*=8.24 Hz, 1 H) 7.65-7.70 (m, 3 H) 7.98 (d, *J*=8.24 Hz, 1 H) 10.3 (s, 1 H).

The following compounds were synthesized by an analogous method as described above for 7H-indolo[2,1-a][2]beozazepine-10-carboxamide, 13-cyclohexyl-6-[1-ethyl-3-methyl-5-(4-morpholinylcarbonyl)-1H-pyrazol-4-yl]-3-methoxy-N-[(1-methylethyl)sulfonyl]-:

### 7H-indolo[2,1-a][2]benzazepine-10-carboxamide, 13-cyclohexyl-6-[5-[[(2R,6S)-2,6-dimethyl-4-morpholinyl]carbonyl]-1-ethyl 3-methyl-1H-pyrazol-4-yl]-3-methoxy-N-[(1-methylethyl)sulfonyl]-

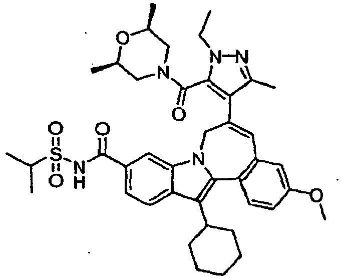

MS m/z 742 (MH⁺). 1H NMR (500 MHz, CHLOROFORM-D) δ ppm 0.75 (m, 6 H) 1.05 - 1.62 (m, 13 H) 1.76 - 2.26 (m, 6 H) 2.33 (m, 1H) 2.39 (s, 3 H) 2.81 - 3.29 (m, 4 H) 3.70-4.01 (m, 5 H) 4.14 (m, 2 H) 4.27 (m, 1 H 4.63 (br d, 1 H) 4.83 (br d, 1 H) 6.70 (s, 1 H) 6.98 (s, 1 H) 7.18 (br d, 1 H) 7.65-7.81 (m, 3 H) 7.96 (d, *J*=8.24 Hz, 1 H) 10.49 (s, 1 H).

### 7H-indolo[2,1-a][2]benzazepine-10-carboxamide, 13-cyclohexyl-6-[1-ethyl-3-melhyl-5-[(4-melhyl-1-piperazinyl)carbonyl]-1H-pyrazol-4-yl]-3-methoxy-N-[(1-methylethyl)sulfonyl]-

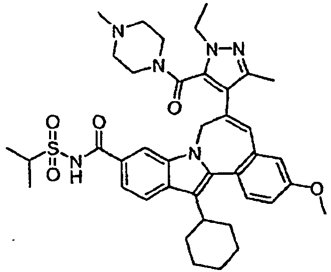

MS m/z 727 (MH⁺). 1H NMR (500 MHz, CHLOROFORM-D) δ ppm 1.22 - 1.62 (m, 13 H) 1.76 - 2.20 (m, 6 H) 2.39 (br s, 6 H) 2.78 - 3.90 (m, 9 H) 3.96 (s, 3 H) 4.08-4.25 (m, 3 H) 4.63 (br d, 1 H) 4.73 (br d, 1 H) 6.78 (s, 1 H) 6.99 (s, 1 H) 7.12 (br d, 1 H) 7.62-7.82 (m, 3 H) 7.98 (br d, 1 H) 10.02 (br s, 1 H).

### 7H-indolo[2,1-a][2]benzazepine-10-carboxamide. 13-cyclohexyl 6-[1-ethyl-3-methyl-5-[[(3R,5S)-3,4,5-trimethyl-1-piperazinyl]carbonyl]-1H-pyrazol-4-yl]-3-methoxy-N-[(1-methy/ethyl)sulfonyl]-

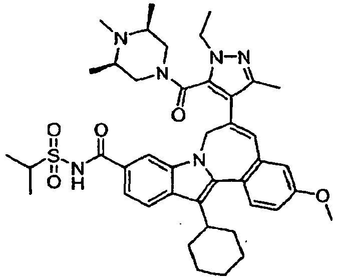

MS m/z 755 (MH⁺). 1H NMR (500 MHz, CHLOROFORM-D) δ ppm 1.04 (m 6 H) 1.25 - 1.62 (m, 13 H) 1.76 - 2.13 (m, 6 H) 2.31 (br s, 3 H) 2.40 (s, 3 H) 2.99-3.12 (m, 3 H) 3.35 - 3.52 (m, 2 H) 3.90-3.99 (m, 4 H) 4.05-4.25 (m, 4 H) 4.64 (br d, 1 H) 4.85 (br d, 1 H) 6.76 (s, 1 H) 6.98 (s, 1 H) 7.13 (d, *J*=8.24 Hz, 1 H) 7.59 (d, *J*=8.24 Hz, 1 H) 7.82 (m, 2 H) 8.00 (d, *J*=8.24 Hz, 1 H) 10.05 (s, 1 H).

### Preparation of compound 1: 13-cyclohexyl-6-[1-cyclopropyl-4-[[(2R,6S)-26-dimethyl-4-morpholinyl]carbonyl]-1H-imidazol-5-yl]-3-methoxy-N-[(1-methylethyl)sulfonyl]-7H-indolo[2,1-a][2]benzazepine-10-carboxamide

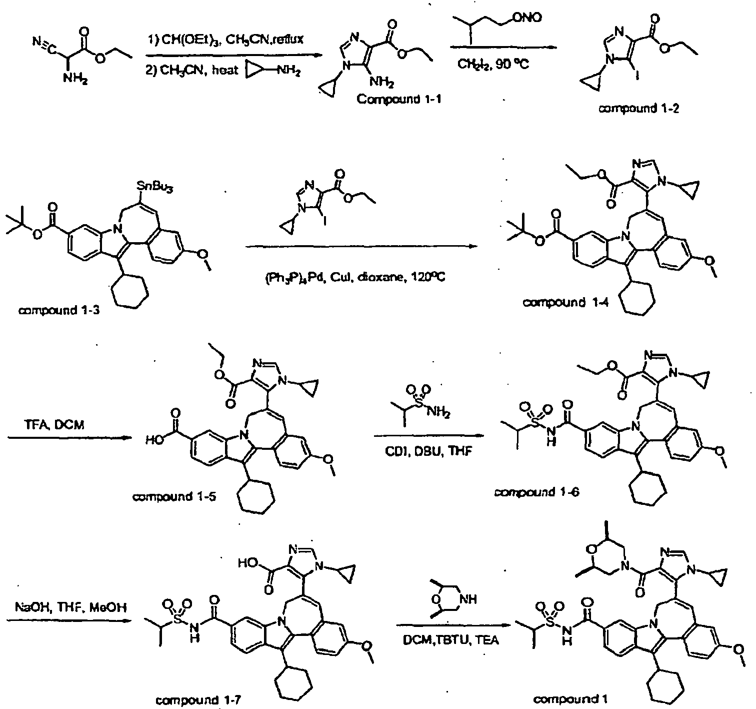

### Step 1: Preparation of compound 1-1

To a solution of ethyl 2-amino-2-cyanoacetate (1.8 g, 15 mmol) in Acetonitrile (25 mL) was added triethoxymethane (2.7 mL, 16.3 mmol). The clear brown solution was refluxed for 1 h, removed the solvent in vacuum to afford a residue.

A mixture of the above residue and cyclopropanamine (1.2 mL, 17.32 mmol) in Acetonitrile (15 mL) in a sealed tube was heated at 50 °C for 0.5 h and 85 °C for 3h, cooled and then removed the solvents. The residue was dissolved in DCM, washed with 2 N NaOH, dried (MgSO4), removed the solvent to afford compound 1-1 as a beige solid (1.8 g, 62%). The crude product was directly used in next reaction.

### Step 2: Preparation of compound 1-2

To a solution of isopentyl nitrite (3.70 mL, 27.7 mmol) in CH₂I₂ (10 mL, 124 mmol) was added dropwise a solution of compound **1-1** (1.8 g, 9.22 mmol) in chloroform (10 mL) at 90°C (bath temperature) over 5 min, 5 ml CHCl₃ was used to washed the flask to complete the transfer. After the reaction mixture was stirred for 1h at 90°C, the solvents were partially removed and the remaining mixture was purified on Thomson 80g column (EtOAc/hexane: 0 to 100%) to afford compound 1-2 as a reddish semi-solid (0.71g, 25%). LC-MS retention time: 1.682 min; MS m/z 307 (M+H)⁺. 1H NMR (400 MHz, *CHLOROFORM-d)* δ ppm 7.69 (1 H, s), 4.39 (2 H, q, *J*=7.05 Hz), 3.12 - 3.27 (1 H, m), 1.41 (3 H, t, *J*=7.18 Hz), 1.16 - 1.26 (2 H, m), 0.96 - 1.07 (2 H, m).

### Step 3: Preparation of compound 1-4

Compound **1-2** (0.6 g, 1.960 mmol), Tetrakis (0.158 g, 0.136 mmol), copper (I) iodide (0.260 g, 1.365 mmol) and compound **1-3** (1 g, 1.365 mmol) was added in a microwave vial. Capped the vessel, evacuated and filled with nitrogen. Dioxane (5 ml) was added and the mixture was evacuated and refilled with nitrogen. The reaction mixture was heated in oil bath at 120°C for 4 h. Removed the solvent and the residue was purified on Thomson 80g column (EtOAc/hexane: 0 to 80%) to afford compound **1-4** as a pale yellow solid (0.24 g, 28%). LC-MS retention time: 3.603 min; MS m/z 622 (M+H)⁺. 1H NMR (400 MHz, *CHLOROFORM-d*) δ ppm 7.78 - 7.84 (2 H, m), 7.37 - 7.70 (2 H, m), 7.04 (1 H, dd, *J*=8.69, 2.64 Hz), 6.92 (1 H, d, *J*=2.52 Hz), 6.82 (1 H, s), 5.09 (1 H, d, *J*=14.35 Hz), 4.70 (1 H, d, *J*=15.61 Hz), 4.38 (1 H, q, *J*=7.05 Hz), 3.89 (3 H, s), 2.77 - 2.91 (2 H, m), 1.99 - 2.17 (2 H, m), 1.85 - 1.98 (1 H, m), 1.67 - 1.83 (2 H, m), 1.57 (9 H, s), 1.39 (3 H, t, *J*=7.05 Hz), 1.12 - 1.65 (7 H, m), 0.70 (1 H, br. s.), 0.59 (1 H, br. s.), 0.03 (1 H, br. s.), -0.66 (1 H, br. s.).

### Step 4: Preparation of compound 1-5

To a solution of compound **1-3** (0.24 g, 0.386 mmol) in DCM (0.5 mL) was added TFA (1 mL, 12.98 mmol). The mixture was stirred at rt for 1.5 h, removed the solvents in vacuum to afford compound 1-5 as a brown foam (0.218 g, 100%). LC-MS retention time: 3.300 min; MS m/z 566 (M+H)⁺.

### Step 5: Preparation of compound 1-6

A mixture of compound **1-4** (0.218 g, 0.385 mmol) and CDI (0.125 g, 0.771 mmol) in THF (5ml) was stirred at 50°C for 0.5 h, cooled down and added propane-2-sulfonamide (0.100 g, 0.812 mmol) and DBU (0.2 ml, 1.327 mmol). The mixture was stirred at ambient temperature for 3 days and diluted with EtOAc and washed with HCl (0.1 N), brine, removed the solvent, purified on Thomson 25g column (MeOH/DCM: 0 to 25%) to afford compound **1-6** (0.136 g, 53%). LC-MS retention time: 3.208 min; MS m/z 671 (M+H)⁺. 1H NMR (500 MHz, *CHLOROFORM-d*) δ ppm 8.12 (1H, br. s.), 7.89 (1 H, d, *J*=8.55 Hz), 7.52 (2 H, d, *J*=8.55 Hz), 7.32 (1 H, br. s.), 7.06 (1 H, dd, *J*=8.70, 2.59 Hz), 6.93 (1 H, d, *J*=2.44 Hz), 6.82 (1 H, s), 5.23 (1 H, d, *J*=14.65 Hz), 4.71 (1 H, d, *J*=14.34 Hz), 4.29 (2 H, br. s.), 3.97 - 4.06 (1 H, m), 3.90 (3 H, s), 2.78 - 2.93 (2 H, m), 1.86 - 2.23 (4 H, m), 1.12 - 1.84 (15 H, m), 0.72 (1 H, br. s.), 0.59 (1 H, br. s.), 0.03 (1 H, br. s.), -0.50 (1 H, br. s.).

### Step 6: Preparation of compound 1-7

A mixture of compound **1-5** (0.136 g, 0.203 mmol) in THF (3 mL), NaOH (1.5 mL, 1.500 mmol), and MeOH (1.5 mL) was stirred at rt over night, diluted with EtOAc and washed with 0.2 N HCl, brine, dried (MgSO4), removed the solvent to afford compound **1-7** as a yellow solid (0.102 g, 78%). LC-MS retention time: 3.055 min; MS m/z 643 (M+H)⁺. 1H NMR (400 MHz, *CHLOROFORM-d*) δ ppm 8.23 (1 H, br. s.), 7.84 (2 H, br. s.), 7.37- 7.47 (1 H, m), 7.08 (1 H, br. s.), 6.97 (1 H, dd, *J*=8.69, 2.39 Hz), 6.71 - 6.84 (1 H, m), 5.81 (1 H, d, *J*=14.60 Hz), 4.68 (1 H, d, *J*=15.11 Hz), 3.89 - 3.97 (1 H, m), 3.83 (3 H, s), 2.85 (2 H, br. s.), 1.12 - 2.22 (17 H, m), 0.74 (1 H, br. s.), 0.46 (1 H, br. s.), -0.12 (1 H, br. s.), -0.30 (1 H, br. s.).

### Step 7: Preparation of compound 1

A mixture of compound **1-6** (0.082 g, 0.128 mmol), DCM (1 mL), TEA (0.1 ml, 0.717 mmol), (2R,6S)-2,6-dimethylmorpholine (0.03 mL, 0.244 mmol), and TBTU (0.060 g, 0.187 mmol) was stirred for 3 h and quenched with MeOH and then removed the solvents. The residue was purified by Prep-HPLC to afford compound 1 and isolated as mono TFA salt (0.0361 g, 31%). LC-MS retention time: 3.170 min; MS m/z 740 (M+H)⁺; 1H NMR (400 MHz, *CHLOROFORM-d*) δ ppm 8.88 (1 H, br. s.), 7.89 - 8.11 (2 H, m), 7.72 (1 H, d, *J*=8.56 Hz), 7.62 (1 H, d, *J*=8.31 Hz), 7.54 - 7.65 (1 H, m), 7.05 - 7.19 (2 H, m), 7.00 (1 H, br. s.), 5.03 - 5.19 (1 H, m), 4.62 (1 H, d, *J*=14.35 Hz), 4.01 - 4.12 (1 H, m), 3.97 (3 H, s), 3.02 - 3.61 (4 H, m), 2.85 - 2.99 (1 H, m), 1.65 - 2.55 (9 H, m), 0.97 - 1.63 (14 H, m), 0.89 - 0.97 (1 H, m), 0.73 - 0.86 (3 H, m), 0.66 (2 H, br. s.).

### Preparation of compound 2: 13-cyclohexyl-6-[1-cyclopropyl-4-(4-morpholinylcarbonyl)-1H-1-imidazol-5-yl]-3-methoxy-N-[(1-methylethyl)suyonyl]-7H-indolo[2,1-a][2]benzazepine-10-carboxamide

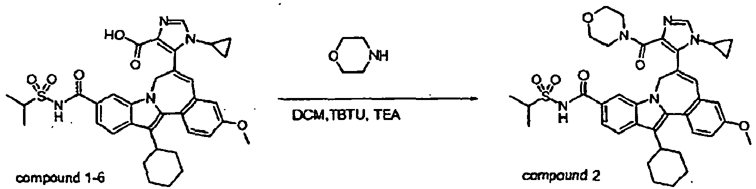

A mixture of compound **1-6** (0.020 g, 0.031 mmol), DCM (1 mL), morpholine (0.01mL, 0.115 mmol), TEA (0.05 ml, 0.359 mmol), and TBTU (0.030 g, 0.093 mmol) was stirred at rt for 3 h and quenched with MeOH, and then removed the solvents. The residue was purified by Prep-HPLC to afford compound 2 and isolated as mono TFA salt (0.0072 g, 28%). LC-MS retention time: 3.071 min; MS m/z 712 (M+H)⁺; 1H NMR (400 MHz, *CHLOROFORM-d*) δ ppm 8.69 (1 H, br. s.), 7.88 - 7.97 (2 H, m), 7.63 (1 H, d, *J*=8.56 Hz), 7.57 (1 H, d, *J*=8.81 Hz), 7.13 (1 H, dd, *J*=8.56, 2.52 Hz), 7.06 (1 H, s), 6.96 (1 H, d, *J*=2.52 Hz), 5.06 (1 H, d, *J*=14.60 Hz), 4.61 (1 H, d, *J*=15.36 Hz), 3.95 - 4.05 (1 H, m), 3.93 (3 H, s), 2.71 - 3.55 (10 H, m), 2.4-4 (1 H, br. s.), 1.89 - 2.21 (4 H, m), 1.71 - 1.85 (2 H, m), 1.14 - 1.63 (10 H, m), 0.92 - 1.11 (4 H, m).

### Preparation of compound 3: 6-[1-cyclobutyl-4-[[(2R,6S)-2,6-dimethyl-4-morpholinyl]carbonyl]-1H-imidazol-5-yl]-13-cyclohexyl-3-methoxy-N-[(1-methylethyl)sulfonyl]-7H-indolo[2,1-a][2]benzazepine-10-carboxamide

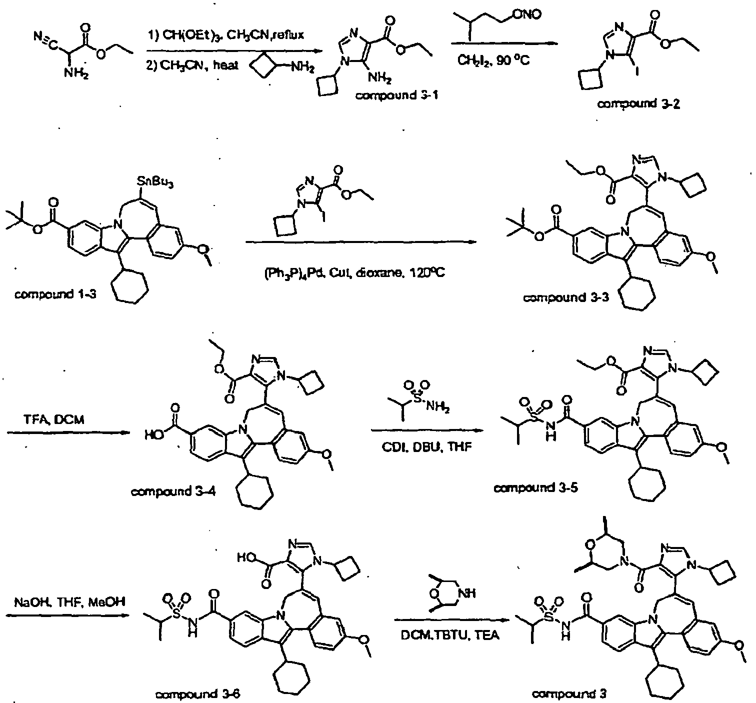

### Step 1: Preparation of compound 3-1

Compound **3-1** was prepared by using a similar method as described in synthesis of compound **1-1** except cyclobutanamine (1.4 mL, 16.40 mmol) was used in place of cyclopropanamine (1.8g, 62%). The crude product was directly used in the next reaction.

### Step 2: Preparation of compound 3-2

To a solution ofisopentyl nitrite (3.26 mL, 24.37 mmol) in diiodomethane (10 mL, 124 mmol) was added dropwise a solution of ethyl 5-amino-1-cyclobutyl-1H-imidazole-4-carboxylate (1.7 g, 8.12 mmol) in chloroform (10 mL) at 90°C (bath temperature) over 5 min, 5 ml CHC13 was used to washed the flask to complete the transfer. The mixture was stirred for 1h at the temperature. The solvent was partially removed and then purified on Thomson 110g column (EtOAc/hexane:0 to 100%) to afford a reddish oil which was re-purified on Thomson 90g column (EtOAc/hexane: 0 to 100%) to afford compound **3-2** as a brown solid (0.8 g, 31%). LC-MS retention time: 1.888 min; MS m/z 321 (M+H)⁺; 1H NMR (400 MHz, *CHLOROFORM-d*) δ ppm 7.83 (1 H, s), 4.58 - 4.71 (1 H, m), 4.37 (2 H, q, *J*=7.22 Hz), 2.56 - 2.68 (2 H, m), 2.25 - 2.42 (2 H, m), 1.88 - 2.00 (2 H, m), 1.39 (3 H, t, *J*=7.18 Hz).

### Step 3: Preparation of compound 3-3

In a microwave vial was added ethyl 1-cyclobutyl-5-iodo-1H-imidazole-4-carboxylate (0.7 g, 2.187 mmol), Tetrakis (0.158 g, 0.136 mmol), copper (I) iodide (0.260 g, 1.365 mmol) and compound **1-3** (1 g, 1.365 mmol). Capped the vessel evacuated and filled with nitrogen. Dioxane (10 ml) was added and the mixture was evacuated and refilled with nitrogen. The reaction mixture was heated in a oil bath at 120 °C for 4 h, concentrated and the residue was purified on Thomson 80g column (EtOAc/hexane; 0 to 100%) to afford compound **3-3** as a beige solid (0.22 g, 25%). LC-MS retention time: 3.636 min; MS m/z 636 (M+H)⁺; 1H NMR (400 MHz, *CHLOROFORM-d*) δ ppm 7.77 - 7.83 (2 H, m), 7.57 - 7.70 (1 H, m), 7.41 - 7.56 (1 H, m), 7.04 (1 H, dd, *J*=8.56, 2.77 Hz), 6.90 (1 H, d, *J*=2.52 Hz), 6.68 (1 H, s), 5.02 (1 H, d, *J*=14.10 Hz), 4.68 (1 H, d, *J*=14.35 Hz), 4.37 (2 H, br. s.), 3.89 - 4.00 (1 H, m), 3.89 (3 H, s), 2.76 - 2.93 (1 H, m), 2.26 - 2.43 (1 H, m), 1.65 - 2.21 (9 H, m), 1.58 (9 H, s), 0.84 - 1.52 (10 H, m).

### Step 4: Preparation of compound 3-4

To a solution of compound **3-3** (0.24 g, 0.377 mmol) in DCM (0.5 mL) was added TFA (1 mL), 12.98 mmol). The mixture was stirred at rt for 2h, and then removed the solvents in vacuum to afford the compound **3-4** as a brown foam (0.219 g, 100%). LC-MS retention time: 3.320 min; MS m/z 580 (M+H)⁺.

### Step 5: Preparation of compound 3-5

A mixture of compound 3-4 (0.219 g, 0.378 mmol) and CDI (0.122 g, 0.752 mmol) in THF (5ml) was stirred at 50°C for 0.5 h, cooled and added propane-2-sulfonamide (0.100 g, 0.812 mmol) and DBU (0.2 ml, 1.327 mmol). The mixture was stirred at ambient temperature for 3 days. The reaction mixture was diluted with EtOAc, washed HCl (0.1N), brine and dried (MgSO4). Crude product was purified on a Thomson 25g column (MeOH/DCM: 0 to 25%) to afford the compound **3-5** (0.087 g, 34%). LC-MS retention time: 3.230 min; MS m/z 685 (M+H)⁺; 1H NMR (500 MHz, *CHLOROFORM-d*) δ ppm 830 (1 H, br. s.), 7.89 (1 H, d, *J*=8.55 Hz), 7.54 (1 H, br. s.), 7.52 (1 H, d, *J*=8.85 Hz), 7.28 - 7.36 (1 H, m), 7.06 (1 H, dd, *J*=8.55, 2.44 Hz), 6.92 (1 H, d, *J*=2.44 Hz), 6.70 (1 H, s), 5.24 (1 H, br. s.), 4.69 (1 H, d, *J*=14.04 Hz), 4.29 (2 H, br. s.), 3.91 - 4.07 (2 H, m), 3.91 (3 H, s), 2.82 - 2.94 (1 H, m), 1.88 - 2.24 (6 H, m), 1.00 - 1.85 (19 H, m).

### Step 6: Preparation of compound 3-6

A mixture of compound **3-5** (0.087 g, 0.127 mmol) in THF (3 mL), NaOH (1.5 mL, 1.500 mmol), and MeOH (1.5 mL) was stirred at rt overnight, diluted with EtOAc and wasted with HCl (0.2N), brine, dried (MgSO4), removed the solvent to afford compound **3-6** as a yellow solid (0.079 g, 95%). LC-MS retention time: 3.108 min; MS m/z 657 (M+H)⁺.

### Step 7: Preparation of compound 3

A mixture of compound **3-6** (0.059 g, 0.090 mmol), TEA (0.06 ml, 0.430 mmol), (2R,6S)-2,6-dimethylmorpholine (0.03mL, 0.244 mmol), DCM (1 mL) and TBTU (0.060 g, 0.187 mmol) was stirred at rt for 5 h and quenched with MeOH (1 mL), removed the solvent, purified by Prep-HPLC to afford compound 3 and isolated as mono TFA salt (0.0311 g, 37%). LC-MS retention time: 3.181 min; MS m/z 754 (M+H)⁺; 1H NMR (400 MHz, *CHLOROFORM-d*) δ ppm 9.04 (1 H, br. s.), 7.87 - 8.12 (2 H, m), 7.74 (1 H, d, *J*=8.06 Hz), 7.61 (1 H, br. s.), 7.18 (1 H, d, *J*=8.56 Hz), 6.98 (2 H, br. s.), 5.01 - 5.17 (1 H, m), 3.99 (3 H, s), 3.94 - 4.78 (8 H, m), 3.05 - 3.54 (2 H, m), 2.83 - 2.97 (1 H, m), 2.34 - 2.75 (5 H, m), 1.73 - 2.24 (9 H, m), 1.16 - 1.67 (10 H, m), 0.65 - 1.07 (4 H, m).

### Preparation of compond 4: 6-[1-cyclobutyl-4-(4-morpholinylcarbonyl)-1H-imidazol-5-yl]-13-cyclohexyl-3-methoxy-N-[(1-methylethyl)sulfonyl]-7H-indolo[2,1-a][2]benzazepine-10-carboxamide

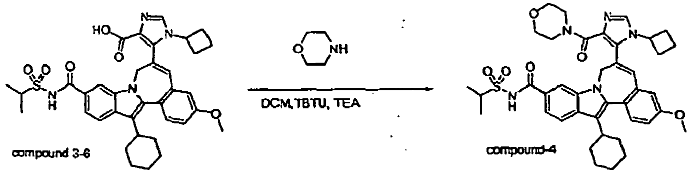

A mixture of compound **3-6** (0.020 g, 0.030 mmol), DCM (1 mL), TEA (0.05 ml, 0.359 mmol), morpholine (0.01mL, 0.115 mmol), and TBTU (0.030 g, 0.093 mmol) was stirred at rt for 5 h and quenched with MeOH (1 mL), removed the solvents and purified by Prep-HPLC to afford compound 4 and isolated as mono TFA salt (0.0046 g, 17%). LC-MS retention time: 3.100 min; MS m/z 726 (M+H)⁺; 1H NMR (400 *MH₄ CHLOROFORM-d*) δ ppm 8.87 (1 H, br. s.), 8.03 (1 H, br. s.), 7.97 (1 H, d, *J*=8.31 Hz), 7.70 (1 H, d, *J*=8.56 Hz), 7.60 (1 H, d, *J*=8.56 Hz), 7.17 (1 H, dd, *J*=8.69, 2.64 Hz), 6.93 - 7.00 (2 H, m), 5.08 (1 H, d, *J*=14.10 Hz), 4.52 - 4.66 (2 H, m), 3.99 - 4.09 (1 H, m), 3.98 (3 H, s), 2.27 - 3.46 (14 H, m), 1.73 - 2.25 (8 H, m), 1.13 - 1.68 (10 H, m).

### Preparation of compound 5: 13-cyclohexyl-3-methoxy-6-(1-(1-methylethyl)-4-(4-morpholinylcarbonyl)-1H-imidazol-5-yl]-N-[(1-methylethyl)sulfonyl]-7H-indolo[2,1-a][2]benzazepine-10-carboxamide

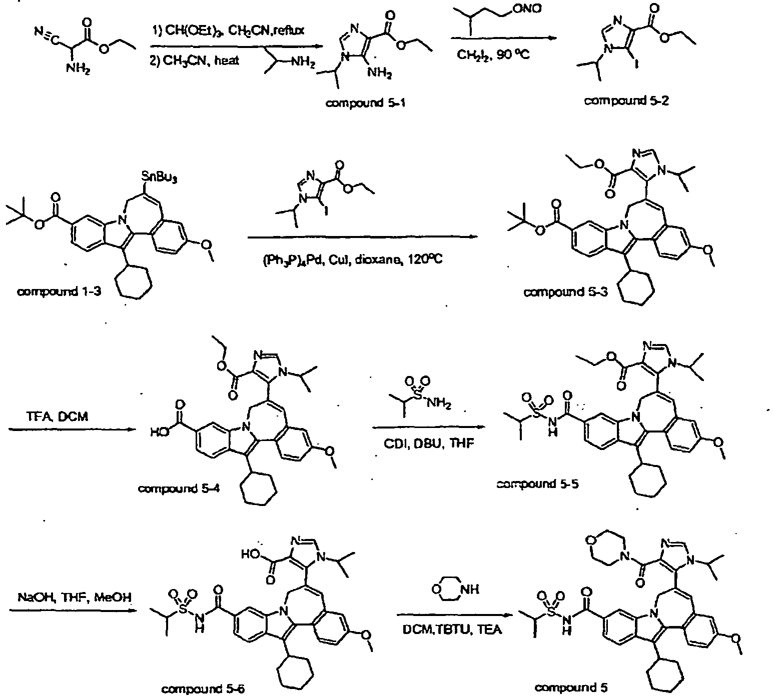

### Step 1: Preparation of compound 5-1

To a solution of ethyl 2-amino-2-cyanoacetate (3.5 g, 27.3 mmol) in Acetonitrile (50 mL) was added triethoxymethane (5.00 mL, 30.0 mmol). The clear brown solution was refluxed for 50 min and then removed the solvent in vacuum to afford a brown residue. The residue and propan-2-amine (2.56 mL, 30.0 mmol) were dissolved in Acetonitrile (50 mL), heated at 50°C in a seal tube for 30 min, and at 85 °C for 30 min. The reaction mixture was cooled and removed the solvent. The residue was dissolved in DCM and washed with 2 N NaOH, dried (MgSO4), removed the solvent The resultant residue was dissolved in DCM (-5 ml) and enough hexane was added to precipitate out the product which was filtered to afford the compound 5-1 as a brown solid (1.6 g, 30%). The crude product was directly used in next step.

### Step 2: Preparation of compound 5-2

To a solution of isopentyl nitrite (3.25 mL, 24.34 mmol) in CH₂I₂ (30 mL, 372 mmol) was added dropwise a solution of compound **5-1** (1.6 g, 8.11 mmol) in chloroform (8 mL) at 90 °C (bath temperature) over 5 min. The reaction mixture was stirred at 90 °C for 1 h, and concentrated, purified on Thomson 110g column (EtOAc/hexane:0 to 100%) and re-purified on Thomson 90g column (EtOAc/hcxane: 0 to 100%) to afford compound **5-2** as a brown solid (1.18 g, 47%). LC-MS retention time: 1.677 min; MS m/z 309 (M+H)⁺; 1H NMR (400 MHz, *CHLOROFORM-d*) δ ppm 7. 81 (1 H, s), 4.45 - 4.60 (1 H, m), 4.40 (2 H, q, *J*=7.13 Hz), 1.53 (6 H, d, *J*=6.80 Hz), 1.42 (3 H, t, *J*=7.18 Hz).

### Step 3: Preparation of compound 5-3

Compound **5-2** (0.290 g, 0.941 mmol), Tetrakis (0.095 g, 0.082 mmol), compound **1-3** (0.600 g, 0.819 mmol) and CuI (0.03g) were added in a microwave vial, capped, evacuated and filled with nitrogen. Dioxane (3.5 ml) was added and the mixture was evacuated and refilled with nitrogen and then heated in a oil bath at 120°C for 2 h. Removed the solvent, and the residue was purified on Thomson 90 g column (EtOAc/hexane: 5 to 100%) to afford compound **5-3** (0.31g, 61%). LC-MS retention time: 3.573 min; MS m/z 624 (M+H)⁺; 1H NMR (400 MHz, *CHLOROFORM-d*) δ ppm 7.75 - 7.85 (2 H, m), 7.59 - 7.70 (1 H, m), 7.41 - 7.57 (2 H, m), 7.04 (1 H, dd, *J*=8.69, 2.64 Hz), 6.93 (1 H, d, *J*=2.52 Hz), 6.67 (1 H, s), 4.98 (1 H, d, *J*=14.10 Hz), 4.67 - 4.77 (1 H, m), 4.31 - 4.46 (2 H, m), 3.89 (3 H, s), 3.45 - 3.61 (1 H, m), 2.72 - 2.90 (1 H, m), 1.05 - 2.23 (25 H, m), 0.89 - 0.93 (3 H, m).

### Step 4: Preparation of compound 5-4

A mixture of compound **5-3** (0.430 g, 0.689 mmol) in DCM (1 mL) and TFA (5 ml, 64.9 mmol) was stirred for 0.5 h. The reaction mixture was concentrated in vacuum to afford compound **5-4** (0.391 g, 100%). LC-MS retention time: 3.278 min; MS m/z 568 (M+H)⁺.

### Step 5: Preparation of compound 5-5

A mixture of compound **5-4** (0.391 g, 0.689 mmol), propane-2-sulfonamide (0.255 g, 2.066 mmol), DMAP (0.589 g, 4.82 mmol), and N-(3-Dimethylaminopropyl)-N'-ethylcarbodiimide hydrochloride (0.180 g, 0.939 mmol) in DCM (1 mL) was stirred at rt for 3 days. MeOH (1mL) was added and the mixture was stirred for 0.5 h, diluted with EtOAc, washed with satd. NH4Cl, brine, dried (Na2SO4) and purified on Thomson 25g column (MeOH/DCM: 0 to 25%) to afford the compound 5-5 as a yellow glass (0.255 g, 55%). LC-MS retention time: 3.191 min; MS m/z 673 (M+H)⁺.

### Step 6: Preparation of compound 5-6

A mixture of compound 5-5 (0.25 g, 0.372 mmol) in THF (3 mL), MeOH (1.5 mL) and NaOH (1.5 mL, 1.500 mmol) was stirred at rt overnight The reaction mixture was diluted with EtOAc and washed with 0.2 N HCl, brine, dried (MgSO4), removed the solvent to afford compound **5-6** as a yellow solid (0.172 g, 72%). LC-MS retention time: 3.070 min; MS m/z 645 (M+H)⁺.

### Step 7: Preparation of compound 5

A mixture of compound **5-6** (0.040 g, 0.062 mmol), morpholine (0.04 mL, 0.459 mmol), TBTU (0.0398 g, 0.124 mmol) in DCM (1mL) was stirred at rt for 1 h. Quenched with MeOH, removed the solvents and purified by prep HPLC to afford the compound 5 as a yellow solid and isolated as mono TFA salt (0.0135 g, 24%). LC-MS retention time: 3.060 min; MS m/z 714 (M+H)⁺; 1H NMR (400 MHz, *CHLOROFORM d*) δ ppm 8.04 (1 H, br. s.), 7.92 (1 H, d, *J*=8.56 Hz), 7.64 (1 H, d, *J*=7.05 Hz), 7.55 (1 H, d, *J*=8.56 Hz), 7.12 (1 H, dd, *J*=8.69, 264 Hz), 6.95 (1 H, d, *J*=2.52 Hz), 6.92 (1 H, br. s.), 5.08 (1 H, d, *J*=14.86 Hz), 4.58 (1 H, d, *J*=15.36 Hz), 4.26 (1 H, br. s.), 3.94 - 4.03 (1 H, m), 3.93 (3 H, s), 2.75 - 3.79 (8 H, m), 1.87 - 2.18 (4 H, m), 1.69 - 1.84 (2 H, m), 1.08 - 1.60 (18 H, m).

### Preparation of compound 6: 13-cyclohexyl-3-methoxy-6-[4-[(3-methyl-3,8-diazabicyclo[3.2.1]oct-8-yl)carbonyl]-1-(1-methylethyl)-1H-imidazol-5-yl]-N-[(1-methylethyl)sulfonyl]-7H-indolo[2,1-a][2]benzazepine-10-carboxamide

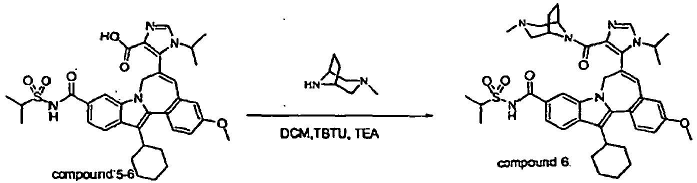

The compound **5-6** (0.046 g, 0.071 mmol) was dissolved in DCM (2 ml) and added TEA (0.1 ml, 0.717 mmol), 3-methyl-3,8-diazabicyclo[3.2.1]octane, 2 HCl (0.020 g, 0.100 mmol) and TBTU (0.035 g, 0.109 mmol). The mixture was stirred for 1.5 h, quenched with MeOH, removed the solvent and purified by prep HPLC to afford the compound 6 and isolated as bis-TFA salt (0.023 g, 32%). LC-MS retention time: 3.056 min; MS m/z 753 (M+H)⁺.

### Preparation of compound 7: 13-cyclohexyl-3-methoxy-N-[(1-methylethyl)sulfonyl]-6-[1-(1-methylethyl)-4-[(3R,5S)-3,4,5-trimethyl-1-piperazinyl]carbonyl]-1H-imidazol-5-yl]-7H-indolo[2,1-a][2]benzazepine-10-carboxamide

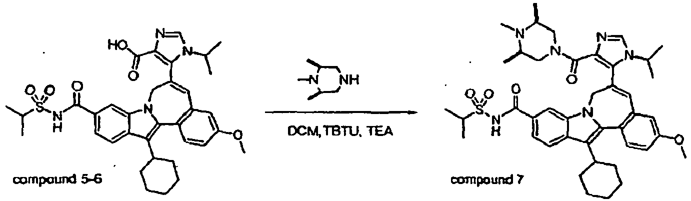

The compound 5-6 (0.046 g, 0.071 mmol) was dissolved in 2 ml of DCM and added TEA (0.1 ml, 0.717 mmol), (2R,6S)-1,2,6-trimethylpiperazine, 2 HCl (0.030 g, 0.149 mmol) and TBTU (0.035 g, 0.109 mmol). The mixture was stirred for 1.5 h, quenched with MeOH, removed the solvent and purified by prep HPLC to afford the compound **6** and isolated as bis-TFA salt (0.0264 g, 36%). LC-MS retention time: 3.035; MS m/z 755 (M+H)⁺.

### Preparation of compound 8: 13-cyclohexyl-6-[4-[[(2R,6S)-2,6-dimethyl-4-morpholinyl]carbonyl]-1-(1-methylethyl)-1H-imidazol-5-yl]-3-methoxy-N[(1-methylethyl)sulfonyl]-7H-indololo[2,1-a][2]benzazepine-10-carboxamide

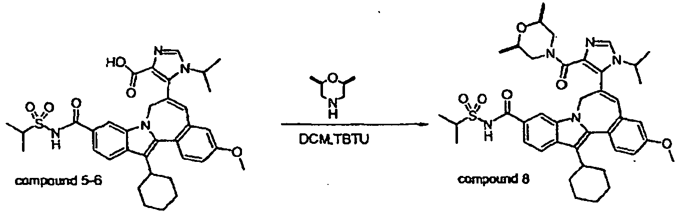

To a mixture of compound **5-6** (0.040 g, 0.062 mmol) and (2R,6S)-2,6-dimethylmorpholine (0.04 mL, 0.325 mmol) in DCM was added (2R,6S)-2,6-dimethylmorpholine (0.04 FL, 0.325 mmol) and TBTU (0.0398 g, 0.124 mmol). The reaction mixture was stirred at rt for 1.5 h. Quenched with MeOH, removed the solvent, and purified by prep HPLC to afford the compound **8** as a yellow solid and isolated as mono TFA salt (0.0105 g, 19%).

### Preparation of compound 9: 13-cyclohexyl-N-[(dimethylamino)sulfonyl]-3-methoxy-6-[1-(1-methylethyl)-5-(4-morpholinylcarbonyl)-1H-imidazol-4-yl]-7H-indolof2,1-a][2]benzazepine-ne-10-carboxamide

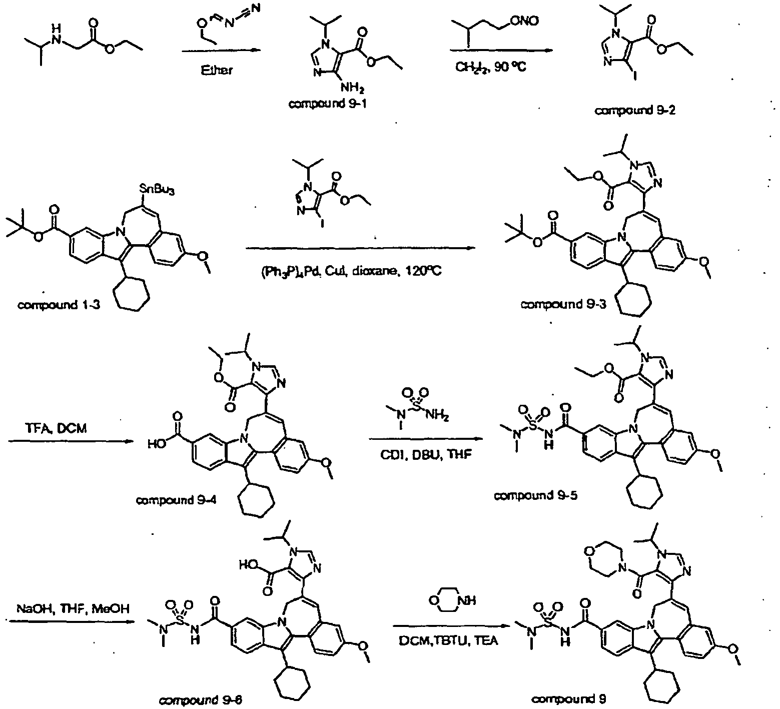

### Step 1: Preparation of compound 9-1

A solution of ethyl N-cyanoformimidate (1.095 g, 11.16 mmol) in Ether (5 ml) was . added dropwise to a solution of ethyl 2-(isopropylamino)acetate (1.62 g, 11.16 mmol) in ether (5 ml). The reaction mixture was stirred at rt for 1 h and removed the solvent. To the residue was added EtOH (10.00 mL) and potassium ethoxide (1.127 g, 13.39 mmol). The reaction mixture was stirred for 1 h, removed the solvents to afford a tan solid (1.9 a, 86%). The crude product was used in next step.

### Step 2: Preparation of compound 9-2

To a solution of isopentyl nitrite (3.86 **nL, ? 8.9** mmol) in **CH₂I₂** (20 mL, 248 mmol) was added ethyl 4-amino-1-isopropyl-1H-imidazole-5-carboxylate (1.9 g, 9.63 mmol) in chloroform (8 mL) at 90°C over 5 min. To complete the addition CHCl3 (10mL) was used to wash down the solid. After the reaction mixture was stirred for 1h, the solvent was partially removed and purified on Thomson 90g column (EtOAc/hexane: 0 to 60%) to afford the compound **9-2** as a brown solid (0.739 g, 25%). LC-MS retention time: 2.098 min; MS m/z 309 (M+H)⁺; 1H NMR (400 MHz, *CHLOROFORM-d*) δ ppm 7.65 (1 H, s), 5.22 - 5.30 (1 H, m), 4.36 (2 H, q, *J*=7.22 Hz), 1.47 (6 H, d, *J*=6.80 Hz), 1.43 (3 H, t, *J*=7.18 Hz).

### Step 3: Preparation of compound 9-3

In a microwave vial was added ethyl 4-iodo-1-isopropyl-1H-imidazole-5-carboxylate (0.389 g, 1.263 mmol), Tetrakis (0.142 g, 0.123 mmol), CuI (0.03 g) and compound **1-3** (0.9 g, 1.228 mmol). Capped the vial evacuated and filled with nitrogen. Dioxane (3.5 ml) was added and evacuated, filled with nitrogen. The reaction mixture was heated in a oil bath at 120 °C for 2 h. Cooled and removed the solvent and the residue was purified on Thomson 80g column (EtOAc/hexane: 0 to 80%) to afford compound **9-3** as a pale yellow solid (0.364 g, 47%). LC-MS retention time: 3.588 min; MS m/z 624 (M+H)⁺.

### Step 4: Preparation of compound 9-4

A mixture of compound **9-3** (0.364 g, 0.584 mmol) and TFA (2 ml, 26.0 mmol) in DCM (1 mL) was stirred at rt for 0.5 h and removed the excess TFa and solvent in vacuum to afford compound the compound **9-4** as ayellow solid (0.331g, 100%). LC-MS retention time: 3.265 min; MS m/z 568 (M+H)⁺.

### Step 5: Preparation of compound 9-5

A mixture of compound **9-4** (0.331 g, 0.583 mmol), N,N-dimethyl sulfamide (0.22 g, 1.772 mmol)), DMAP (0.499 g, 4.08 mmol), and N-(3-Dimethylaminopropyl)-N'-ethylcarbodiimide hydrochloride (0.22 g, 1.148 mmol) in DCM (4 mL) was stirred at rt for 3 days and quenched with 1 ml of MeOH, stirred for 5 min, removed the solvents. The residue was purified on Thomson 40g column (EtOAc/hexane: 5 to 100%) to afford the compound **9-5** as a DMAP salt (0.42g, 90%). LC-MS retention time: 3.221 min; MS m/z 674 (M+H)⁺.

### Step 6: Preparation of compound 9-6

A mixture of compound **9-5** (0.42 g, 0.528 mmol) in THF (5 mL), MeOH (3 mL) and sodium hydroxide (3 mL, 3.00 mmol) was stirred at rt for 16 h. The reaction mixture was partially concentrated, diluted with EtOAc, washed with pH 4 buffer, dried (MgSO4), and removed the solvent to afford the compound **9-6** as a yellow solid. LC-MS retention time: 2.981 min; MS m/z 646 (M+H)⁺.

### Step 7: Preparation of compound 9

A mixture of compound **9-6** (0.040 g, 0.062 mmol), morpholine (0.03 mL, 0.344 mmol) and TBTU (0.035 g, 0.109 mmol) in DCM (1 mL) was stirred for 1.5 h, and diluted with MeOH (1 ml), removed the solvent and purified by Prep-HPLC to afford the compound **9** as a yellow solid and isolated as mono TFA salt (0.0091 g, 18%). LC-MS retention time: 3.061 min; MS m/z 715(M+H)⁺; 1H NMR (400 MHz, *CHLOROFORM-d*) δ ppm 8.50 (1 H, br. s.), 8.25 (1 H, br. s.), 7.88 (1 H, d, *J*=8.31 Hz), 7.67 (1 H, br. s.), 7.48 - 7.56 (1 H, m), 7.09 (1 H, dd, *J*=8.56, 2.52 Hz), 7.01 (1 H, br. s.), 6.92 (1 H, br. s.), 5.51 (1 H, d, *J*=14.35 Hz), 4.59 - 4.71 (2 H, m), 4.53 (1 H, d, *J*=13.35 Hz), 3.89 (3 H, s), 3.74 - 3.83 (1 H, m), 3.42 (2 H, br. s.), 3.21 - 3.30 (1 H, m), 3.03 (6 H, s), 2.83 - 2.95 (1 H, m), 2.28 (1 H, br. s.), 1.86 - 2.20 (7 H, m), 1.73 - 1.83 (2 H, m), 1.09 - 1.72 (9 H, m).

### Preparation of compound 10: 13-cyclohexyl-N-[(dimethylamino)sulfonyl]-6-[5-[[((2R,6S)-2,6-dimethyl-4-morpholinyl]carbonyl]-1-(1-methylethyl)-1H-imidazol-4-yl]-3-methoxy-7H-indolo[2,1-a][2]benzazepine-10-carboxamide

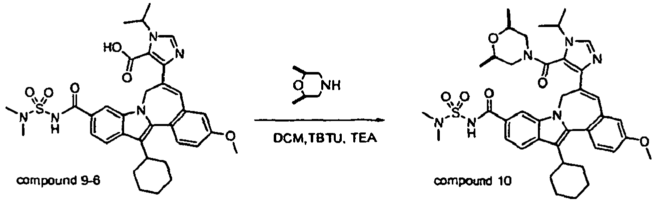

A mixture of compound **9-6** (0.04 g, 0.062 mmol), (2R,6S)-2,6-dimethylmorpholine (0.03 mL, 0.244 mmol), and TBTU (0.035 g, 0.109 mmol) in DCM (1 mL) was stirred for 1 h, quenched with MeOH, removed the solvent and purified by Prep-HPLC to afford compound 10 as a yellow solid and isolated as mono TFA salt (0.011 g. 19%). LC-MS retention time: 3.188 min; MS m/z 743 (M+H)⁺; compound **10** exists as rotamers as observed by 1H NMR; major rotamer: 1 H NMR (400 MHz, *CHLOROFORM-d)* δ ppm 8.21 - 8.44 (2 H, m), 7.82 - 7.92 (1 H, m), 7.61 - 7.74 (1 H, m), 5.47 - 5.68 (1 H, m), 4.62 - 4.83 (1 H, m), 4.24 - 4.56 (1 H, m), 3. 84 - 3.89 (3 H, m), 3.20 - 3.79 (7 H, m), 3.03 (6 H, s), 2.74 - 2.96 (2 H, m), 2.28 - 2.56 (1 H, m), 1.89 - 2.17 (4 H, m), 1.11- 1.81 (15 H, m), 0.63 - 1.05 (3 H, m), -0.04 (1 H, d, *J*=5.79 Hz).

### Prpeparation of compound 11: 13-cyclohexyl-6-[4-[[(2R,6S)-2,6-dimethyl-4-morpholinyl]carbonyl]-1-(1-methylethyl)-1H-1,2,3-triazol-5-yl]-3-methoxy-N-[(1-methylethyl)sulfonyl]-7H-indolo[2,1-a][2]benzazepine-10-carboxamide

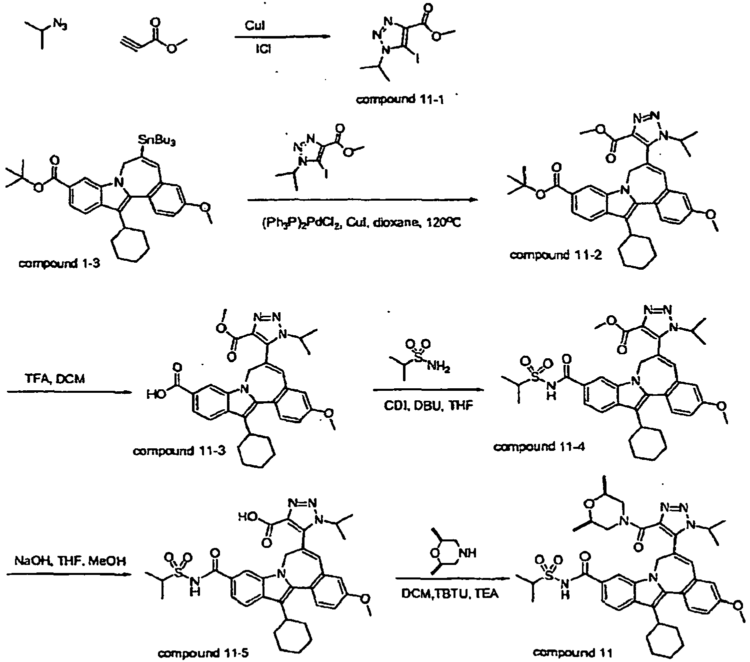

### Step 1: Preparation of compound 11-1

To a solution of 2-azidopropane (0.5 g, 5.87 mmol) in THF (20 mL) was added methyl propiolate (0.631 mL, 7.05 mmol) under N2 and followed by addition of TEA (0.983 mL, 7.05 mmol) dropwise. Iodine monochloride (1.145 g, 7.05 mmol) was added (washed with 3 mL of THF to complete the transfer) and added powdered copper (I) iodide (1.343 g, 7.05 mmol) at -30 °C bath temperature. The reaction mixture was stirred at -30 °C for 5 min, removed the bath and stirred at rt for 20 h. The reaction mixture was concentrated and directly purified on Thomson 80g column (EtOAc/hexane: 0 to 80%) to afford the compound 10-1 as a yellow solid (0.257 g, 15%). LC-MS retention time: 1.727 min; MS m/z 296 (M+H)⁺; 1H NMR (400 MHz, *CHLOROFORM-d)* δ ppm 4.71 - 4.90 (1 H, m), 3.96 (3 H, s), 1.63 (6 H, d, *J*=6.80 Hz).

### Step 2: Preparation of compound 11-2

Powdered 3A molecular sieve (0.75 g) was added into a 20 ml microwave viall, flame dried under vacuum, refilled with N2 and cooled. Compound **1-3** (0.682 g, 0.931 mmol), methyl 5-iodo-1-isopropyl-1H-1,2,3-triazole-4-carboxylate (0.229 g, 0.776 mmol), and Bis(triphenylphosphine)palladium(II) dichloride (0.055 g, 0.078 mmol) were added. Capped the vial, evacuated and filled with nitrogen. Dioxane (5 ml) was added and the mixture was evacuated and filled with nitrogen and heated in a oil bath at 120 °C for 20h. The reaction mixture was cooled down and filtered through celite, removed the solvent and the residue was purified on Thomson 25g column (EtOAc/hexane: 0 to 100%) to afford the compound 10-2 as a dark brown solid (0.269 g, 57%). LC-MS retention time: 3.743 min; MS m/z 611 (M+H)⁺;1H NMR (400 MHz, *CHLOROFORM-d*) δ ppm 7.81 (1 H, d, *J*=8.31 Hz), 7.74 (1 H, s), 7.64 (1 H, dd, *J*=8.56, 1.26 Hz), 7.51 (1 H, d, *J*=8.56 Hz), 7.08 (1 H, dd, *J*=8.56, 2.77 Hz), 6.93 (1 H, d, *J*=2.77 Hz), 4.87 - 5.00 (1 H, m), 4.79 - 4.87 (1 H, m), 4.67 - 4.78 (1 H, m), 3.96 (3 H, s), 3.90 (3 H, s), 2.76 - 2.87 (0 H, m), 1.98 - 2.17 (3 H, m), 1.86 - 1.98 (1 H, m), 1.69 - 1.84 (2 H, m), 1.63 (6 H, d, *J*=6.80 Hz), 1.56 (9 H, s), 1.13 - 1.70 (6 H, m).

### Step 3: Preparation of compound 11-3

A mixture of compound **11-2** (0.269 g, 0.440 mmol), DCM (0.5 mL), and TFA (1 ml, 12.98 mmol)) was stirred at rt for 1 h. Removed the solvents in vacuum to afford the compound 10-3 (0.244 g, 100%). LC-MS retention time: 3.575 min; MS m/z 555 (M+H)⁺.

### Step 4: Preparation of compound 11-4

A mixture of compound **11-1** (0.244 g, 0.440 mmol), propane-2-sulfonamide (0.163 g, 1.320 mmol) and DMAP (0.376 g, 3.08 mmol), N-(3-Dimethylaminopropyl)-N'-ethylcarbodiimide hydrochloride (0.180 g, 0.939 mmol) in DCM (1 mL) was stirred at rt overnight. The reaction mixture was diluted with EtOAc and washed with cold 0.1 N HCl, water, brine, dried (MgSO4), removed the solvent, and purified on Thomson 12 g column [EtOAc-MeOH (9-1)/hexane: 5 to 100%] to afford the compound **1.0-4** as a glass (0.150 g, 52%). LC-MS retention time: 3.310 min; MS m/z 660 (M+H)⁺.

### Step 5: Preparation of compound 11-5

A mixture of compound **11-4** (0.15 g, 0.227 mmol) in THF (2 mL)), NaOH (0.909 mL, 0.909 mmol), and MeOH (1mL) was stirred at rt for 20 h, diluted with EtOAc and washed with 0.2 N HCl brine, dried (MgSO4), removed the solvent to afford the compound **10-5** as a yellow solid (0.134 g, 91%). LC-MS retention time: 3.233 min; MS m/z 646 (M+H)⁺.

### Step 6: Preparation of compound 11

A mixture of compound **11-5** (0.0446 g, 0.069 mmol), (2R,6S)-2,6-dimethylmorpholine (0.008 g, 0.069 mmol),TEA (0.03 mL, 0.215 mmol), and TBTU (0.022 g, 0.069 mmol) in DCM was stirred at rt for 2 h. Quenched with MeOH, removed the solvent and purified by prep HPLC to afford the compound 11 as a yellow solid (0.0122 g, 23%). LC-MS retention time: 3.273 min; MS m/z 743 (M+H)⁺; 1H NMR (400 MHz, *CHLOROFORM-d*) δ ppm 7.89 (1 H, d, *J*=8.56 Hz), 7.41 - 7.59 (2 H, m), 7.08 (1 H, d, *J*=8.56 Hz), 6.94 (1 H, d, *J*=2.27 Hz), 6.74 (1 H, s), 4.91 - 5.13 (1 H,m), 4.53 - 4.74 (1 H, m), 3.96 - 4.35 (2 H, m), 3.90 (3 H, s), 3.34 - 3.60 (1 H, m), 2.40 - 2.99 (11 H, m), 1.89 - 2.17 (4 H, m), 1.67 - 1.84 (2 H, m), 0.66 -1.56 (18 H, m).

### Preparation of compound 12: 13-cyclohexyl-3-methoxy-N[(1-methylethyl)sulfonyl]-6-[1-(1-methylethyl)-4-[[(3R, 5S)-3,4,5-trimethyl-1-piperazinyl]carbonyl]-1H-1,2,3-triazol-5-yl]-7H-indolo[2,1-a][2]benzazepine-10-carboxamide

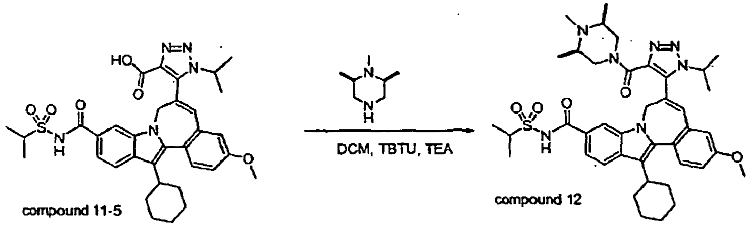

A mixture of compound **11-5** (0.0446 g, 0.069 mmol), (2R, 6S)-1,2,6-trimethylpiperazine (0.0124 g, 0.097 mmol),TEA (0.1 mL, 0.717 mmol) and TBTU (0.031 g, 0.097 mmol) in DCM was stirred at rt for 1 h. Quenched with MeOH, removed the solvent and purified by prep HPLC to afford the compound 12 as a yellow solid (0.0129 g, 21%) and isolated as mono TFA salt LC-MS retention time: 3.081 mm; MS m/z 756 (M+H)⁺; 1H NMR (400 MHz, *CHLOROFORM-d)* δ ppm 7.76 - 7.92 (2 H, m), 7.41 - 7.56 (2 H, m), 7.06 (1 H, dd, *J*=8.69, 2.39 Hz), 6.98 (1 H, br. s.), 6.75 (1 H, s), 5.33 (1 H, br. s.), 5.02 (1 H, br. s.), 4.66 (1 H, d, *J*=13.85 Hz), 3.99 (1 H, br. s.), 3.89 (3 H, s), 2.63 - 3.78 (10 H, m), 1.00 - 2.21 (28 H, m).

### Preparation of compound 13: 13-cyclohexyl-N-[(dimethylamino)sulfonyl]-3-methoxy-6-[1-(1-methylethyl)-4-(4-morpholinylcarbonyl)-1H-1,2,3-triazol-5-yl]-7H-indolo[2,1-a][2]benzazepine-10-carboxamide

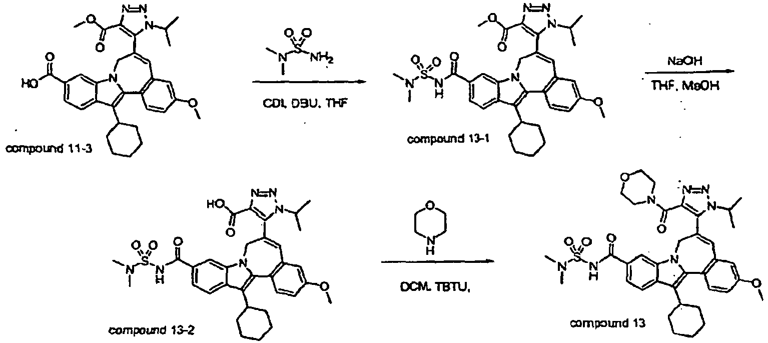

### Step 1: Preparation of compound 13-1

A mixture of compound **11-3** (0.296 g, 0.534 mmol) and CDI (0.169 g, 1.042 mmol) in THF (5ml) was stirred at 50°C for 0.5 h, cooled and added N,N-dimethylsulfamide (0.129 g, 1.039 mmol) and DBU (0.4 ml, 2.65 mmol). The mixture was stirred at ambient temperature overnight. Removed the solvent in vacuum and added DCM (5mL) and DMAP (0.250 g, 2.046 mmol), and 1-(3-Dimethylaminopropyl)-3-ethylcarbodiimide hydrochloride (0.140 g, 0.730 mmol). The reaction mixture was stirred overnight, concentrated and directly loaded on Thomson 40g column (EtOAc/Hexane: 5 to 100%). The collection was dissolved in EtOAc and washed with cold HCl (1N), brine, dried (MgSO4), removed the solvent to afford compound **13-1** as a yellow solid (0.240 g, 68%). LC-MS retention time: 3.250 min; MS m/z 661 (M+H)⁺; 1H NMR (400 MHz, *CHLOROFORM-d)* δ ppm 9.06 (1 H, br. s.), 8.21 (1 H, br. s.), 7.90 (1 H, d, *J*=8.56 Hz), 7.48 - 7.57 (1 H, m), 7.19 (1 H, br. s.), 7.09 (1 H, dd, *J*=8.69, 2.64 Hz), 6.95 (1 H, d, *J*=2.52 Hz), 6.75 (1 H, s), 5.21 (1 H, br. s.), 4.76 (1 H, br. s.), 3.93 - 4.02 (1 H, m), 3.91 (3 H, s), 3.77 (3 H, s), 2.92 (6 H, s), 2.81 - 2.89 (1 H, m), 1.09 - 2.27 (16 H, m).

### Step 2: Preparation of compound 13-2

A mixture of compound **13-1** (0.24 g, 0.363 mmol) in THF (4 mL), NaOH (2 mL, 2.000 mmol) and MeOH (2mL) was stirred at rt for 20 h. Diluted with EtOAc and washed with 0.2 N HCl, brine, dried (MgSO4), removed the solvent to afford a brown solid. LC-MS retention time: 3.220 min; MS m/z 647 (M+H)⁺, 1H NMR (400 MHz, *CHLOROFORM-d*) δ ppm 8.51 (1 H, br. s.), 7.89 (1 H, d, *J*=8.56 Hz), 7.65 (1 H, d, *J*=7.81 Hz), 7.43 (1 H, d, *J*=8.56 Hz), 6.99 (1 H, d, *J*=9.32 Hz), 6.93 (1 H, d, *J*=2.52 Hz), 6.77 (1 H, s), 5.47 (1 H, br. s.), 4.66 (1 H, d, *J*=14.86 Hz), 3.91 - 4.00 (1 H, m), 3.80 (3 H, s), 2.83 (6 H, s), 1.04 - 2.24 (16 H, m).

### Step 3: Preparation of compound 13

To a mixture of compound **13-2** (0.040 g, 0.062 mmol) and morpholine (0.02ml, 0.230 mmol) in DCM (1 mL) was added TEA (0.03 ml, 0.215 mmol) and TBTU (0.028 g, 0.087 mmol). The reaction mixture was stirred at rt for 2 h and another portion of morpholine (0.02ml), 0.230 mmol) and TBTU (0.028 g, 0.087 mmol) was added and stirred for 1 h. Quenched with MeOH, removed the solvents, and the residue was dissolved in MeOH, filtered and purified by prep-HPLC to afford a yellow solid which was re-purified by prep TLC (MeOH/DCM: 2 to 10%) to afford the compound **13** as a glass (0.0028 g, 6%). LC-MS retention time: 3.198 min; MS m/z 716 (M+H)⁺; 1H NMR (400 MHz, *CHLOROFORM*-*d*) δ ppm 7.87 (1 H, d, *J*=8.06 Hz), 7.75 (1 H, br. s.), 7.50 (1 H, d, *J*=8.81 Hz), 7.36 (1 H, d, *J*=7.81 Hz), 7.04 - 7.09 (1 H, m), 6.93 (1 H, br. s.), 6.70 (1 H, s), 5.04 (1 H, d, *J*=18.63 Hz), 4.66 - 4.78 (1 H, m), 4.27 (1 H, br. s.), 4.11 (1 H, br. s.), 3.90 - 4.01 (1 H, m), 3.89 (3 H, s), 3.50 - 3.75 (4 H, m), 3.02 (6 H, s), 2.83 (1 H, br. s.), 2.67 (2 H, br. s.), 1.86 - 2.14 (4 H, m), 0.74 - 1.82 (12 H, m).

### Preparation of compound 14: 13-cyclohexyl-6-[4-[(3-methyl-3,8-diazabicyclo[3.2.1]oct-8-yl)carbonyl]-1-(1-methylethyl)-1H-1,2,3-triazol-5-yl]-N-[(dimethylamino)sulfonyl]-3-methoxy-7H-indolo[2,1-a][2]benzazepine-10-carboxamide

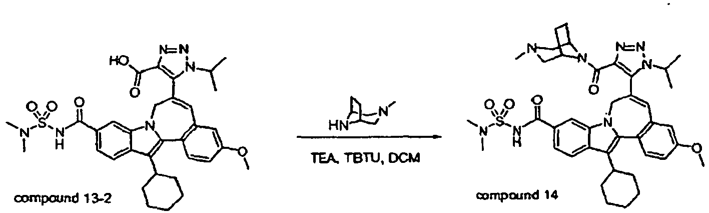

To a mixture of compound 13-2 (0.040 g, 0.062 mmol) and 3-methyl-3,8-diazabicyclo[3.2.1]octane, HCl (0.025 g, 0.154 mmol) in DCM (1 mL) was added TEA (0.10 ml, 0.717 mmol) and TBTU (0.028 g, 0.087 mmol). The reaction mixture was stirred at rt for 2 b and another portion of 3-methyl-3,8-diazabicyclo[3.2.1]octane, HCl (0.025 g, 0.154 mmol) and TBTU (0.028 g, 0.087 mmol) were added and stirred for 1h. Quenched with MeOH, removed the solvent, and purified by prep HPLC to afford the compound **14** (0.0223 g, 41.5%) as a yellow solid and isolated as mono TFA salt LC-MS retention time: 3.086 min; MS m/z 755 (M+H)⁺; 1H NMR (400 MHz, *CHLOROFORM d*) δ ppm 8.66 (1 H, br. s.), 7.87 (1 H, d, *J*=8.56 Hz), 7.79 (1 H, br. s.), 7.46 - 7.56 (1 H, m), 7.32 (1 H, d, *J*=8.31 Hz), 7.08 (1 H, dd, *J*=8.69, 2.64 Hz), 6.92 (1 H, br. s.), 6.69 (1 H, br. s.), 5.81 (1 H, br. s.), 5.10 (1 H, d, *J*=15.36 Hz), 4.82 - 5.04 (2 H, m), 4.71 (1 H, d, *J*=14.86 Hz), 3.89 (3 H, s), 3.64 - 3.84 (3 H, m), 3.57 (1 H, d, *J*=11.58 Hz), 3.01 (6 H, s), 2.70 - 3.28 (5 H, m), 1.87 - 2.59 (8 H, m), 1.71 - 1.85 (2 H, m), 1.03 - 1.58 (6 H, m), 0.39 - 0.88 (2 H, m).

### Preparation of compound 15: 13-cyclohexyl-6-[4-[[(2R,6S)-2,6-dimethyl-4-morpholinyl]carbonyl]-1-1-(1-methylethyl)-1H-1,2,3-triazol-5-yl]-N-[(dimethylamino)sulfonyl]-3-methoxy-7H-indolo[2,1-a][2]benzazepine-10-carboxamide

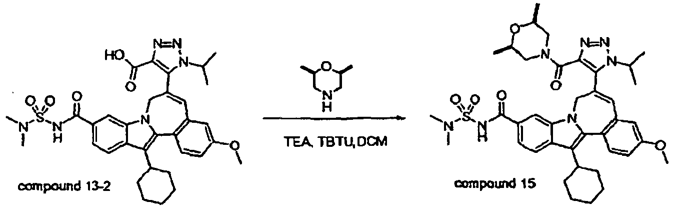

To a mixture of compound **13-2** (0.040 g, 0.062 mmol) and (2R,6S)-2,6-dimethylmorpholine (0.02 ml, 0.163 mmol) in DCM (1 mL) was added TEA (0.03 ml, 0.215 mmol) and TBTU (0.0278 g, 0.087 mmol). The reaction mixture was stirred at rt for 2 h. Quenched with MeOH, removed the solvents. The residue was dissolved in MeOH, filtered, and purified by prep HPLC to afford the compound 15 as a yellow solid (0.0193 g, 41%). LC-MS retention time: 3.253 min; MS m/z 744 (M+H)⁺; 1H NMR (400 MHz, *CHLOROFORM-d*) δ ppm 7.89 (1 H, d, *J*=8.56 Hz), 7.78 (1 H, br. s.), 7.35 - 7.56 (2 H, m), 7.09 (1 H, d, *J*=8.56 Hz), 6.93 (1 H, br. s.), 6.74 (1 H, br. s.), 4.94 - 5.09 (1 H, m), 4.67 (1 H, d, *J*=14.60 Hz), 3.94 - 4.36 (2 H, m), 3.90 (3 H, s), 3.43 - 3.64 (1 H, m), 3.02 (6 H, s), 2.51 - 3.38 (7 H, m), 1.87 - 2.19 (4 H, m), 1.78 (2 H, d, *J*=9.32 Hz), 0.70 - 1.56 (14 H, m).

### Preparation of compound 16: 13-cyclohexyl-6-[4-[(6,6-difluorohexahydro-4-methyl-1H-1,4-diazepin-1-yl)carbonyl]-1-(1-methylethyl)-1H-1,2,3-triazol-5-yl]-N [(dimethylamino)sulfonyl]-3-methoxy-7H-indolo[2,1-a][2]benzazepine-10-carboxamide

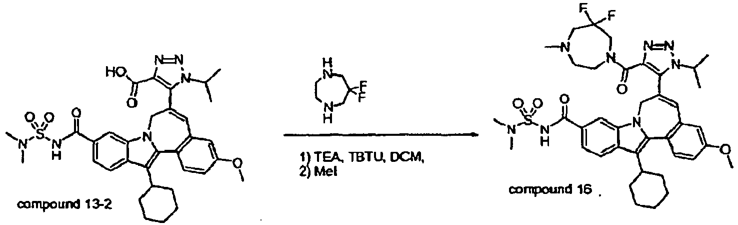

To a mixture of compound **13-2** (0.040 g, 0.062 mmol) and 6,6-difluoro-1,4-diazepane, hydrobromide (0.0268 g, 0.124 mmol) in DCM (1 mL) was added TEA (0.10 ml, 0.717 mmol) and TBTU (0.0278 g, 0.087 mmol). The reaction mixture was stirred at rt for 1 h. MeI (0.06 ml, 0.960 mmol) was injected to the reaction mixture and stirred at rt for 1.5 h. Quenched with MeOH, removed the solvents, the residue was dissolved in McOH, filtered, and purified by prep HPLC to afford a yellow solid which was re-purified by prep TLC to afford the compound **16** as a glass (0.0073 g, 15%). LC-MS retention time: 3.076 min; MS m/z 779 (M+H)⁺.

### Preparation of compound 17: 13-cyclohexyl-6-[4-[[(3R,5S)-3,4,5-trimethyl-1-piperazinyl]carbonyl]-1-(1-methylethyl)-1H-1,2,3-triazol-5-yl]-N [(dimethylamino)sulfonyl]-3-methoxy-7H-indolo[2.1-a][2]benzazepine-10-carboxamide

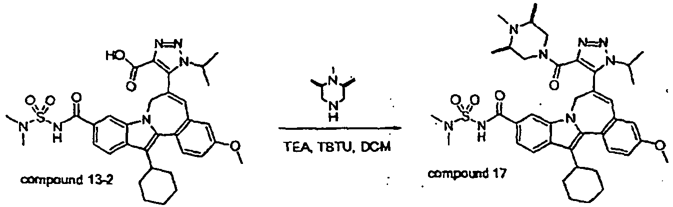

To a mixture of compound **13-2** (0.040 g, 0.062 mmol) and (2R,6S)-1,2,6-trimethylpiperazine, 2 HCl (0.025 g, 0.124 mmol) in DCM (1 mL)) was added TEA (0.10 ml, 0.717 mmol) and TBTU (0.028 g, 0.087 mmol). The reaction mixture was stirred at rt for 2 h and another portion of TEA (0.10 ml, 0.717 mmol) and TBTU (0.028 g, 0.087 mmol) were added and stirred for 1h. Quenched with MeOH, removed the solvents, the residue was dissolved in MeOH, filtered, and purified by prep-HPLC to afford the compound **17** as a yellow solid and isolated as mono TFA salt (0.0293 g, 54%). LC-MS retention time: 3.060 min; MS m/z 757 (M+H)⁺.

### Biological Methods

The compounds demonstrated activity against HCV NS5B as determined in the following HCV RdRp assays.

*HCV NS5B RdRp cloning, expression, and purification.* The cDNA encoding the NS5B protein of HCV, genotype 1b, was cloned into the pET21a expression vector. The protein was expressed with an 18 amino acid C-terminal truncation to enhance the solubility. The E. coli competent cell line BL21(DE3) was used for expression of the protein. Cultures were grown at 37 °C for ~ 4 hours until the cultures reached an optical density of 2.0 at 600 nm. The cultures were cooled to 20 °C and induced with I mM IPTG. Fresh ampicillin was added to a final concentration of 50 µg/ml and the cells were grown overnight at 20 °C.

Cell pellets (3L) were lysed for purification to yield 15-24 mgs of purified NS5B. The lysis buffer consisted of 20 mM Tris-HCl, pH 7.4, 500 mM NaCl, 0.5% triton X-100, 1 mM DTT, 1mM EDTA, 20% glycerol, 0.5 mg/ml lysozyme, 10 mM MgCl2, 15 ug/ml deoxyribonuclease I, and Complete TM protease inhibitor tablets (Roche). After addition of the lysis buffer, frozen cell pellets were resuspended using a tissue homogenizer. To reduce the viscosity of the sample, aliquots of the lysate were sonicated on ice using a microtip attached to a Branson sonicator. The sonicated lysate was centrifuged at 100,000 x g for 1hr at 4 °C and filtered through a 0.2 µm filter unit (Corning).

The protein was purified using three sequential chromatography steps: Heparin sepharose CL-6B, polyU sepharose 4B, and Hitrap SP sepharose (Pharmacia). The chromatography buffers were identical to the lysis buffer but contained no lysozyme, deoxyribonuclease I, MgCl2 or protease inhibitor and the NaCl concentration of the buffer was adjusted according to the requirements for charging the protein onto the column. Each column was eluted with a NaCl gradient which varied in length from 5-50 column volumes depending on the column type. After the final chromatography step, the resulting purity of the enzyme is >90% based on SDS-PAGE analysis. The enzyme was aliquoted and stored at -80 °C.

*Standard HCV NS5B RdRp enzyme assay.* HCV RdRp genotype 1b assays were run in a final volume of 60 µl in 96 well plates (Costar 3912). The assay buffer is composed of 20 mM Hepes, pH 7.5, 2.5 mM KCI, 2.5 mM MgCl2, 1 mM DTT, 1.6 U RNAse inhibitor (Promega N2515), 0.1 mg/ml BSA (Promega R3961), and 2 % glycerol. All compounds were serially diluted (3-fold) in DMSO and diluted further in water such that the final concentration of DMSO in the assay was 2%. HCV RdRp genotype Ib enzyme was used at a final concentration of 28 nM. A polyA template was used at.6 nM, and a biotinylated oligo-dT12 primer was used at 180 nM final concentration. Template was obtained commercially (Amersham 27-4110). Biotinylated primer was prepared by Sigma Genosys. 3H-UTP was used at 0.6 µCi (0.29 µM total UTP). Reactions were initiated by the addition of enzyme, incubated at 30 °C for 60 min, and stopped by adding 25 µl of 50 mM EDTA containing SPA beads (4 µg/µl, Amersham RPNQ 0007). Plates were read on a Packard Top Count NXT after > 1hr incubation at room temperature.

*Modified HCV NS5B RdRp enzyme assay.* A modified enzyme assay was performed essentially as described for the standard enzyme assay except for the following: The biotinylated oligo dT12 primer was precaptured on streptavidin-coated SPA beads by mixing primer and beads in assay buffer and incubating at room temperature for one hour. Unbound primer was removed after centrifugation. The primer-bound beads were resuspended in 20 mM Hepes buffer, pH 7.5 and used in the assay at final concentrations of 20 nM primer and 0.67 µg/µl beads. Order of addition in the assay: enzyme (14 nM) was added to diluted compound followed by the addition of a mixture of template (0.2 nM), 3H-UTP (0.6 µCi, 0.29 µM), and primer-bound beads, to initiate the reaction; concentrations given are final. Reactions were allowed to proceed for 4 hours at 30° C.

IC₅₀ values for compounds were determined using seven different [I]. IC₅₀ values were calculated from the inhibition using the formula y = A+((B-A)/(1+((C/x)^D))).

*FRET Assay Preparation.* To perform the HCV FRET screening assay, 96-well cell culture plates were used. The FRET peptide (Anaspec, Inc.) (Taliani et al., Anal. Biochem. 1996, 240, 60-67) contains a fluorescence donor, EDANS, near one end of the peptide and an acceptor, DABCYL, near the other end. The fluorescence of the peptide is quenched by intermolecular resonance energy transfer (RET) between the donor and the acceptor, but as the NS3 protease cleaves the peptide the products are released from RET quenching and the fluorescence of the donor becomes apparent. The assay reagent was made as follows: 5X cell Luciferase cell culture lysis reagent from Promega (#E153A) diluted to 1X with dH₂O, NaCl added to 150 mM final, the FRET peptide diluted to 20 µM final from a 2 mM stock.

To prepare plates, HCV replicon cells, with or without a Renilla luciferase reporter gene, were trypsinized and placed into each well of a 96-well plate with titrated test compounds added in columns 3 through 12; columns 1 and 2 contained a control compound (HCV protease inhibitor), and the bottom row contained cells without compound. The plates were then placed in a CO₂ incubator at 37 °C.

*Assays.* Subsequent to addition of the test compounds described above (FRET Assay Preparation), at various times the plate was removed and Alamar blue solution (Trek Diagnostics, #00-100) was added per well as a measure of cellular toxicity. After reading in a Cytoflour 4000 instrument (PE Biosystems), plates were rinsed with PBS and then used for FRET assay by the addition of 30 ul of the FRET peptide assay reagent described above (FRET Assay Preparation) per well. The plate was then placed into the Cytoflour 4000 instrument which had been set to 340 excite/490 emission, automatic mode for 20 cycles and the plate read in a kinetic mode. Typically, the signal to noise using an endpoint analysis after the reads was at least three-fold. Alternatively, after Alamar blue reading, plates were rinsed with PBS, 50 ul of DMEM (high glucose) without phenol red was added and plates were then used for luciferase assay using the Promega Dual-Glo Luciferase Assay System.

Compound analysis was determined by quantification of the relative HCV replicon inhibition and the relative cytotoxicity values. To calculate cytoxicity values, the average Alamar Blue fluorescence signals from the control wells were set as 100% non-toxic. The individual signals in each of the compound test wells were then divided by the average control signal and multiplied by 100% to determine percent cytotoxicity. To calculate the HCV replicon inhibition values, an average background value was obtained from the two wells containing the highest amount of HCV protease inhibitor at the end of the assay period. These numbers were similar to those obtained from naïve Huh-7 cells.

The background numbers were then subtracted from the average signal obtained from the control wells and this number was used as 100% activity. The individual signals in each of the compound test wells were then divided by the averaged control values after background subtraction and multiplied by 100% to determine percent activity. EC₅₀ values for a protease inhibitor titration were calculated as the concentration which caused a 50% reduction in FRET or luciferase activity. The two numbers generated for the compound plate, percent cytoxicity and percent activity were used to determine compounds of interest for further analysis.

Representative data for some compounds are reported in Table 1.

**Table 1.**

| Structure | IC₅₀ (µM) | EC₅₀ (µM) |
|---|---|---|
| 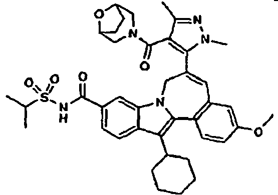 | B | B |
| 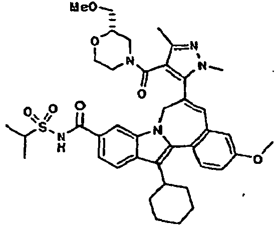 | B | B |
| 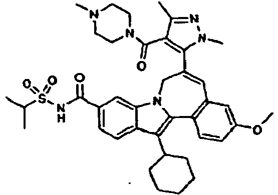 | B | B |
| 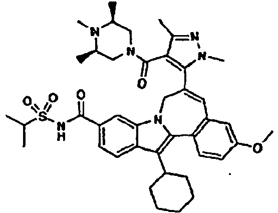 | B | B |
| 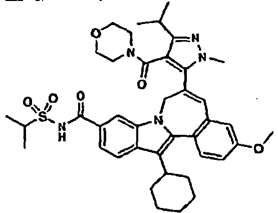 | B | B |
| 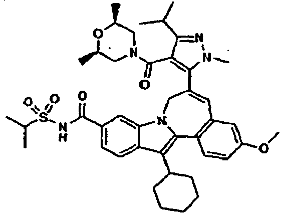 | B | B |
| 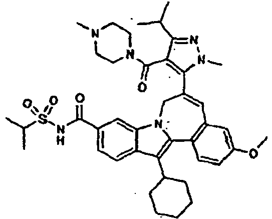 | B | B |
| 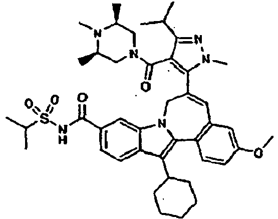 | B | B |
| 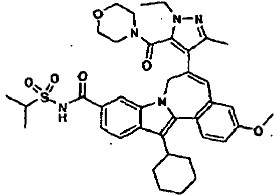 | B | B |
| 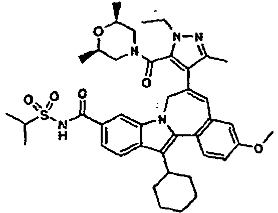 | B | B |
| 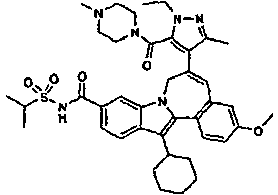 | B | B |
| 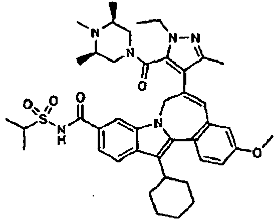 | B | B |
| 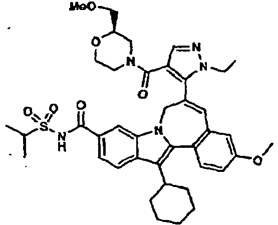 | B | B |
| 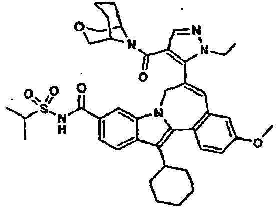 | B | B |
| 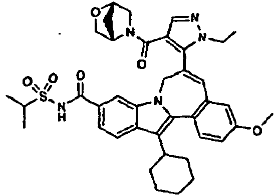 | B | B |
| 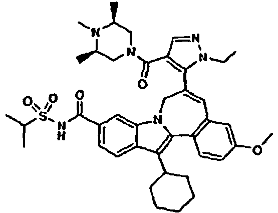 | B | B |
| 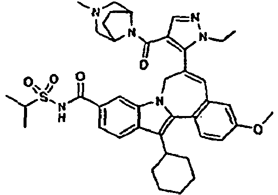 | B | B |
| 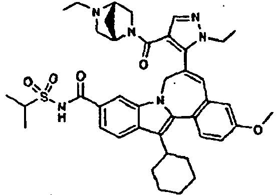 | B | B |
| 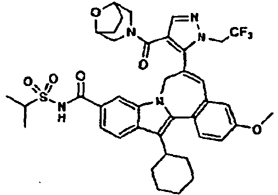 | B | B |
| 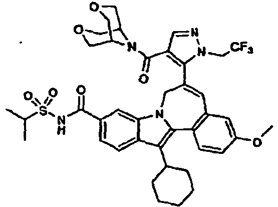 | B | B |
| 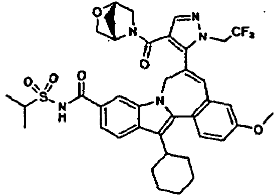 | B | B |
| 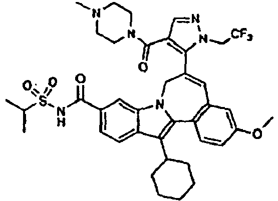 | B | B |
| 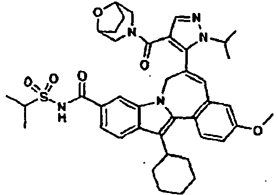 | B | B |
| 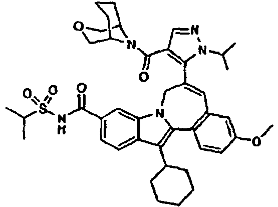 | B | B |
| 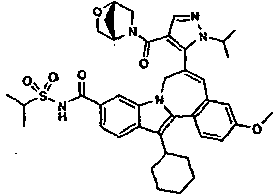 | B | B |
| 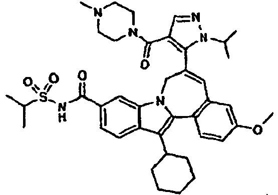 | B | B |
| 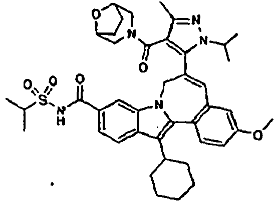 | B | B |
| 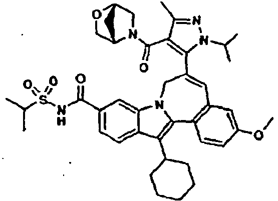 | B | B |
| 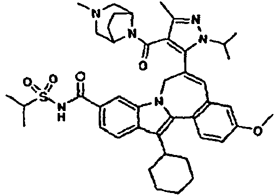 | B | B |
| 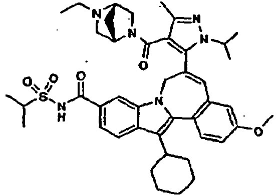 | B | B |
| 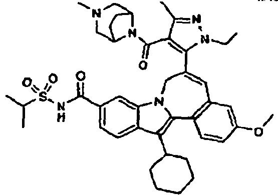 | B | B |
| 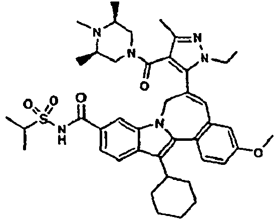 | B | B |
| 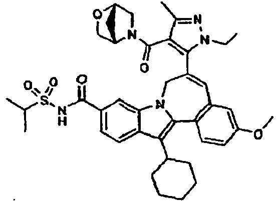 | B | B |
| 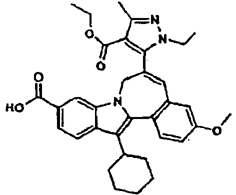 | B | A |
| 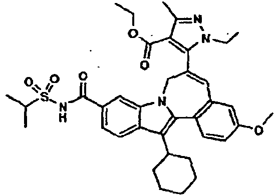 | B | A |
| 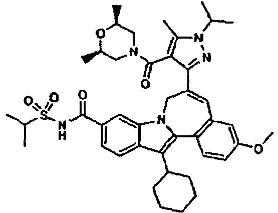 | B | B |
| 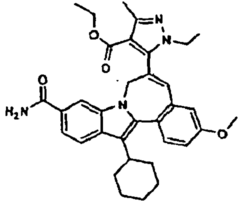 | B | A |
| 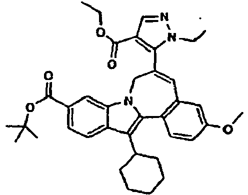 | A | A |
| 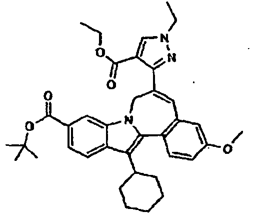 | | |
| 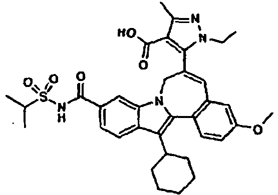 | | |
| 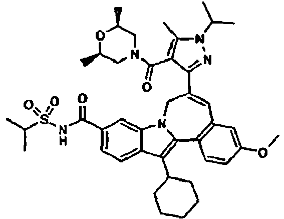 | B | B |
| 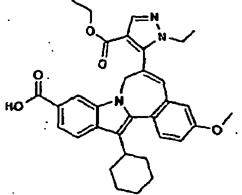 | B | A |
| 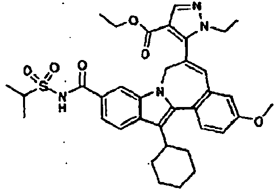 | B | A |
| 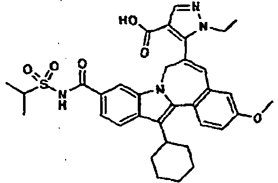 | | |
| 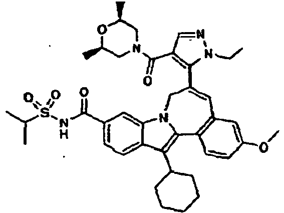 | B | B |
| 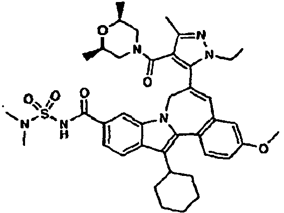 | B | B |
| 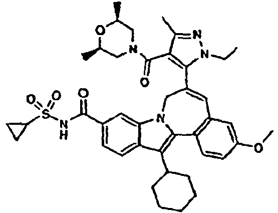 | B | B |
| 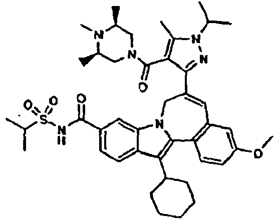 | B | B |
| 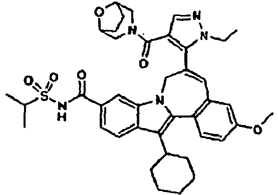 | B | B |
| 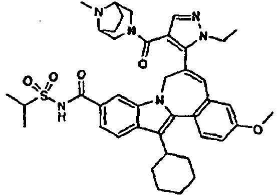 | B | B |
| 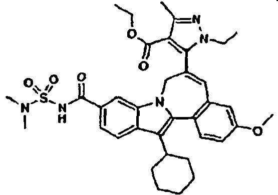 | | |
| 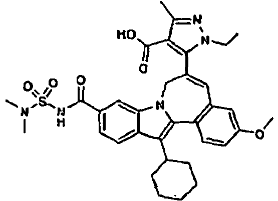 | | |
| 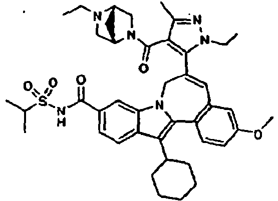 | B | B |
| 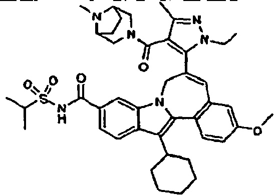 | B | B |
| 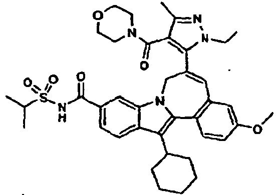 | B | B |
| 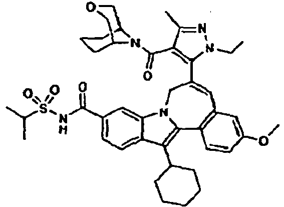 | B | B |
| 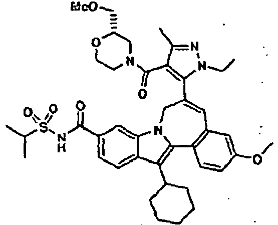 | B | B |
| 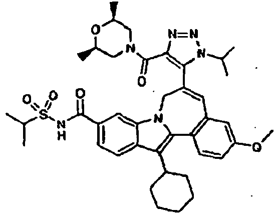 | B | B |
| 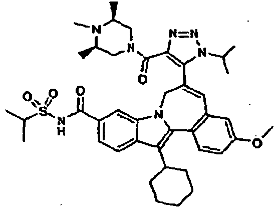 | B | B |
| 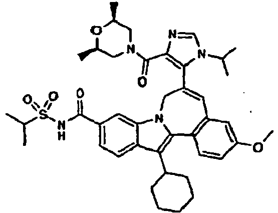 | B | B |
| 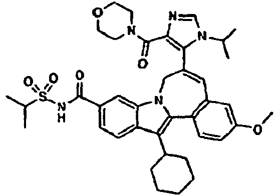 | B | B |
| 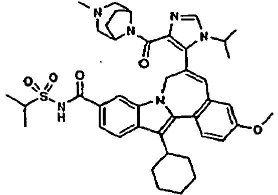 | B | B |
| 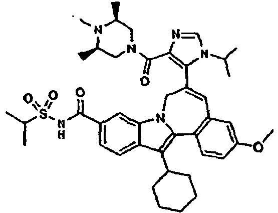 | B | B |
| 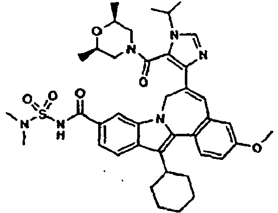 | B | B |
| 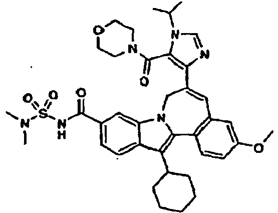 | B | B |
| 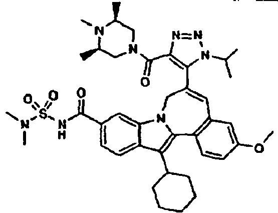 | B | B |
| 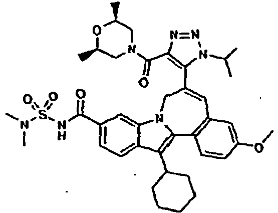 | B | B |
| 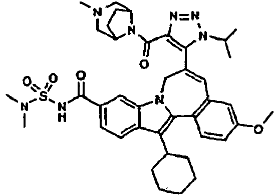 | B | B |
| 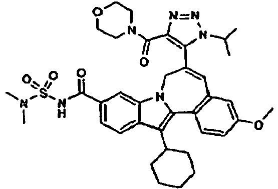 | B | B |
| 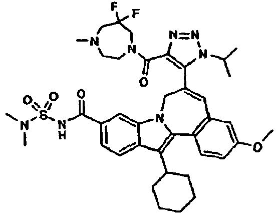 | B | B |

| | | |
|---|---|---|
| A>0.5 µM; B 0.001 µM - 0.5 µM; C <0.02 µM but an exact value was not determined; D>0.04 µM: but an exact value was not determined. | | |

### Pharmaceutical Compositions and Methods of Treatment

The compounds demonstrate activity against HCV NS5B and can be useful in treating HCV and HCV infection. Therefore, another aspect of the invention is a composition comprising a compound, or a pharmaceutically acceptable salt thereof, and a pharmaceutically acceptable carrier.

Another aspect of the invention is a composition further comprising a compound having anti-HCV activity.

Another aspect of the invention is a composition where the compound having anti-HCV activity is an interferon. Another aspect of the invention is where the interferon is selected from interferon alpha 2B, pegylated interferon alpha, consensus interferon, interferon alpha 2A, and lymphoblastoid interferon tau.

Another aspect of the invention is a composition where the compound having anti-HCV activity is a cyclosporin. Another aspect of the invention is where the cyclosporin is cyclosporin A.

Another aspect of the invention is a composition where the compound having anti-HCV activity is selected from the group consisting of interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

Another aspect of the invention is a composition where the compound having anti-HCV activity is effective to inhibit the function of a target selected from HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection.

Another aspect of the invention is a composition comprising a compound, or a pharmaceutically acceptable salt thereof, a pharmaceutically acceptable carrier, an interferon and ribavirin.

Another aspect of the invention is a compound of the invention for use in a method of inhibiting the function of the HCV replicon comprising contacting the HCV replicon with a compound or a pharmaceutically acceptable salt thereof.

Another aspect of the invention is a compound of the invention for use in a method of inhibiting the function of the HCV NS5B protein comprising contacting the HCV NS5B protein with a compound or a pharmaceutically acceptable salt thereof.

Another aspect of the invention is a compound of the invention for use in a method of treating an HCV infection in a patient comprising administering to the patient a therapeutically effective amount of a compound or a pharmaceutically acceptable salt thereof. In another embodiment the compound is effective to inhibit the function of the HCV replicon. In another embodiment the compound is effective to inhibit the function of the HCV NS5B protein.

Another aspect of the invention is a compound of the invention for use in a method of treating an HCV infection in a patient comprisiug administering to the patient a therapeutically effective amount of a compound, or a pharmaceutically acceptable salt thereof, in conjunction with (prior to, after, or concurrently) another compound having anti-HCV activity.

Another aspect of the invention is the method where the other compound having anti-HCV activity is an interferon.

Another aspect of the invention is the method where the interferon is selected from interferon alpha 2B, pegylated interferon alpha, consensus interferon, interferon alpha 2A, and lymphoblastoid interferon tau.

Another aspect of the invention is the method where the other compound having anti-HCV activity is a cyclosporin.

Another aspect of the invention is the method where the cyclosporin is cyclosporin A.

Another aspect of the invention is the method where the other compound having anti-HCV activity is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

Another aspect of the invention is the method where the other compound having anti-HCV activity is effective to inhibit the function of a target selected from the group consisting of HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection.

Another aspect of the invention is the method where the other compound having anti-HCV activity is effective to inhibit the function of target in the HCV life cycle other than the HCV NS5B protein.

"Therapeutically effective" means the amount of agent required to provide a meaningful patient benefit as understood by practitioners in the field of hepatitis and HCV infection.

"Patient" means a person infected with the HCV virus and suitable for therapy as understood by practitioners in the field of hepatitis and HCV infection.

"treatment," "therapy," "regimen," "HCV infection," and related terms are used as understood by practitioners in the field of hepatitis and HCV infection.

The compounds of this invention are generally given as pharmaceutical compositions comprised of a therapeutically effective amount of a compound or its pharmaceutically acceptable salt and a pharmaceutically acceptable carrier and may contain conventional excipients. A therapeutically effective amount is that which is needed to provide a meaningful patient benefit. Pharmaceutically acceptable carriers are those conventionally known carriers having acceptable safety profiles. Compositions encompass all common solid and liquid forms including capsules, tablets, losenges, and powders as well as liquid suspensions, syrups, elixers, and solutions. Compositions are made using common formulation techniques, and conventional excipients (such as binding and wetting agents) and vehicles (such as water and alcohols) are generally used for compositions.

Solid compositions are normally formulated in dosage units and compositions providing from about 1 to 1000 mg of the active ingredient per dose are preferred. Some examples of dosages are 1 mg, 10 mg, 100 mg, 250 mg, 500 mg, and 1000 mg. Generally, other agents will be present in a unit range similar to agents of that class used clinically. Typically, this is 0.25-1000 mg/unit.

Liquid compositions are usually in dosage unit ranges. Generally, the liquid composition will be in a unit dosage range of 1-100 mg/mL. Some examples of dosages are 1 mg/mL, 10 mg/mL, 25 mg/mL, 50 mg/mL, and 100 mg/mL. Generally, other agents will be present in a unit range similar to agents of that class used clinically. Typically, this is 1-100 mg/mL.

The invention encompasses all conventional modes of administration; oral and parenteral methods are preferred. Generally, the dosing regimen will be similar to other agents used clinically. Typically, the daily dose will be 1-100 mg/kg body weight daily. Generally, more compound is required orally and less parenterally. The specific dosing regime, however, will be determined by a physician using sound medical judgement

The invention also encompasses methods where the compound is given in combination therapy. That is, the compound can be used in conjunction with, but separately from, other agents useful in treating hepatitis and HCV infection. In these combination methods, the compound will generally be given in a daily dose of 1-100 mg/kg body weight daily in conjunction with other agents. The other agents generally will be given in the amounts used therapeutically. The specific dosing regime, however, will be determined by a physician using sound medical judgement.

Some examples of compounds suitable for compositions and methods are listed in Table 2.

**Table 2.**

| Brand Name | Type of Inhibitor or Target | Source Company |
|---|---|---|
| Omega IFN | IFN-ω | Intarcia Therapeutics |
| BILN-2061 | serine protease inhibitor | Boehringer Ingelheim Pharma KG, Ingelheim, Germany |
| Summetrel | antiviral | Endo Pharmaceuticals Holdings Inc., Cbadds Ford, PA |
| Roferon A | IFN-α2a | F. Hoffmann-La Roche LTD, Basel, Switzerland |
| Pegasys | PEGylated IFN-α2a | F. Hoffmann-La Roche LTD, Basel, Switzerland |
| Pegasys and Ribavirin | PEGylated IFN-α2a/ribavirin | F. Hoffmann-La Roche LTD, Basel, Switzerland |
| CeuCept | HCV IgG immunosuppressant | F. Hoffmann-La Roche LTD, BaseL Switzerland |
| Wellferon | lymphoblastoid IFN-αn1 | GlaxoSmithKline plc, Uxbridge, UK |
| Albuferon - α | albumin IFN-α2b | Human Genome Sciences Inc., Rockville, MD |
| Levovirin | ribavirin | ICN Pharmaceuticals, Costa Mesa, CA |
| IDN-6556 | caspase inhibitor | Idun Pharmaceuticals Inc., San Diego, CA |
| IP-501 | antifibrotic | Indevus Pharmaceuticals Inc., Lexington, MA |
| Actimmune | INF-γ | InterMune Inc., Brisbane, CA |
| Infergen A | IFN alfacon-1 | InterMune Pharmaceuticals Inc., Brisbane, CA |
| ISIS 14803 | antisense | ISIS Pharmaceuticals Inc, Carlsbad, CA/Elan Pharmaceuticals Inc., New York, NY |
| JTK-003 | RdRp inhibitor | Japan Tobacco Inc., Tokyo, Japan |
| Pegasys and Ceplene | PEGylated IFN-α2a/ immune modulator | Maxim Pharmaceuticals Inc., San Diego, CA |
| Ceplene | immune modulator | Maxim Pharmaceuticals Inc., San Diego, CA |
| Civacir | HCV IgG immunosuppressant | Nabi Biopharmaceuticals Inc., Boca Raton, FL |
| Intron A and Zadaxin | IFN-α2b/α1-thymosin | RegeneRx Biopharmiceuticals Inc., Bethesda, MD/ SciClone Pharmaceuticals Inc, San Mateo, CA |
| Levovirin | IMPDH inhibitor | Ribapharm Inc., Costa Mesa, CA |
| Viramidine | Ribavirin Prodrug | Ribapharm Inc., Costa Mesa, CA |
| Heptazyme | ribozyme | Ribozyme Pharmaceuticals, Inc., Boulder, CO |
| Intron A | IFN-α2b | Schering-Plough Corporation, Kenilworth, NJ |
| PEG-Intron | PEGylated IFN-α2b | Schering-Plough Corporation, Kenilworth, NJ |
| Rebetron | IFN-α2b/ribavirin | Schering-Plough Corporation, Kenilworth, NJ |
| Ribavirin | ribavirin | Schering-Plough Corporation, Kenilworth, NJ |
| PEG-Intron / Ribavirin | PEGylated IFN-α2b/ribavirin | Schering- Plough Corporation, Kenilworth, NJ |
| Zadazim | Immune modulator | SciClone Pharmaceuticals Inc., San Mateo, CA |
| Rebif | IFN-β1a | Serono, Geneva, Switzerland |
| IFN-β and EMZ701 | IFN-β and EMZ701 | Transition Therapeutics Inc., Ontario, Canada |
| Batabulin (T67) | β-tubulin inhibitor | Tularik Inc., South San Francisco, CA |
| Merimepodib (VX-497) | IMPDH inhibitor | Vertex Pharmaceuticals Inc., Cambridge, MA |
| Telaprevir (VX-950, LY-570310) | NS3 serine protease inhibitor | Vertex Pharmaceuticals Inc., Cambridge, MA/ Eli Lilly and Co. Inc., Indianapolis, IN |
| Omniferon | natural IFN-α | Viragen Inc., Plantation, FL |
| XTL-6865 (XTL-002) | monoclonal antibody | XTL Biopharmaceuticals Ltd., Rehovot, Isreal |
| HCV-796 | NS5B Replicase Inhibitor | Wyeth / Viropharma |
| NM-283 | NS5B Replicase Inhibitor | Idenix / Novanis |
| GL-59728 | NS5B Replicase Inhibitor | Gene Labs / Novartis |
| GL-60667 | NS5B Replicase Inhibitor | Gene Labs / Novartis |
| 2' C MeA | NS5B Replicase Inhibitor | Gilead |
| PSI6130 | NS5B Replicase Inhibitor | Roche |
| R1626 | NS5B Replicase Inhibitor | Roche |
| SCH 503034 | serine protease inhibitor | Schering Plough |
| NIM811 | Cyclophilin Inhibitor | Novartis |
| Suvus | Methylene blue | Bioenvision |
| Multiferon | Long lasting IFN | Viragen/Valentis |
| Actilon (CPG10101) | TLR9 agonist | Coley |
| Interferon-β | Interferon-β-1a | Serono |
| Zadaxin | Immunomodulator | Sciclone |
| Pyrazolopyrimidine compounds and salts From WO-2005047288 26 May 2005 | HCV Inhibitors | Arrow Therapeutics Ltd. |
| 2°C Methyl adenosine | NS5B Replicase Inhibitor | Merck |
| GS-9132 (ACH-806) | HCV Inhibitor | Achillion / Gilead |

## Claims

1. A compound selected from the group consisting of
7H-indolo[2,1-a][2]benzazepine-10-carboxamide, 13-cyclohexyl-6-[1-ethyl-3-methyl-4-[(3-methyl-3,8-diazabicyclo[3.2.1]oct-8-yl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-4-[[(3R,5S)-3,4,5-trimethyl-1-piperazinyl]carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-4-[(1S,4S)-2-oxa-5-azabicyclo[2.2.1]hept-5-ylcarbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1,3-dimethyl-4-(8-oxa-3-azabicyclo[3.2.1]oct-3-ylcarbonyl)-1H-pyrazol-5-yl]-3-methoxy-N-[(1-methylethyl)sulfonyl]-;
7H-indolo[2,1-a][2]benzazepine-10-carboxamide, 13-cyclohexyl-3-methoxy-6-[4-[[(2S)-2-(methoxymethyl)-4-morpholinyl]carbonyl]-1,3-dimethyl-1H-pyrazol-5-yl]-N-[(1-methylethyl)sulfonyl]-;
7H-indolo[2,1-a][2]benzazepine-10-carboxamide, 13-cyclohexyl-6-[1,3-dimethyl-4-[(4-methyl-1-piperazinyl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1,3-dimethyl-4-[[(3R,5S)-3,4,5-trimethyl-1-piperazinyl]carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[4-[[(2R,6S)-2,6-dimethyl-4-morpholinyl]carbonyl]-1-(1-methylethyl)-1H-1,2,3-triazol-5-yl]-3-methoxy-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[1-(1-methylethyl)-4-[[(3R,5S)-3,4,5-trimethyl-1-piperazinyl]carbonyl]-1H-1,2,3-triazol-5-yl]-;
7H-indolo[2,1-a][2]benzazepine-10-carboxylic acid, 13-cyclohexyl-6-[4-(ethoxycarbonyl)-1-ethyl-3-methyl-1H-pyrazol-5-yl]-3-methoxy-;
1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-, ethyl ester;
7H-indolo[2,1-a][2]benzazepine-10-carboxamide, 13-cyclohexyl-6-[4-[[(2R,6S)-2,6-dimethyl-4-morpholinyl]carbonyl]-5-methyl-1-(1-methylethyl)-1H-pyrazol-3-yl]-3-methoxy-N-[(1-methylethyl)sulfonyl]-;
1H-pyrazole-4-carboxylic acid, 5-[10-(aminocarbonyl)-13-cyclohexyl-3-methoxy-7H-indolo[2,1-a][2]benzazepin-6-yl]-1-ethyl-3-methyl-, ethyl ester;
7H-indolo[2,1-a][2]benzazepine-10-carboxylic acid, 13-cyclohexyl-6-[4-(ethoxycarbonyl)-1-ethyl-1H-pyrazol-5-yl]-3-methoxy-, 1,1-dimethylethyl ester;
7H-indolo[2,1-a][2]benzazepine-10-carboxylic acid, 13-cyclohexyl-6-[4-(ethoxycarbonyl)-1-ethyl-1H-pyrazol-3-yl]-3-methoxy-, 1,1-dimethylethyl ester;
1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-3-methyl-;
7H-indolo[2,1-a][2]benzazepine-10-carboxamide, 13-cyclohexyl-6-[4-[[(2R,6S)-2,6-dimethyl-4-morpholinyl]carbonyl]-1-(1-methylethyl)-1H-imidazol-5-yl]-3-methoxy-N-[(1-methylethyl)sulfonyl]-;
7H-indolo[2,1-a][2]benzazepine-10-carboxamide, 13-cyclohexyl-3-methoxy-6-[1-(1-methylethyl)-4-(4-morpholinylcarbonyl)-1H-imidazol-5-yl]-N-[(1-methylethyl)sulfonyl]-;
7H-indolo[2,1-a][2]benzazepine-10-carboxamide, 13-cyclohexyl-3-methoxy-6-[4-[(3-methyl-3,8-diazabicyclo[3.2.1]oct-8-yl)carbonyl]-1-(1-methylethyl)-1H-imidazol-5-yl]-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[1-(1-methylethyl)-4-[[(3R,5S)-3,4,5-trimethyl-1-piperazinyl]carbonyl]-1H-imidazol-5-yl]-;
7H-indolo[2,1-a][2]benzazepine-10-carboxamide, 13-cyclohexyl-3-methoxy-N-[(1-methylethyl)sulfonyl]-6-[5-methyl-4-[(3-methyl-3,8-diazabicyclo[3.2.1]oct-8-yl)carbonyl]-1-(1-methylethyl)-1H-pyrazol-3-yl]-;
7H-indolo[2,1-a][2]benzazepine-10-carboxylic acid, 13-cyclohexyl-6-[4-(ethoxycarbonyl)-1-ethyl-1H-pyrazol-5-yl]-3-methoxy-;
1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-, ethyl ester;
7H-indolo[2,1-a][2]benzazepine-10-carboxamide, 13-cyclohexyl-N-[(dimethylamino)sulfonyl]-6-[5-[[(2R,6S)-2,6-dimethyl-4-morpholinyl]carbonyl]-1-(1-methylethyl)-1H-imidazol-4-yl]-3-methoxy-;
7H-indolo[2,1-a][2]benzazepine-10-carboxamide, 13-cyclohexyl-N-[(dimethylamino)sulfonyl]-3-methoxy-6-[1-(1-methylethyl)-5-(4-morpholinylcarbonyl)-1H-imidazol-4-yl]-;
1H-pyrazole-4-carboxylic acid, 5-[13-cyclohexyl-3-methoxy-10-[[[(1-methylethyl)sulfonyl]amino]carbonyl]-7H-indolo[2,1-a][2]benzazepin-6-yl]-1-ethyl-;
7H-indolo[2,1-a][2]benzazepine-10-carboxamide, 13-cyclohexyl-N-[(dimethylamino)sulfonyl]-3-methoxy-6-[1-(1-methylethyl)-4-[[(3R,5S)-3,4,5-trimethyl-1-piperazinyl]carbonyl]-1H-1,2,3-triazol-5-yl]-;
7H-indolo[2,1-a][2]benzazepine-10-carboxamide, 13-cyclohexyl-N-[(dimethylamino)sulfonyl]-6-[4-[[(2R,6S)-2,6-dimethyl-4-morpholinyl]carbonyl]-1-(1-methylethyl)-1H-1,2,3-triazol-5-yl]-3-methoxy-;
7H-indolo[2,1-a][2]benzazepine-10-carboxamide, 13-cyclohexyl-N-[(dimethylamino)sulfonyl]-3-methoxy-6-[4-[(3-methyl-3,8-diazabicyclo[3.2.1]oct-8-yl)carbonyl]-1-(1-methylethyl)-1H-1,2,3-triazol-5-yl]-;
7H-indolo[2,1-a][2]benzazepine-10-carboxamide, 13-cyclohexyl-6-[4-[[(2R,6S)-2,6-dimethyl-4-morpholinyl]carbonyl]-1-ethyl-1H-pyrazol-5-yl]-3-methoxy-N-[(1-methylethyl)sulfonyl]-;
7H-indolo[2,1-a][2]benzazepine-10-carboxamide, 13-cyclohexyl-N-[(dimethylamino)sulfonyl]-6-[4-[[(2R,6S)-2,6-dimethyl-4-morpholinyl]carbonyl]-1-ethyl-3-methyl-1H-pyrazol-5-yl]-3-methoxy-;
7H-indolo[2,1-a][2]benzazepine-10-carboxamide, 13-cyclohexyl-N-[(dimethylamino)sulfonyl]-3-methoxy-6-[1-(1-methylethyl)-4-(4-morpholinylcarbonyl)-1H-1,2,3-triazol-5-yl]-;
7H-indolo[2,1-a][2]benzazepine-10-carboxamide, 13-cyclohexyl-6-[4-[(6,6-difluorohexahydro-4-methyl-1H-1,4-diazepin-1-yl)carbonyl]-1-(1-methylethyl)-1H-1,2,3-triazol-5-yl]-N-[(dimethylamino)sulfonyl]-3-methoxy-;
7H-indolo[2,1-a][2]benzazepine-10-carboxamide, 13-cyclohexyl-6-[1-ethyl-4-[[(2R)-2-(methoxymethyl)-4-morpholinyl]carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-(3-oxa-9-azabicyclo[3.3.1]non-9-ylcarbonyl)-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-(2-oxa-5-azabicyclo[2.2.1]hept-5-ylcarbonyl)-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-[[(3R,5S)-3,4,5-trimethyl-1-piperazinyl]carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-[(3-methyl-3,8-diazabicyclo[3.2.1]oct-8-yl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-[(5-ethyl-2,5-diazabicyclo[2.2.1]hept-2-yl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[1-methyl-3-(1-methylethyl)-4-(4-morpholinylcarbonyl)-1H-pyrazol-5-yl]-;
7H-indolo[2,1-a][2]benzazee-10-carboxamide, 13-cyclohexyl-6-[4-[[(2R,6S)-2,6-dimethyl-4-morpholinyl]carbonyl]-1-methyl-3-(1-methylethyl)-1H-pyrazol-5-yl]-3-methoxy-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[1-methyl-3-(1-methylethyl)-4-[(4-methyl-1-piperazinyl)carbonyl]-1H-pyrazol-5-yl]-;
7H-indolo[2,1-a][2]benzazepine-10-carboxamide, 13-cyclohexyl-3-methoxy-N-[(1-methylethyl)sulfonyl]-6-[1-methyl-3-(1-methylethyl)-4-[[(3R,5S)-3,4,5-trimethyl-1-piperazinyl]carbonyl]-1H-pyrazol-5-yl]-;
7H-indolo[2,1-a][2]benzazepine-10-carboxamide, 13-cyclohexyl-3-methoxy-N-[(1-methylethyl)sulfonyl]-6-[4-(8-oxa-3-azabicyclo[3.2.1]oct-3-ylcarbonyl)-1-(2,2,2-trifluoroethyl)-1H-pyrazol-5-yl]-;
7H-indolo[2,1-a][2]benzazepine-10-carboxamide, 13-cyclohexyl-6-[4-(3,7-dioxa-9-azabicyclo[3.3.1]non-9-ylcarbonyl)-1-(2,2,2-trifluoroethyl)-1H-pyrazol-5-yl]-3-methoxy-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[4-(2-oxa-5-azabicyclo[2.2.1]hept-5-ylcarbonyl)-1-(2,2,2-trifluoroethyl)-1H-pyrazol-5-yl]-;
7H-indolo[2,1-a][2]benzazepine-10-carboxamide, 13-cyclohexyl-3-methoxy-N-[(1-methylethyl)sulfonyl]-6-[4-[(4-methyl-1-piperazinyl)carbonyl]-1-(2,2,2-trifluoroethyl)-1H-pyrazol-5-yl]-;
7H-indolo[2,1-a][2]benzazepine-10-carboxamide, 13-cyclohexyl-3-methoxy-6-[1-(1-methylethyl)-4-(8-oxa-3-azabicyclo[3.2.1]oct-3-ylcarbonyl)-1H-pyrazol-5-yl]-N-[(1-methylethyl)sulfonyl]-;
7H-indolo[2,1-a][2]benzazepine-10-carboxamide, 13-cyclohexyl-3-methoxy-6-[1-(1-methylethyl)-4-(3-oxa-9-azabicyclo[3.3.1]non-9-ylcarbonyl)-1H-pyrazol-5-yl]-N-[(1-methylethyl)sulfonyl]-;
7H-indolo[2,1-a][2]benzazepine-10-carboxamide, 13-cyclohexyl-3-methoxy-6-[1-(1-methylethyl)-4-(2-oxa-5-azabicyclo[2.2.1]hept-5-ylcarbonyl)-1H-pyrazol-5-yl]-N-[(1-methylethyl)sulfonyl]-;
7H-indolo[2,1-a][2]benzazepine-10-carboxamide, 13-cyclohexyl-3-methoxy-6-[1-(1-methylethyl)-4-[(4-methyl-1-piperazinyl)carbonyl]-1H-pyrazol-5-yl]-N-[(1-methylethyl)sulfonyl]-;
7H-indolo[2,1-a][2]benzazepine-10-carboxamide, 13-cyclohexyl-3-methoxy-N-[(1-methylethyl)sulfonyl]-6-[3-methyl-1-(1-methylethyl)-4-(8-oxa-3-azabicyclo[3.2.1]oct-3-ylcarbonyl)-1H-pyrazol-5-yl]-;
7H-indolo[2,1-a][2]benzazepine-10-carboxamide, 13-cyclohexyl-3-methoxy-N-[(1-methylethyl)sulfonyl]-6-[3-methyl-1-(1-methylethyl)-4-(2-oxa-5-azabicyclo[2.2.11]hept-5-ylcarbonyl)-1H-pyrazol-5-yl]-;
7H-indolo[2,1-a][2]benzazepine-10-carboxamide, 13-cyclohexyl-3-methoxy-N-[(1-methylethyl)sulfonyl]-6-[3-methyl-4-[(3-methyl-3,8-diazabicyclo[3.2.1]oct-8-yl)carbonyl]-1-(1-methylethyl)-1H-pyrazol-5-yl]-;
7H-indolo[2,1-a][2]benzazepine-10-carboxamide, 13-cyclohexyl-6-[4-[(5-ethyl-2,5-diazabicyclo[2.2.1]hept-2-yl)carbonyl]-3-methyl-1-(1-methylethyl)-1H-pyrazol-5-yl]-3-methoxy-N-[(1-methylethyl)sulfonyl]-;
7H-indolo[2,1-a][2]benzazepine-10-carboxamide, 13-cyclohexyl-N-(cyclopropylsulfonyl)-6-[4-[[(2R,6S)-2,6-dimethyl-4-morpholinyl]carbonyl]-1-ethyl-3-methyl-1H-pyrazol-5-yl]-3-methoxy-;
7H-indolo[2,1-a][2]benzazepine-10-carboxamide, 13-cyclohexyl-3-methoxy-N-[(1-methylethyl)sulfonyl]-6-[5-methyl-1-(1-methylethyl)-4-[[(3R,5S)-3,4,5-trimethyl-1-piperazinyl]carbonyl]-1H-pyrazol-3-yl]-;
7H-indolo[2,1-a][2]benzazepine-10-carboxamide, 13-cyclohexyl-6-[1-ethyl-4-(8-oxa-3-azabicyclo[3.2.1]oct-3-ylcarbonyl)-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-4-[(8-methyl-3,8-diazabicyclo[3.2.1]oct-3-yl)carbonyl]-1H-pyrazol-5-yl]-3-methoxy-N-[(1-methylethyl)sulfonyl]-;
1H-pyrazole-4-carboxylic acid, 5-[13-cyclohexyl-10-[[[(dimethylamino)sulfonyl]amino]carbonyl]-3-methoxy-7H-indolo[2,1-a][2]benzazepin-6-yl]-1-ethyl-3-methyl-, ethyl ester;
7H-indolo[2,1-a][2]benzazepine-10-carboxamide, 13-cyclohexyl-6-[1-ethyl-3-methyl-5-(4-morpholinylcarbonyl)-1H-pyrazol-4-yl]-3-methoxy-N-[(1-methylethyl)sulfonyl]-;
7H-indolo[2,1-a][2]benzazepine-10-carboxamide, 13-cyclohexyl-6-[5-[[(2R,6S)-2,6-dimethyl-4-morpholinyl]carbonyl]-1-ethyl-3-methyl-1H-pyrazol-4-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-5-[(4-methyl-1-piperazinyl)carbonyl]-1H-pyrazol-4-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-5-[[(3R,5S)-3,4,5-trimethyl-1-piperazinyl]carbonyl]-1H-pyrazol-4-yl]-3-methoxy-N-[(1-methylethyl)sulfonyl]-;
1H-pyrazole-4-carboxylic acid, 5-[13-cyclohexyl-10-[[[(dimethylamino)sulfonyl]amino]carbonyl]-3-methoxy-7H-indolo[2,1-a] [2]benzazepin-6-yl]-1-ethyl-3 -methyl-;
7H-indolo[2,1-a][2]benzazepine-10-carboxamide, 13-cyclohexyl-6-[1-ethyl-4-[(5-ethyl-2,5-diazabicyclo[2.2.1]hept-2-yl)carbonyl]-3-methyl-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-4-[(8-methyl-3,8-diazabicyclo[3.2.1]oct-3-yl)carbonyl]-1H-pyrazol-5-yl]-3-methoxy-N-[(1-methylethyl)sulfonyl]-;
7H-indolo[2,1-a][2]benzazepine-10-carboxamide, 13-cyclohexyl-6-[1-ethyl-3-methyl-4-(4-morpholinylcarbonyl)-1H-pyrazol-5-yl]-3-methoxy-N-[(1-methylethyl)sulfonyl]-;
7H-indolo[2,1-a][2]benzazepine-10-carboxamide; 13-cyclohexyl-6-[1-ethyl-3-methyl-4-(3-oxa-9-azabicyclo[3.3.1]non-9-ylcarbonyl)-1H-pyrazol-5-yl]-3-methoxy-N-[(1-methylethyl)sulfonyl]-; and
7H-indolo[2,1-a][2]benzazepine-10-carboxamide, 13-cyclohexyl-6-[1-ethyl-4-[[(2S)-2-(methoxymethyl)-4-morpholinyl]carbonyl]-3-methyl-1H-pyrazol-5-yl]-3-methoxy-N-[(1-methylethyl)sulfonyl]-;
or a pharmaceutically acceptable salt thereof.

2. A composition comprising a compound of claim 1, or a pharmaceutically acceptable salt thereof, and a pharmaceutically acceptable carrier.

3. The composition of claim 2 further comprising at least one additional compound having therapeutic benefits for HCV wherein the compound is selected from the group consisting of interferons, cyclosporins, interleukins, HCV metalloprotease inhibitors, HCV serine protease inhibitors, HCV polymerase inhibitors, HCV helicase inhibitors, HCV NS4B protein inhibitors, HCV entry inhibitors, HCV assembly inhibitors, HCV egress inhibitors, HCV NS5A protein inhibitors, HCV NS5B protein inhibitors, and HCV replicon inhibitors.

4. A compound as defined in claim 1 for use in a method of treating hepatitis C infection.

5. The compound for use according to claim 4 further comprising administering at least one additional compound having therapeutic benefits for HCV wherein the compound is selected from the group consisting of interferons, cyclosporins, interleukins, HCV metalloprotease inhibitors, HCV serine protease inhibitors, HCV polymerase inhibitors, HCV helicase inhibitors, HCV NS4B protein inhibitors, HCV entry inhibitors, HCV assembly inhibitors, HCV egress inhibitors, HCV NS5A protein inhibitors, HCV NS5B protein inhibitors, and HCV replicon inhibitors.

## Patentansprüche

1. Verbindung, ausgewählt aus
7H-Indolo[2,1-a] [2]benzazepin-10-carboxamid, 13-Cyclohexyl-6-[1-ethyl-3-methyl-4-[(3-methyl-3,8-diazabicyclo[3.2.1]oct-8-yl)carbonyl]-1H-pyrazol-5-yl]-3-methoxy-N-[(1-methylethyl)sulfonyl]-;
7H-Indolo[2,1-a] [2]benzazepin-10-carboxamid, 13-Cyclohexyl-6-[1-ethyl-3-methyl-4-[[(3R,5S)-3,4,5-trimethyl-1-piperazinyl]carbonyl]-1H-pyrazol-5-yl]-3-methoxy-N-[(1-methylethyl)sulfonyl]-;
7H-Indolo[2,1-a][2]benzazepin-10-carboxamid, 13-Cyclohexyl-6-[1-ethyl-3-methyl-4-[(1S,4S)-2-oxa-5-azabicyclo[2.2.1]hept-5-ylcarbonyl]-1H-pyrazol-5-yl]-3-methoxy-N-[(1-methylethyl)sulfonyl]-;
7H-Indolo[2,1-a][2]benzazepin-10-carboxamid, 13-Cyclohexyl-6-[1,3-dimethyl-4-(8-oxa-3-azabicyclo[3.2.1]oct-3-ylcarbonyl)-1H-pyrazol-5-yl]-3-methoxy-N-[(1-methylethyl)sulfonyl]-;
7H-Indolo[2,1-a][2]benzazepin-10-carboxamid, 13-Cyclohexyl-3-methoxy-6-[4-[[(2S)-2-(methoxymethyl)-4-morpholinyl]carbonyl]-1,3-dimethyl-1H-pyrazol-5-yl]-N-[(1-methylethyl)sulfonyl]-;
7H-Indolo[2,1-a][2]benzazepin-10-carboxamid, 13-Cyclohexyl-6-[1,3-dimethyl-4-[(4-methyl-1-piperazinyl)carbonyl]-1H-pyrazol-5-yl]-3-methoxy-N-[1-methylethyl)sulfonyl]-;
7H-Indolo[2,1-a][2]benzazepin-10-carboxamid, 13-Cyclohexyl-6-[1,3-dimethyl-4-[[(3R,5S)-3,4,5-trimethyl-1-piperazinyl]carbonyl]-1H-pyrazol-5-yl]-3-methoxy-N-[(1-methylethyl)sulfonyl]-;
7H-Indolo[2,1-a][2]benzazepin-10-carboxamid, 13-Cyclohexyl-6-[4-[[(2R,6S)-2,6-dimethyl-4-morpholinyl]carbonyl]-1-(1-methylethyl)-1H-1,2,3-triazol-5-yl]-3-methoxy-N-[(1-methylethyl)sulfonyl]-;
7H-Indolo[2,1-a][2]benzazepin-10-carboxamid, 13-Cyclohexyl-3-methoxy-N-[(1-methylethyl)sulfonyl]-6-[1-(1-methylethyl)-4-[[(3R,5S)-3,4,5-trimethyl-1-piperazinyl]carbonyl]-1H-1,2,3-triazol-5-yl] -;
7H-Indolo[2,1-a] [2]benzazepin-10-carbonsäure, 13-Cyclohexyl-6-[4-(ethoxycarbonyl)-1-ethyl-3-methyl-1H-pyrazol-5-yl]-3-methoxy-;
1H-Pyrazol-4-carbonsäure, 5-[13-Cyclohexyl-3-methoxy-10-[[[(1-methylethyl)sulfonyl]amino]carbonyl]-7H-indolo[2,1-a] [2]benzazepin-6-yl]-1-ethyl-3-methyl-, Ethylester;
7H-Indolo[2,1-a][2]benzazepin-10-carboxamid, 13-Cyclohexyl-6-[4-[[(2R,6S)-2,6-dimethyl-4-morpholinyl]carbonyl]-5-methyl-1-(1-methylethyl)-1H-pyrazol-3-yl]-3-methoxy-N-[(1-methylethyl)sulfonyl]-;
1H-Pyrazol-4-carbonsäure, 5-[10-(Aminocarbonyl)-13-cyclohexyl-3-methoxy-7H-indolo[2,1-a][2]benzazepin-6-yl]-1-ethyl-3-methyl-, Ethylester;
7H-Indolo[2,1-a][2]benzazepin-10-carbonsäure, 13-Cyclohexyl-6-[4-(ethoxycarbonyl)-1-ethyl-1H-pyrazol-5-yl]-3-methoxy-, 1,1-Dimethylethylester;
7H-Indolo[2,1-a][2]benzazepin-10-carbonsäure, 13-Cyclohexyl-6-[4-(ethoxycarbonyl)-1-ethyl-1H-pyrazol-3-yl]-3-methoxy-, 1,1-Dimethylethylester;
1H-Pyrazol-4-carbonsäure, 5-[13-Cyclohexyl-3-methoxy-10-[[[(1-methylethyl)sulfonyl]amino]carbonyl]-7H-indolo[2,1-a][2]benzazepin-6-yl]-1-ethyl-3-methyl-;
7H-Indolo[2,1-a][2]benzazepin-10-carboxamid, 13-Cyclohexyl-6-[4-[[(2R,6S)-2,6-dimethyl-4-marpholinyl]carbonyl]-1-(1-methylethyl)-1H-imidazol-5-yl]-3-methoxy-N-[(1-methylethyl)sulfonyl]-;
7H-Indolo[2,1-a][2]benzazepin-10-carboxamid, 13-Cyclohexyl-3-methoxy-6-[1-(1-methylethyl)-4-(4-morpholinylcarbonyl)-1H-imidazol-5-yl]-N-[(1-methylethyl)sulfonyl] -;
7H-Indolo[2,1-a][2]benzazepin-10-carboxamid, 13-Cyclohexyl-3-methoxy-6-[4-[(3-methyl-3,8-diazabicyclo[3.2.1]oct-8-yl)carbonyl]-1-(1-methylethyl)-1H-imidazol-5-yl]-N-[(1-methylethyl)sulfonyl]-;
7H-Indolo[2,1-a][2]benzazepin-10-carboxamid, 13-Cyclohexyl-3-methoxy-N-[(1-methylethyl)sulfonyl]-6-[-(1-methylethyl)-4-[[(3R,5S)-3,4,5-trimethyl-1-piperazinyl]carbonyl]-1H-imidazol-5-yl]-;
7H-Indolo[2,1-a][2]benzazepin-10-carboxamid, 13-Cyclohexyl-3-methoxy-N-[(1-methylethyl)sulfonyl]-6-[5-methyl-4-[(3-methyl-3,8-diazabicyclo[3.2.1]oct-8-yl)carbonyl]-1-(1-methylethyl)-1H-pyrazol-3-yl]-;
7H-Indolo[2,1-a] [2]benzazepin-10-carbonsäure, 13-Cyclohexyl-6-[4-(ethoxycarbonyl)-1-ethyl-1H-pyrazol-5-yl]-3-methoxy-;
1H-Pyrazol-4-carbonsäure, 5-[13-Cyclohexyl-3-methoxy-10-[[[(1-methylethyl)sulfonyl]amino]carbonyl]-7H-indolo[2,1-a][2]benzazepin-6-yl]-1-ethyl-, Ethylester;
7H-Indolo[2,1-a][2]benzazepin-10-carboxamid, 13-Cyclohexyl-N-[(dimethylamino)sulfonyl]-6-[5-[[(2R,6S)-2,6-dimethyl-4-morpholinyl]carbonyl]-1-(1-methylethyl)-1H-imidazol-4-yl]-3-methoxy-;
7H-Indolo[2,1-a][2]benzazepin-10-carboxamid, 13-Cyclohexyl-N-[(dimethylamino)sulfonyl]-3-methoxy-6-[1-(1-methylethyl)-5-(4-morpholinylcarbonyl)-1H-imidazol-4-yl]-;
1H-Pyrazol-4-carbonsäure, 5-[13-Cyclohexyl-3-methoxy-10-[[[(1-methylethyl)sulfonyl]amino]carbonyl]-7H-indolo[2,1-a][2]benzazepin-6-yl]-1-ethyl-;
7H-Indolo[2,1-a][2]benzazepin-10-carboxamid, 13-Cyclohexyl-N-[(dimethylamino)sulfonyl]-3-methoxy-6-[1-(1-methylethyl)-4-[[(3R,5S)-3,4,5-trimethyl-1-piperazinyl]carbonyl]-1H-1,2,3-triazol-5-yl]-;
7H-Indolo[2,1-a][2]benzazepin-10-carboxamid, 13-Cyclohexyl-N-[(dimethylamino)sulfonyl]-6-[4-[[(2R,6S)-2,6-dimethyl-4-morpholinyl]carbonyl]-1-(1-methylethyl)-1H-1,2,3-triazol-5-yl]-3-methoxy-;
7H-Indolo[2,1-a][2]benzazepin-10-carboxamid, 13-Cyclohexyl-N-[(dimethylamino)sulfonyl]-3-methoxy-6-[4-[(3-methyl-3,8-diazabicyclo[3.2.1]oct-8-yl)carbonyl]-1-(1-methylethyl)-1H-1,2,3-triazol-5-yl]-;
7H-Indolo[2,1-a][2]benzazepin-10-carboxamid, 13-Cyclohexyl-6-[4-[[(2R,6S)-2,6-dimethyl-4-morpholinyl]carbonyl]-1-ethyl-1H-pyrazol-5-yl]-3-methoxy-N-[(1-methylethyl)sulfonyl]-;
7H-Indolo[2,1-a][2]benzazepin-10-carboxamid, 13-Cyclohexyl-N-[(dimethylamino)sulfonyl]-6-[-4-[[(2R,6S)-2,6-dimethyl-4-morpholinyl]carbonyl]-1-ethyl-3-methyl-1H-pyrazol-5-yl]-3-methoxy-;
7H-Indolo[2,1-a][2]benzazepin-10-carboxamid, 13-Cyclohexyl-N-[(dimethylamino)sulfonyl]-3-methoxy-6-[1-(1-methylethyl)-4-(4-morpholinylcarbonyl)-1H-1,2,3-triazol-5-yl]-;
7H-Indolo[2,1-a][2]benzazepin-10-carboxamid, 13-Cyclohexyl-6-[4-[(6,6-difluorhexahydro-4-methyl-1H-1,4-diazepin-1-yl)carbonyl]-1-(1-methylethyl)-1H-1,2,3-triazol-5-yl]-N-[(dimethylamino)sulfonyl]-3-methoxy-;
7H-Indolo [2,1-a][2]benzazepin-10-carboxamid, 13-Cyclohexyl-6-[1-ethyl-4-[[(2R)-2-(methoxymethyl)-4-morpholinyl]carbonyl]-1H-pyrazol-5-yl]-3-methoxy-N-[(1-methylethyl)sulfonyl]-;
7H-Indolo [2, 1-a][2]benzazepin-10-carboxamid, 13-Cyclohexyl-6-[1-ethyl-4-(3-oxa-9-azabicyclo[3.3.1]non-9-ylcarbonyl)-1H-pyrazol-5-yl]-3-methoxy-N-[1-methylethyl}sulfonyl]-;
7H-Indolo[2,1-a][2]benzazepin-10-carboxamid, 13-Cyclohexyl-6-[1-ethyl-4-(2-oxa-5-azabicyclo [2.2.1]hept-5-ylcarbonyl)-1H-pyrazol-5-yl]-3-methoxy-N-[(1-methylethyl)sulfonyl]-;
7H-Indolo[2,1-a][2]benzazepin-10-carboxamid, 13-Cyclohexyl-6-[1-ethyl-4-[[(3R,5S)-3,4,5-trimethyl-1-piperazinyl]carbonyl]-1H-pyrazol-5-yl]-3-methoxy-N-[(1-methylethyl)sulfonyl]-;
7H-Indolo[2,1-a][2]benzazepin-10-carboxamid, 13-Cyclohexyl-6-[1-ethyl-4-[(3-methyl-3,8-diazabicyclo[3.2.1]oct-8-yl)carbonyl]-1H-pyrazol-5-yl]-3-methoxy-N-[(1-methylethyl)sulfonyl]-;
7H-Indolo[2,1-a][2]benzazepin-10-carboxamid, 13-Cyclohexyl-6-[1-ethyl-4-[(5-ethyl-2,5-diazabicyclo[2.2.1]hept-2-yl)carbonyl]-1H-pyrazol-5-yl]-3-methoxy-N-[(1-methylethyl)sulfonyl]-;
7H-Indolo[2,1-a][2]benzazepin-10-carboxamid, 13-Cyclohexyl-3-methoxy-N-[(1-methylethyl)sulfonyl]-6-[1-methyl-3-(1-methylethyl)-4-(4-morpholinylcarbonyl)-1H-pyrazol-5-yl]-;
7H-Indolo[2,1-a][2]benzazepin-10-carboxamid, 13-Cyclohexyl-6-[4-[[(2R,5S)-2,6-dimethyl-4-morpholinyl]carbonyl]-1-methyl-3-(1-methylethyl)-1H-pyrazol-5-yl] -3-methoxy-N-[(1-methylethyl)sulfonyl]-;
7H-Indolo[2,1-a][2]benzazepin-10-carboxamid, 13-Cyclohexyl-3-methoxy-N-[(1-methylethyl)sulfonyl]-6-[1-methyl-3-(1-methylethyl)-4-[(4-methyl-1-piperazinyl)carbonyl]-1H-pyrazol-5-yl]-;
7H-Indolo[2,1-a][2]benzazepin-10-carboxamid, 13-Cyclohexyl-3-methoxy-N-[(1-methylethyl)sulfonyl]-6-[1-methyl-3-(1-methylethyl)-4-[[(3R,5S)-3,4,5-trimethyl-1-piperazinyl]carbonyl]-1H-pyrazol-5-yl]-;
7H-Indolo[2,1-a][2]benzazepin-10-carboxamid, 13-Cyclohexyl-3-methoxy-N-[(1-methylethyl)sulfonyl]-6-[4-(8-oxa-3-azabicyclo[3.2,1]oct-3-ylcarbonyl)-1-(2,2,2-trifluorethyl)-1H-pyrazol-5-yl]-;
7H-Indolo[2,1-a][2]benzazepin-10-carboxamid, 13-Cyclohexyl-6-[4-(3,7-dioxa-9-azabicyclo[3.3.1]non-9-ylcarbonyl)-1-(2,2,2-trifluorethyl)-1H-pyrazol-5-yl]-3-methoxy-N-[(1-methylethyl)sulfonyl]-;
7H-Indolo[2,1-a][2]benzazepin-10-carbaxamid, 13-Cyclohexyl-3-methoxy-N-[(1-methylethyl)sulfonyl]-6-[4-(2-oxa-5-azabicyclo[2.2.1]hept-5-ylcarbonyl)-1-(2,2,2-trifluorethyl)-1H-pyrazol-5-yl]-;
7H-Indolo[2,1-a][2]benzazepin-10-carboxamid, 13-Cyclohexyl-3-methoxy-N-[(1-methylethyl)sulfonyl]-6-[4-[(4-methyl-1-piperazinyl)carbonyl]-1-(2,2,2-trifluorethyl)-1H-pyrazol-5-yl]-;
7H-Indolo[2,1-a][2]benzazepin-10-carboxamid, 13-Cyclohexyl-3-methoxy-6-[1-(1-methylethyl)-4-(8-oxa-3-azabicyclo[3.2.1]oct-3-ylcarbanyl)-1H-pyrazol-5-yl]-N-[(1-methylethyl)sulfonyl]-;
7H-Indolo[2,1-a][2]benzazepin-10-carboxamid, 13-Cyclohexyl-3-methoxy-b-[1-(1-methylethyl)-4-(3-oxa-9-azabicyclo[3.3.1]non-9-ylcarbanyl)-1H-pyrazol-5-yl]-N-[(1-methylethyl)sulfonyl]-;
7H-Indolo[2,1-a][2]benzazepin-10-carboxamid, 13-Cyclohexyl-3-methoxy-6-[1-(1-methylethyl)-4-(2-oxa-5-azabicyclo[2.2.1]hept-5-ylcarbonyl)-1H-pyrazol-5-yl]-N-[(1-methylethyl)sulfonyl]-;
7H-Indalo[2,1-a][2]benzazepin-10-carboxamid, 13-Cyclohexyl-3-methoxy-6-[1-(1-methylethyl)-4-[(4-methyl-1-piperazinyl)carbonyl]-1H-pyrazol-5-yl]-N-[(1-methylethyl)sulfonyl]-;
7H-Indolo[2,1-a][2]benzazepin-10-carboxamid, 13-Cyclohexyl-3-methoxy-N-[(1-methylethyl)sulfonyl]-6-[3-methyl-1-(1-methylethyl)-4-(8-oxa-3-azabicyclo[3.2,1]oct-3-ylcarbonyl)-1H-pyrazol-5-yl]-;
7H-Indolo[2,1-a][2]benzazepin-10-carboxamid, 13-Cyclohexyl-3-methoxy-N-[(1-methylethyl)sulfonyl]-6-[3-methyl-1-(1-methylethyl)-4-(2-oxa-5-azabicyclo[2.2.1]hept-5-ylcarbonyl)-1H-pyrazol-5-yl]-;
7H-Indolo[2,1-a][2]benzazepin-10-carboxamid, 13-Cyclohexyl-3-methoxy-N-[(1-methylethyl)sulfonyl]-6-[3-methyl-4-[(3-methyl-3,8-diazabicyclo[3.2.1]oct-8-yl)carbonyl]-1-(1-methylethyl)-1H-pyrazol-5-yl]-;
7H-Indolo[2,1-a][2]benzazepin-10-carboxamid, 13-Cyclohexyl-6-[4-[(5-ethyl-2,5-diazabicyclo[2,2,1]hept-2-yl)carbonyl]-3-methyl-1-(1-methylethyl)-1H-pyrazol-5-yl]-3-methoxy-N-[(1-methylethyl)sulfonyl]-;
7H-Indolo[2,1-a] [2]benzazepin-10-carboxamid, 13-Cyclohexyl-N-(cyclopropylsulfonyl)-6-[4-[[(2R,6S)-2,6-dimethyl-4-morpholinyl]carbonyl]-1-ethyl-3-methyl-1H-pyrazol-5-yl]-3-methoxy-;
7H-Indolo[2,1-a][2]benzazepin-10-carboxamid, 13-Cyclohexyl-3-methoxy-N-[(1-methylethyl)sulfonyl]-6-[5-methyl-1-(1-methylethyl)-4-[[(3R,5S)-3,4,5-trimethyl-1-piperazinyl]carbonyl]-1H-pyrazol-3-yl]-;
7H-Indolo[2,1-a][2]benzazepin-10-carboxamid, 13-Cyclohexyl-6-[1-ethyl-4-(8-oxa-3-azabicyclo[3.2.1]oct-3-ylcarhonyl)-1H-pyrazol-5-yl]-3-methoxy-N-[(1-methylethyl)sulfonyl]-;
7H-Indolo[2,1-a][2]benzazepin-10-carboxamid, 13-Cyclohexyl-6-[1-ethyl-4-[(8-methyl-3,8-diazabicyclo[3.2.1]oct-3-yl)carbonyl]-1H-pyrazol-5-yl]-3-methoxy-N-[(1-methylethyl)sulfonyl]-;
1H-Pyrazol-4-carbonsäure, 5-[13-Cyclohexyl-10-[[[(dimethylamino)sulfonyl]amino]carbonyl]-3-methoxy-7H-indolo[2,1-a][2]benzazepin-6-yl]-1-ethyl-3-methyl-, Ethylester;
7H-Indolo[2,1-a][2]benzazepin-10-carboxamid, 13-Cyclohexyl-6-[1-ethyl-3-methyl-5-(4-morpholinylcarbonyl)-1H-pyrazol-4-yl]-3-methoxy-N-[(1-methylethyl)sulfonyl]-;
7H-Indolo[2,1-a][2]benzazepin-10-carboxamid, 13-Cyclohexyl-6-[5-[[(2R,6S)-2,6-dimethyl-4-morpholinyl]carbonyl]-1-ethyl-3-methyl-1H-pyrazol-4-yl]-3-methoxy-N-[(1-methylethyl)sulfonyl]-;
7H-Indolo[2,1-a][2]benzazepin-10-carboxamid, 13-Cyclohexyl-6-[1-ethyl-3-methyl-5-[(4-methyl-1-piperazinyl)carbonyl]-1H-pyrazol-4-yl]-3-methoxy-N-[(1-methylethyl)sulfonyl]-;
7H-Indolo[2,1-a][2]benzazepin-10-carboxamid, 13-Cyclohexyl-6-[1-ethyl-3-methyl-5-[[(3R,5S)-3,4,5-trimethyl-1-piperazinyl]carbonyl]-1H-pyrazol-4-yl]-3-methoxy-N-[(1-methylethyl)sulfonyl]-;
1 H-Pyrazol-4-carbonsäure, 5-[13-Cyclohexyl-10-[[[(dimethylamino)sulfonyl]amino]carbonyl]-3-methoxy-7H-indolo[2,1-a][2]benzazepin-6-yl]-1-ethyl-3-methyl-;
7H-Indolo[2,1-a][2]benzazepin-10-carboxamid, 13-Cyclohexyl-6-[1-ethyl-4-[(5-ethyl-2,5-diazabicyclo[2.2.1]hept-2-yl)carbonyl]-3-methyl-1H-pyrazol-5-yl]-3-methoxy-N-[(1-methylethyl)sulfonyl]-;
7H-Indolo [2,1-a][2]benzazepin-10-carboxamid, 13-Cyclohexyl-6- [1-ethyl-3-methyl-4-[(8-methyl-3,8-diazabicyclo[3.2.1]oct-3-yl)carbonyl]-1H-pyrazol-5-yl]-3-methoxy-N-[(1-methylethyl)sulfonyl] -;
7H-Indolo[2,1-a][2]benzazepin-10-carboxamid, 13-Cyclohexyl-6-[1-ethyl-3-methyl-4-(4-morpholinylcarbonyl)-1H-pyrazol-5-yl]-3-methoxy-N-[(1-methylethyl)sulfonyl]-;
7H-Indolo[2,1-a][2]benzazepin-10-carboxamid, 13-Cyclohexy]-6-[1-ethy]-3-methyl-4-(3-oxa-9-azabicyclo[3.3.1]non-9-ylcarbonyl)-1H-pyrazol-5-yl]-3-methoxy-N-[(1-methylethyl)sulfonyl]-; und
7H-Indolo[2,1-a][2]benzazepin-10-carboxamid, 13-Cyclohexyl-6-[1-ethyl-4-[[(2S)-2-(methoxymethyl)-4-morpholinyl]carbonyl]-3-methyl-1H-pyrazol-5-yl]-3-methoxy-N-[(1-methylethyl)sulfonyl]-;
oder ein pharmazeutisch verträgliches Salz davon.

2. Zusammensetzung, umfassend eine Verbindung nach Anspruch 1 oder ein pharmazeutisch verträgliches Salz davon und einen pharmazeutisch verträglichen Träger.

3. Zusammensetzung nach Anspruch 2, ferner umfassend mindestens eine zusätzliche Verbindung, die therapeutische Vorteile für HCV aufweist, wobei die Verbindung aus Interferonen, Cyclosporinen, Interleukinen, HCV-Metalloproteasehemmem, HCV-Serinproteasehemmern, HCV-Polymerasehemmern, HCV-Helicasehemmern, HCV-NS4B-Proteinhemmern, HCV-Entry-Hemmern, HCV-Assembly-Hemmern, HCV-Egress-Hemmern, HCV-NS5A-Proteinhemmern, HCV-NS5B-Proteinhemmern und HCV-Replicon-Hemmern ausgewählt ist.

4. Verbindung nach Anspruch 1 zur Verwendung in einem Verfahren zur Behandlung von Hepatitis-C-Infektion.

5. Verbindung zur Verwendung nach Anspruch 4, ferner umfassend, mindestens eine zusätzliche Verbindung zu verabreichen, die therapeutische Nutzen für HCV aufweist, wobei die Verbindung aus Interferonen, Cyclosporinen, Interleukinen, HCV-Metalloproteasehemmern, HCV-Serinproteasehemmern, HCV-Polymerasehemern, HCV-Helicasehemmern, HCV-NS4B-Proteinhemmern, HCV-Entry-Hernmern, HCV-Assembly-Hemmern, HCV-Egress-Hemmern, HCV-NS5A-Proteinhemmern, HCV-NS5B-Proteinhemmern und HCV-Replicon-Hemmern ausgewählt ist.

## Revendications

1. Composé choisi dans le groupe constitué par
7H-indolo[2,1-a] [2]benzazépine-10-carboxamide, 13-cyclohexyl-6-[1-éthyl-3-méthyl-4-[(3-méhyl-3,8-diazabicyclo [3.2.1]oct-8-yl)carbonyl]-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1-éthyl-3-méthyl-4-[[(3R,5S)-3,4,5-triméthyl-1-pipérazinyl]carbonyl]-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1-éthyl-3-méthyl-4-[((1S,4S)-2-oxa-5-azabicyclo[2.2.1]hept-5-ylcarbonyl]-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1,3-diméthyl-4-(8-oxa-3-azabicyclo[3.2.1]oct-3-ylcarbonyl)-1H-pyrazol-5-yl]-3-méthoxy-N-[(1-méthyléthyl)sulfonyle]-;
7H-indolo[2,1-a][2]benzazépine-10-carboxamide, 13-cyclohexyl-3-méthoxy-6-[4-[[(2S)-2-(méthoxyméthyl)-4-morpholinyl]carbonyl]-1,3H-diméthyl-1H-pyrazol-5-yl]-N-[(1-méthyléthyl)sulfonyle]-;
7H-indolo[2,1-a][2]benzazépine-10-carboxamide, 13-cyclohexyl-6-[1,3-diméthyl-4-[(4-méthyl-1-pipérazinyl)carbonyl]-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1,3-diméthyl-4-[[(3R,5S)-3,4,5-triméthyl-1-pipérazinyl]carbonyl]-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[4-[[(2R,6S)-2,6-diméthyl-4-morpholinyl]carbonyl]-1-(1-méthyléthyl)-1H-1,2,3-triazol-5-yl]-3-méthoxy-N-[(1-méthyléthyl)sulfonyle]-;
7H-indolo[2,1-a][2]benzazépine-10-carboxamide, 13-cyclohexyl-3-méthoxy-N-[(1-méthyléthyl)sulfonyl]-6-[1-(1-méthyléthyl)-4-[[(3R,5S)-3,4,5-triméthyl-1-pipérazinyl]carbonyl]-1H-1,2,3-triazol-5-yle]-;
acide 7H-indolo[2,1-a][2]benzazépine-10-carboxylique, 13-cyclohexyl-6-[4-(éthoxycarbonyl)-1-éthyl-3-méthyl-1H-pyrazol-5-yl]-3-méthoxy-;
acide 1H-pyrazole-4-carboxylique, 5-[13-cyclohexyl-3-méthoxy-10-[[[(1-méthyléthyl)sulfonyl]amino]carbonyl]-7H-indolo[2,1-a][2]benzazépin-6-yl]-1-éthyl-3-méthyle-, ester éthylique;
7H-indolo[2,1-a][2]benzazépine-10-carboxamide, 13-cyclohexyl-6-[4-[[(2R,6S)-2,6-diméthyl-4-morpholinyl]carbonyl]-5-méthyl-1-(1-méthyléthyl)-1H-pyrazol-3-yl]-3-méthoxy-N-[(1-méthyléthyl)sulfonyle]-;
acide 1H-pyrazole-4-carboxylique, 5-[10-(aminocarbonyl)-13-cyclohexyl-3-méthoxy-7H-indolo[2,1-a][2]benzazépin-6-yl]-1-éthyl-3-méthyle-, ester éthylique;
acide 7H-indolo[2,1-a][2]benzazépine-10-carboxylique, 13-cyclohexyl-C-[4-(éthoxycarbonyl)-1-éthyl-1H-pyrazol-5-yl]-3-méthoxy-, ester 1,1-diméthyléthylique;
acide 7H-indolo[2,1-a][2]benzazépine-10-carboxylique, 13-cyclohexyl-6-[4-(éthoxycarbonyl)-1-éthyl-1H-pyrazol-3-yl]-3-méthoxy-, ester 1,1-diméthyléthylique;
acide 1H-pyrazole-4-carboxylique, 5-[13-cyclohexyl-3-méthoxy- 10-[[[(1-méthyléthyl)sulfonyl]amino]carbonyl]-7H-indolo[2,1-a][2]benzazépin-6-yl]-1-éthyl-3-éthyle-;
7H-indolo[2,1-a][2]benzazépine-10-carboxamide, 13-cyclohexyl-6-[4-[[(2R,6S)-2,6-diméthyl-4-morpholinyl]carbonyl]-1-(1-méthylethyl)-1H-imidazol-5-yl]-3-méthoxy-N-[(1-méthyléthyl)sulfonyle]-;
7H-indolo[2,1-a][2]benzazépine-10-carboxamide, 13-cyclohexyl-3-méthoxy-6-[1-(1-méthyléthyl)-4-(4-morpholinylcarbonyl)-1H-imidazol-5-yl]-N-[(1-méthyléthyl)sulfonyle]-;
7H-indolo[2,1-a][2]benzazépine-10-carboxamide, 13-cyclohexyl-3-méthoxy-6-[4-[(3-méthyl-3,8-diazabicyclo[3.2.1]oct-8-yl)carbonyl]-1-(1-méthyléthyl)-1H-imidazol-5-yl]-N-[(1-méthyléthyl)sulfonyle]-;
7H-indolo[2,1-a][2]benzazépine-10-carboxamide, 13-cyclohexyl-3-méthoxy-N-[(1-méthyléthyl)sulfonyl]-6-[1-(1-méthyléthyl)-4-[[(3R,5S)-3,4,5-triméthyl-1-pipérazinyl]carbonyl]-1H-imidazol-5-yle]-;
7H-indolo[2,1-a][2]benzazépine-10-carboxamide, 13-cyclohexyl-3-méthoxy-N-[(1-méthyléthyl)sulfonyl]-6-[5-méthyl-4-[(3-méthyl-3,8-diazabicyclo[3.2.1]oct-8-yl)carbonyl]-1-(1-méthyléthyl)-1H-pyrazal-3-yle]-;
acide 7H-indolo[2,1-a] [2]benzazépine-10-carboxylique, 13-cyclohexyl-6-[4-(éthoxycarbonyl)-1-éthyl-1H-pyrazol-5-yl]-3-méthoxy-;
acide 1H-pyrazole-4-carboxylique, 5-[13-cyclohexyl-3-méthoxy-10-[[[(1-méthyléthyl)sulfonyl]amino]carbonyl]-7H-indolo[2,1-a] [2]benzazépin-6-yl]-1-éthyle-, ester éthylique;
7H-indolo[2,1-a][2]benzazépine-10-carboxamide, 13-cyclohexyl-N-[(diméthylamino)sulfonyl]-6-[5-[[(2R,6S)-2,6-diméthyl-4-morpholinyl]carbonyl]-1-(1-méthyléthyl)-1H-imidazol-4-yl]-3-méthoxy-;
7H-indolo[2,1-a][2]benzazépine-10-carboxamide, 13-cyclohexyl-N-[(diméthylamino)sulfonyl]-3-méthoxy-6-[1-(1-méthyléthyl)-5-(4-morpholinylcarbonyl)-1H-imidazol-4-yle]-;
acide 1H-pyrazole-4-carboxylique, 5-[13-cyclohexyl-3-méthoxy-10-[[[(1-méthyléthyl)sulfonyl]amino]carbonyl]-7H-indolo[2,1-a] [2]benzazépin-6-yl]-1-éthyle-;
7H-indolo [2,1-a][2]benzazépine-10-carboxamide, 13-cyclohexyl-N-[(diméthylamino)sulfonyl]-3-méthoxy-6-[1-(1-méthyléthyl)-4-[[(3R,5S)-3,4,5-triméthyl-1-pipérazinyl]carbonyl]-1H-1,2,3-triazol-5-yle]-;
7H-indolo[2,1-a][2]benzazépine-10-carboxamide, 13-cyclohexyl-N-[(diméthylamino)sulfonyl]-6-[4-[[(2R,65)-2,6-diméthyl-4-morpholinyl]carbonyl]-1-(1-méthyléthyl)-1H-1,2,3-triazol-5-yl]-3-méthoxy-;
7H-indolo[2,1-a][2]benzazépine-10-carboxamide, 13-cyclohexyl-N-[(diméthylamino)sulfonyl]-3-méthoxy-6-[4-[(3-méthyl-3,8-diazabicyclo[3.2.1]oct-8-yl)carbonyl]-1-(1-méthyléthyl)-1H-1,2,3-triazol-5-yle]-;
7H-indolo[2,1-a][2]benzazépine-10-carboxamide, 13-cyclohexyl-6-[4-[[(2R,6S)-2,6-diméthyl-4-morpholinyl]carbonyl]-1-éthyl-1H-pyrazol-5-yl]-3-méthoxy-N-[(1-méthyléthyl)sulfonyle]-;
7H-indolo[2,1-a][2]benzazépine-10-carboxamide, 13-cyclohexyl-N-[(diméthylamino)sulfonyl]-6-[4-[[(2R,6S)-2,6-diméthyl-4-morpholinyl]carbonyl]-1-éthyl-3-méthyl-1H-pyrazol-5-yl]-3-méthoxy-;
7H-indolo[2,1-a] [2]benzazépine-10-carboxamide, 13-cyclohexyl-N-[(diméthylamino)sulfonyl]-3-méthoxy-6-1-(1-méthyléthyl)-4-(4-morpholinylcarbonyl)-1H-1,2,3-triazol-5-yle]-;
7H-indolo[2,1-a][2]benzazépine-10-carboxamide, 13-cyclohexyl-6-[4-[(6,6-difluorohexahydro-4-méthyl-1H-1,4-diazépin-1-yl)carbonyl]-1-(1-méthyléthyl)-1H-1,2,3-triazol-5-yl]-N-[(diméthylamino)sulfonyl]-3-méthoxy-;
7H-indolo[2,1-a][2]benzazépine-10-carboxamide, 13-cyclohexyl-6-[1-éthyl-4-[[(2R)-2-(méthoxyméthyl)-4-morpholinyl]carbonyl]-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1-éthyl-4-(3-oxa-9-azabicyclo[3.3.1]non-9-ylcarbonyl)-1H-pyrazal-5-yl]-3-méthoxy-N-[(1-méthyléthyl)sulfonyle]-;
7H-indolo[2,1-a][2]benzazépine-10-carboxamide, 13-cyclohexyl-6-[1-éthyl-4-(2-oxa-5-azabicyclo[2.2.1]hept-5-ylcarbonyl)-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1-éthyl-4-[[(3R,5S)-3,4,5-triméthyl-1-pipérazinyl]carbonyl]-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1-éthyl-4-[(3-méthyl-3,8-diazabicyclo[3.2.1]oct-8-yl)carbonyl]-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1-éthyl-4-[(5-éthyl-2,5-diazabicyclo[2.2.1]hept-2-yl)carbonyl]-1H-pyrazol-5-yl]-3-méthoxy-N-[(1-méthyléthyl)sulfonyle]-;
7H-indolo[2,1-a][2]benzazépine-10-carboxamide, 13-cyclohexyl-3-méthoxy-N-[(1-méthyléthyl)sulfonyl]-6-[1-méthyl-3-(1-méthyléthyl)-4-(4-morpholinylcarbonyl)-1H-pyrazol-5-yle]-;
7H-indolo[2,1-a][2]benzazépine-10-carboxamide, 13-cyclohexyl-6-[4-[[(2R,6S)-2,6-diméthyl-4-morpholinyl]carbonyl]-1-méthyl-3-(1-méthyléthyl)-1H-pyrazol-5-yl]-3-méthoxy-N-[(1-méthyléthyl)sulfonyle]-;
7H-indolo[2,1-a][2]benzazépine-10-carboxamide, 13-cyclohexyl-3-méthoxy-N-[(1-méthyléthyl)sulfonyl]-6-[1-méthyl-3-(1-méthyléthyl)-4-[(4-méthyl-1-pipérazinyl)carbonyl]-1H-pyrazol-5-yle]-;
7H-indolo[2,1-a][2]benzazépine-10-carboxamide, 13-cyclohexyl-3-méthoxy-N-[(1-méthyléthyl)sulfonyl]-6-[1-méthyl-3-(1-méthyléthyl)-4-[[(3R,5S)-3,4,5-triméthyl-1-pipérazinyl]carbonyl]-1H-pyrazol-5-yle]-;
7H-indolo[2,1-a][2]benzazépine-10-carboxamide, 13-cyclohexyl-3-méthoxy-N-[(1-méthyléthyl)sulfonyl]-6-[4-(8-oxa-3-azabicyclo[3.2.1]oct-3-ylcarbonyl)-1-(2,2,2-trifluoroéthyl)-1H-pyrazol-5-yle]-;
7H-indolo[2,1-a][2]benzazépine-10-carboxamide, 13-cyclohexyl-6-[4-(3,7-dioxa-9-azabicyclo[3.3.1]non-9-ylcarbonyl)-1-(2,2,2-trifluoroéthyl)-1H-pyrazol-5-yl]-3-méthoxy-N-[(1-méthyléthyl)sulfonyle]-;
7H-indolo[2,1-a][2]benzazépine-10-carboxamide, 13-cyclohexyl-3-méthoxy-N-[(1-méthyléthyl)sulfonyl]-6-[4-(2-oxa-5-azabicyclo[2.2.1]hept-5-ylcarbonyl)-1-(2,2,2-trifluoroéthyl)-1H-pyrazol-5-yle]-;
7H-indolo[2,1-a][2]benzazépine-10-carboxamide, 13-cyclohexyl-3-méthoxy-N-[(1-méthyléthyl)sulfonyl]-6-[4-[(4-méthyl-1-pipérazinyl)carbonyl]-1-(2,2,2-trifluoroéthyl)-1H-pyrazol-5-yle]-;
7H-indolo[2,1-a][2]benzazépine-10-carboxamide, 13-cyclohexyl-3-méthoxy-6-[1-(1-methyléthyl)-4-(8-oxa-3-azabicyclo[3.2.1]oct-3-ylcarbonyl)-1H-pyrazol-5-yl]-N-[(1-méthyléthyl)sulfonyle]-;
7H-indolo[2,1-a][2]benzazépine-10-carboxamide, 13-cyclohexyl-3-méthoxy-6-[1-(1-méthyléthyl)-4-(3-oxa-9-azabicyclo[3.3.1]non-9-ylcarbonyl)-1H-pyrazol-5-yl]-N-[(1-méthyléthyl)sulfonyle]-;
7H-indolo[2,1-a][2]benzazépine-10-carboxamide, 13-cyclohexyl-3-méthoxy-6-[1-(1-méthyléthyl)-4-(2-oxa-5-azabicyclo[2.2.1]hept-5-ylcarbonyl)-1H-pyrazol-5-yl]-N-[(1-méthyléthyl)sulfonyle]-;
7H-indolo[2,1-a][2]benzazépine-10-carboxamide, 13-cyclohexyl-3-méthoxy-6-[1-(1-méthyléthyl)-4-[(4-méthyl-1-pipérazinyl)carbonyl]-1H-pyrazol-5-yl]-N-[(1-méthyléthyl)sulfonyle] -;
7H-indolo[2,1-a][2]benzazépine-10-carboxamide, 13-cyclohexyl-3-méthoxy-N-[(1-méthyléthyl)sulfonyl]-6-[3-méthyl-1-(1-méthyléthyl)-4-(8-oxa-3-azabicyclo[3.2.1]oct-3-ylcarbonyl)-1H-pyrazol-5-yle]-;
7H-indolo[2,1-a][2]benzazépine-10-carboxamide, 13-cyclohexyl-3-méthoxy-N-[(1-méthyléthyl)sulfonyl]-6-[3-méthyl-1-(1-méthyléthyl)-4-(2-oxa-5-azabicyclo[2.2.1]hept-5-ylcarbonyl)-1H-pyrazol-5-yle]-;
7H-indolo[2,1-a][2]benzazépine-10-carboxamide, 13-cyclohexyl-3-méthoxy-N-[(1-méthyléthyl)sulfonyl]-6-[3-méthyl-4-[(3-méthyl-3,8-diazabicyclo[3.2.1]oct-8-yl)carbonyl]-1-(1-méthyléthyl)-1H-pyrazol-5-yle]-;
7H-indolo[2,1-a][2]benzazépine-10-carboxamide, 13-cyclohexyl-6-[4-[(5-éthyl-2,5-diazabicyclo[2.2.1]hept-2-yl)carbonyl]-3-méthyl-1-(1-méthyléthyl)-1H-pyrazol-5-yl]-3-méthoxy-N-[(1-méthyléthyl)sulfonyle]-;
7H-indolo [2,1-a] [2] benzazépine-10-carboxamide, 13-cyclohexyl-N-(cyclopropylsulfonyl)-6-[4-[[(2R,6S)-2,6-diméthyl-4-morpholinyl]carbonyl]-1-éthyl-3-méthyl-1H-pyrazol-5-yl]-3-médioxy-;
7H-indolo[2,1-a][2]benzazépine-10-carboxamide, 13-cyclohexyl-3-méthoxy-N-[(1-méthyléthyl)sulfonyl]-6-[5-méthyl-1-(1-méthyléthyl)-4-[[(3R,5S)-3,4,5-triméthyl-1-pipérazinyl]carbonyl]-1H-pyrazal-3-yle]-;
7H-indolo[2,1-a][2]benzazépine-10-carboxamide, 13-cyclohexyl-6-[1-éthyl-4-(8-oxa-3-azabicyclo[3.2.1]oct-3-ylcarbonyl)-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1-éthyl-4-[(8-méthyl-3,8-diazabicyclo[3.2.1]oct-3-yl)carbonyl]-1H-pyrazol-5-yl]-3-méthoxy-N-[(1-méthyléthyl)sulfonyle]-;
acide 1H-pyrazole-4-carboxylique, 5-[13-cyclohexyl-10-[[[(diméthylamino)sulfonyl]amino]carbonyl]-3-méthoxy-7H-indolo[2,1-a][2]benzazépin-6-yl]-1-éthyl-3-méthyle-, ester éthylique;
7H-indolo[2,1-a][2]benzazépine-10-carboxamide, 13-cyclohexyl-6-[1-éthyl-3-méthyl-5-(4-morpholinylcarbonyl)- H-pyrazol-4-yl]-3 -méthoxy-N-[(1-méthyléthyl)sulfonyle]-;
7H-indolo[2,1-a][2]benzazépine-10-carboxamide, 13-cyclohexyl-6-[5-[[(2R,6S)-2,6-diméthyl-4-morpholinyl]carbonyl]-1-éthyl-3-méthyl-1H-pyrazol-4-yl]-3-méthoxy-N-[(1-méthyléthyl)sulfonyle]-;
7H-indolo[2,1-a][2]benzazépine-10-carboxamide, 13-cyclohexyl-6-[1-éthyl-3-méthyl-5-[(4-méthyl-1-pipérazinyl)carbonyl]-1H-pyrazol-4-yl]-3-méthoxy-N-[(1-méthyléthyl)sulfonyle]-;
7H-indolo[2,1-a][2]benzazépine-10-carboxamide, 13-cyclohexyl-6-[1-éthyl-3-méthyl-5-[[(3R,5S)-3,4,5-triméthyl-1-pipérazinyl]carbonyl]-1H-pyrazol-4-yl]-3-méthoxy-N-[(1-méthyléthyl)sulfonyle]-;
acide 1H-pyrazole-4-carboxylique, 5-[13-cyclohexyl-10-[[[(diméthylamino)sulfonyl]amino]carbonyl]-3-méthoxy-7H-indolo[2,1-a][2]benzazépin-6-yl]-1-éthyl-3-méthyle-;
7H-indolo[2,1-a][2]benzazépine-10-carboxamide, 13-cyclohexyl-6-[1-éthyl-4-[(5-éthyl-2,5-diazabicyclo[2.2.]hept-2-yl)carbonyl]-3-méthyl-1H-pyrazol-5-yl]-3-méthoxy-N-[(1-méthyléthyl)sulfonyle]-;
7H-indolo[2,1-a][22]benzazépine-10-carboxamide, 13-cyclohexyl-6-[1-éthyl-3-méthyl-4-[(8-méthyl-3,8-diazabicyclo[3.2.1]oct-3-yl)carbonyl]-1H-pyrazol-5-yl]-3-méthoxy-N-[(1-méthyléthyl)sulfonyle]- ;
7H-indolo[2,1-a][2]benzazépine-10-carboxamide, 13-cyclohexyl-6-[1-éthyl-3-méthyl-4-(4-morpholinylcarbonyl)-1H-pyrazol-5-yl]-3-méthoxy-N-[(1-méthyléthyl)sulfonyle]-;
7H-indolo[2,1-a][2]benzazépine-10-carboxamide, 13-cyclohexyl-6-[1-éthyl-3-méthyl-4-(3-oxa-9-azabicyclo[3.3.1]non-9-ylcarbonyl)-1H-pyrazol-5-yl]-3-méthoxy-N-[(1-méthyléthyl)sulfonyle]-; et
7H-indolo[2,1-a][2]benzazépine-10-carboxamide, 13-cyclohexyl-6-[1-éthyl-4-[[(2S)-2-(méthoxyméthyl)-4-morpholinyl]carbonyl]-3-méthyl-1H-pyrazol-5-yl]-3-méthoxy-N-[(1-méthyléthyl)sulfonyle]-;
ou sel pharmaceutiquement acceptable de celui-ci.

2. Composition comprenant un composé selon la revendication 1, ou un sel pharmaceutiquement acceptable de celui-ci, et un véhicule pharmaceutiquement acceptable.

3. Composition selon la revendication 2, comprenant en outre au moins un composé supplémentaire ayant des bénéfices thérapeutiques contre le VHC, dans laquelle le composé est choisi dans le groupe constitué par les interférons, cyclosporines, interleukines, inhibiteurs de la métalloprotéase du VHC, inhibiteurs de la sérine protéase du VHC, inhibiteurs de la polymérase du VHC, inhibiteurs de l'hélicase du VHC, inhibiteurs de la protéine NS4B du VHC, inhibiteurs d'entrée du VHC, inhibiteurs d'assemblage du VHC, inhibiteurs de sortie du VHC, inhibiteurs de la protéine NS5A du VHC, inhibiteurs de la protéine NS5B du VHC et inhibiteurs de réplicons du VHC.

4. Composé selon la revendication 1, pour l'utilisation dans un procédé de traitement d'une infection d'hépatite C.

5. Composé pour l'utilisation selon la revendication 4, comprenant en outre l'administration d'au moins un composé supplémentaire ayant des bénéfices thérapeutiques contre le VHC, lequel composé est choisi dans le groupe constitué par les interférons, cyclosporines, interleukines, inhibiteurs de la métalloprotéase du VHC, inhibiteurs de la sérine protéase du VHC, inhibiteurs de la polymérase du VHC, inhibiteurs de l'hélicase du VHC, inhibiteurs de la protéine NS4B du VHC, inhibiteurs d'entrée du VHC, inhibiteurs d'assemblage du VHC, inhibiteurs de sortie du VHC, inhibiteurs de la protéine NS5A du VHC, inhibiteurs de la protéine NS5B du VHC et inhibiteurs de réplicons du VHC.
